(19) Europäisches Patentamt
European Patent Office
Office européen des brevets

(11) EP 1 002 100 B1

(12) # EUROPÄISCHE PATENTSCHRIFT

(45) Veröffentlichungstag und Bekanntmachung des Hinweises auf die Patenterteilung:
**02.07.2003 Patentblatt 2003/27**

(21) Anmeldenummer: **98938701.4**

(22) Anmeldetag: **23.07.1998**

(51) Int Cl.[7]: **C12N 15/55**, C12N 15/10, C12N 9/20, C12N 1/21, C12Q 1/44

(86) Internationale Anmeldenummer:
**PCT/EP98/04612**

(87) Internationale Veröffentlichungsnummer:
**WO 99/005288 (04.02.1999 Gazette 1999/05)**

(54) **VERFAHREN ZUR HERSTELLUNG UND IDENTIFIZIERUNG VON NEUEN HYDROLASEN MIT VERBESSERTEN EIGENSCHAFTEN**

METHOD FOR PRODUCING AND IDENTIFYING NEW HYDROLASES HAVING IMPROVED PROPERTIES

PROCEDE DE FABRICATION ET D'IDENTIFICATION DE NOUVELLES HYDROLASES A PROPRIETES AMELIOREES

(84) Benannte Vertragsstaaten:
**AT BE CH DE DK ES FR GB IE IT LI NL SE**

(30) Priorität: **25.07.1997 DE 19731990**

(43) Veröffentlichungstag der Anmeldung:
**24.05.2000 Patentblatt 2000/21**

(73) Patentinhaber: **Studiengesellschaft Kohle mbH**
**45470 Mülheim an der Ruhr (DE)**

(72) Erfinder:
- **REETZ, Manfred, T.**
  **D-45470 Mülheim an der Ruhr (DE)**
- **ZONTA, Albin**
  **D-45470 Mülheim an der Ruhr (DE)**
- **SCHIMOSSEK, Klaus**
  **D-45470 Mülheim an der Ruhr (DE)**
- **LIEBETON, Klaus**
  **D-45470 Mülheim an der Ruhr (DE)**
- **JÄGER, Karl-Erich Ruhr Universität Bochum**
  **D-44790 Bochum (DE)**

(74) Vertreter: **Helbing, Jörg, Dr.Dipl.-Chem. et al**
**Patentanwälte**
**von Kreisler-Selting-Werner,**
**Deichmannhaus am Hauptbahnhof**
**50667 Köln (DE)**

(56) Entgegenhaltungen:
**EP-A- 0 443 063** **WO-A-91/15581**
**WO-A-97/20918** **DE-A- 4 408 152**
**DE-A- 4 413 121**

- **SHINKAI A ET AL: "Substitutions of Ser for Asn-163 and Pro for Leu-264 are important for stabilization of lipase from Pseudomonas aeruginosa." JOURNAL OF BIOCHEMISTRY, (1996 NOV) 120 (5) 915-21. JOURNAL CODE: HIF. ISSN: 0021-924X., XP002087459 Japan**
- **STADLER P ET AL: "Stereoselectivity of microbial lipases. The substitution at position sn-2 of triacylglycerol analogs influences the stereoselectivity of different microbial lipases." EUROPEAN JOURNAL OF BIOCHEMISTRY, (1995 JAN 15) 227 (1-2) 335-43. JOURNAL CODE: EMZ. ISSN: 0014-2956., XP002087460 GERMANY: Germany, Federal Republic of**
- **LEUNG D ET AL: "A method for random mutagenesis of a defined DNA segment using a modified polymerase chain reaction" TECHNIQUE, Bd. 1, Nr. 1, August 1989, Seiten 11-15, XP002087461 in der Anmeldung erwähnt**
- **CADWELL R AND JOYCE G ET AL: "Randomization of genes by PCR mutagenesis" PCR METHODS AND APPLICATIONS, Bd. 2, 1992, Seiten 28-33, XP002087462**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 1 002 100 B1

- STEMMER W: "DNA shuffling by random fragmentatio and reassembly: In vitro recombination for molecular evolution" PROCEEDINGS OF THE NATIONAL ACADEMY OF SCIENCES OF USA, Bd. 91, Oktober 1994, Seiten 10747-10751, XP002087463 WASHINGTON US
- ZHANG J-H ET AL: "Directed evolution of a fucosidase from galactosidase by DNA shuffling and screening" PROCEEDINGS OF THE NATIONAL ACADEMY OF SCIENCES OF USA, Bd. 94, April 1997, Seiten 4504-4509, XP002087464 WASHINGTON US
- UZAWA H ET AL: "Determination of the lipase stereoselectivities using circular dichroism (CD);lipases produce chiral di-O-acylglycerols from achiral tri-O-acylglycerols." BIOCHIMICA ET BIOPHYSICA ACTA, (1993 JUL 1) 1168 (3) 253-60. JOURNAL CODE: A0W. ISSN: 0006-3002., XP002087466 Netherlands
- ZANDONELLA G ET AL: "Enantiomeric perylene-glycerolipids as fluorogenic substrates for a dual wavelength assay of lipase activity and stereoselectivity." CHIRALITY, (1996) 8 (7) 481-89. JOURNAL CODE: AVD. ISSN: 0899-0042., XP002087465 United States
- DATABASE WPI Section Ch, Week 9409 Derwent Publications Ltd., London, GB; Class B04, AN 94-068478 XP002100284 & JP 06 014772 A (NAGASE SANGYO KK) , 25. Januar 1994
- BERGMEYER H: "Methods of enzymatic analysis. Volume II" 1986 , METHODS OF ENZYMATIC ANALYSIS, SAMPLES, REAGENTS, ASSESMENT OF RESULT, NR. VOL. 2, PAGE(S) 236 - 239 , BERGMEYER H U XP002100288 siehe das ganze Dokument
- MUDERHWA J ET AL: "Purification and properties of the lipasesfrom Rhodotorula pilimanae Hedrick and Burke" APPLIED MICROBIOLOGY BIOTECHNOLOGY, Bd. 23, 1986, Seiten 348-354, XP002087467
- JENSEN R ET AL: "Determination of lipase specificity" LIPIDS, Bd. 18, Nr. 3, 1983, Seiten 239-252, XP002100282
- ZANDONELLA G ET AL: "Inversion of lipase stereospecificity for fluorogenic alkyldiacyl glycerols." EUROPEAN JOURNAL OF BIOCHEMISTRY, Bd. 231, 1995, Seiten 50-55, XP002100283
- REETZ, MANFRED T. ET AL: "Creation of enantioselective biocatalysts for organic chemistry by in vitro evolution" ANGEW. CHEM., INT. ED. ENGL. (1998), VOLUME DATE 1997, 36(24), 2830-2832 CODEN: ACIEAY;ISSN: 0570-0833, XP002087468
- REETZ M T ET AL: "Overexpression, immobilization and biotechnological application of Pseudomonas lipases." CHEMISTRY AND PHYSICS OF LIPIDS, (1998 JUN) 93 (1-2) 3-14. REF: 75 JOURNAL CODE: CZW. ISSN: 0009-3084., XP002087469 Ireland

## Beschreibung

Die vorliegende Erfindung betrifft ein Verfahren zur Herstellung und Identifizierung von Hydrolase-Mutanten mit verbesserten Eigenschaften hinsichtlich ihrer Stereo- oder Regioselektivität, katalytischen Aktivität oder Stabilität bei chemischen Umsetzungen.

Stand der Technik:

Hydrolasen gehören zu den am weitesten verbreiteten Enzymen in der organischen Synthese. Als Untergruppe der Hydrolasen katalysieren insbesondere Esterasen und Lipasen eine Vielzahl von Reaktionen wie z.B. die Hydrolyse von Carbonsäureestern, oder die Synthese von Estern bzw. Umesterungen in organischen Lösungsmitteln. Ihre hohe Stereoselektivität, Stabilität und gute Verfügbarkeit machen sie für zahlreiche industrielle Prozesse interessant. So werden z.B. Lipasen bereits industriell zur Racematspaltung von chiralen Alkoholen, Säuren oder Aminen eingesetzt, für die Darstellung enantiomerenreiner Arzneimittel, Naturstoffe, Pflanzenschutzmittel oder hochwertiger Fette und Öle (K.Faber, *Biotransformations in Organic Chemistry,* Springer-Verlag, Berlin, 2. Aufl. 1995). Dennoch läßt sich die Enantioselektivität einer Lipase bzw. Esterase gegenüber einem gegebenen Substrat nicht mit Sicherheit vorhersagen und in vielen Fällen verlaufen die Umsetzungen nur mit mäßigen optischen Ausbeuten. Es besteht daher ein Bedarf nach einem Verfahren zur Herstellung von Hydrolasen, das eine gezielte Optimierung der Enantioselektivität hinsichtlich eines gewünschten Produktes und der speziellen Prozeßbedingungen wie Temperatur und Lösungsmittel ermöglicht. Mit Hilfe der heute gebräuchlichen molekularbiologischen Methode der *in vitro*-Mutagenese ließen sich zwar Effekte auf die Enantioselektivität von Lipasen studieren (K.Hult, M. Holmquist, M. Martinelle, *European Symposium on Biocatalysis*, Graz, 1993, Abstracts, L-4), es konnte jedoch keine Optimierung hinsichtlich eines bestimmten Substrates erreicht werden, die zu einem organisch-synthetisch nutzbaren Enzym geführt hat.

Zu den bedeutendsten Einsatzmöglichkeiten der Gentechnik gehört das Proteindesign, bei dem auf der Grundlage bekannter Strukturdaten mit Hilfe der *in vitro*-Mutagenese basenspezifisch Mutationen in die Gensequenz des entsprechenden Proteins eingeführt werden. Durch gezielten Austausch von Aminosäuren ließen sich auf diese Weise schon Enzyme mit verbesserter katalytischer Aktivität oder Stabilität herstellen (A. Shaw, R. Bott, *Current Opinion in Structural Biology,* 1996, 6, 546). Diese Technik, die sogenannte oligonucleotidgerichtete *(oligonucleotidedirected*) oder gezielte Mutagenese (*site-directed mutagenisierendesis),* beruht auf dem Ersatz eines kurzen Sequenzabschnitts des Gens für das natürlich vorkommende Enzym (Wildtyp) durch ein synthetisch mutagenisiertes Oligonucleotid. Nach anschließender Expression des Gens erhält man eine Enzym-Mutante, die vorteilhafte Eigenschaften aufweisen kann. In einem davon abgeleiteten Verfahren, der sogenannten Kassettenmutagenese (*cassette mutagenesis),* werden Oligonucleotide mit partiell randomisierten Sequenzen eingesetzt. Dadurch erhält man eine begrenzt große Bibliothek von Mutanten, die dann hinsichtlich ihrer Eigenschaften getestet werden kann.

Trotz der Vorteile dieser etablierten Methoden eignen sie sich nur schlecht für die schrittweise Optimierung eines Enzyms, bzw. zur Erzeugung von Enzymen mit neuen Eigenschaften. Das bis heute unvollständige Verständnis der Gesetzmäßigkeiten der Proteinfaltung und der Struktur-Funktions-Beziehung bei Proteinen ist die Hauptursache für das Scheitern vieler Projekte auf dem Gebiet des sogenannten rationalen Proteindesigns. Zudem ist ein schrittweiser Optimierungsprozeß nach der klassischen Methode relativ arbeitsaufwendig und garantiert keine signifikante Verbesserung der Enzymeigenschaften *per se.*

In jüngster Zeit wurden neue molekularbiologische Methoden zur Mutagenese beschrieben, die auf der literaturbekannten Polymerase-Kettenreaktion (R.K. Saiki, S.J. Scharf, F. Faloona, K.B. Mullis, G.T. Horn, H.A. Erlich, N. Arnheim, *Science,* 1985, 230, 1350) beruhen (D.W. Leung, E. Chen, D.V. Goeddel, *Technique,* 1989, 1, 11 und W. P. C. Stemmer, A. Crameri, PCT WO 95/22625). Anstelle der gezielten Mutagenese werden hierbei kombinatorische Methoden zur Erzeugung umfangreicher Mutantenbibliotheken eingesetzt, die nachfolgend mit Hilfe geeigneter Screeningverfahren nach Mutanten mit positiven Eigenschaften durchsucht werden. Dabei werden die in der Natur vorkommenden evolutiven Prozesse der Replikation bzw. Rekombination, der Mutation und Selektion auf molekularer Ebene nachgeahmt. Diese als *in vitro*-Evolution (oder *directed evolution*) beschriebene Methode hat sich bereits in einigen Fällen als brauchbare Methode zur Gewinnung neuer Biokatalysatoren bewährt (W.P.C. Stemmer, *Nature,* 1994, 370, 389 und F.H. Arnold, *Chemical Engineering Science,* 1996, 51, 5091).

Trotz der erzielten Fortschritte auf diesem Gebiet läßt sich dieses Verfahren bisher nicht generell auf alle Enzymklassen übertragen, da es meist an geeigneten Testmethoden zur Identifizierung von Mutanten mit positiven Eigenschaften fehlt. Diese sind aber zwingend erforderlich angesichts der großen Anzahl von mutierten Enzymvarianten, die bei der Erzeugung kombinatorischer Mutantenbibliotheken zu erwarten sind. Insbesondere im Fall der für industrielle Prozesse interessanten Lipasen ist es bisher nicht gelungen, Mutanten mit verbesserter Stereoselektivität mit den Methoden der *in vitro*-Evolution zu erzeugen, weil ein effizientes Screening-Verfahren zum Test auf Enantioselektivität bis heute nicht existiert. Die klassische Methode zur Bestimmung der Enantioselektivität einer Lipase- bzw. Esterase-katalysierten Umsetzung beruht auf der flüssig-, bzw. gaschromatographischen Trennung der Reaktionsprodukte und Edukte unter Verwendung von chiral modifizierten, stationären Phasen. Diese Methode ist jedoch aufgrund des enormen Probenaufkommens im Zuge des Screenings von umfangreichen Mutantenbibliotheken ungeeignet, da chromatographische Trennungen mit chiral modifizierten Säulen zeitaufwendig sind und nur nacheinander durchgeführt werden können. Ein weiteres bisher ungelöstes Problem betrifft die häufig zu beobachtende Schwierigkeit, funktionelle Lipasen bzw. Esterasen in Wirtsorganismen mit genügend hoher Aktivitäts-Ausbeute zu exprimieren. Dies ist jedoch für ein leistungsfähiges Screeningsystem unverzichtbar, da zu geringe Enzym-Aktivitäten bei der Bestimmung der Enantioselektivität aufgrund der begrenzten Empfindlichkeit eines Testsystems nur schwer nachzuweisen sind.

**Gegenstand der Erfindung:**

Der Erfindung lag daher die Aufgabe zugrunde, ein einfaches Verfahren zur Herstellung von mutierten Hydrolasen, insbesondere Lipasen bzw. Esterasen, mit verbesserter Stereo-, bzw. Regioselektivität, katalytischer Aktivität und Stabilität gegenüber gegebenen Substraten (z.B. Carbonsäuren, Alkoholen, Aminen sowie deren Derivaten) zu entwickeln, das zudem eine schnelle Identifizierung positiver Mutanten aus umfangreichen Mutantenbibliotheken ermöglicht, sowie die Verwendung der so hergestellten Enzyme bei der Racematspaltung von chiralen Alkoholen, Säuren und Aminen sowie deren Derivaten.

Beschreibung der Erfindung:

Die vorliegende Erfindung betrifft

(1) ein Verfahren zur Herstellung und Identifizierung von Hydrolase-Mutanten mit verbesserten Eigenschaften hinsichtlich Stereo- oder Regioselektivität, dadurch gekennzeichnet, dass

a) ein Ausgangs-Hydrolasegen mittels einer modifizierten Polymerase-Kettenreaktion (PCR) mutagenisiert wird, wobei durch Einstellen der $Mg^{2+}$-, $Mn^{2+}$- und der Desoxynucleotid-Konzentrationen eine durchschnittliche Mutationsfrequenz von 1-2 Basenaustauschen bezogen auf das zu mutagenisierende Hydrolasegen eingestellt wird, sowie durch Einstellung der Zyklenanzahl die Gesamtanzahl der Mutationen in der amplifizierten DNA eingestellt wird,
b) gegebenenfalls ein oder mehrere gemäß Schritt a) mutierte Hydrolasegene oder Gemische von einem oder mehreren Ausgangs-Hydrolasegenen und einem oder mehreren gemäß Schritt a) mutierten Hydrolasegenen durch enzymatische Fragmentierung dieser Gene und nachfolgender enzymatischer Reassemblierung der entstandenen Fragmente zu vollständig rekombinierten Hydrolasegenen mutagenisiert werden,
c) die gemäß Schritt a) oder b) erhaltenen mutierten Hydrolasegene in einen Wirtsorganismus transformiert werden und
d) Hydrolase-Mutanten mit verbesserten Eigenschaften hinsichtlich Stereo- oder Regioselektivität, die von in Schritt c) erhaltenen Transformanten exprimiert werden, durch ein Testverfahren identifiziert werden;

(2) eine Lipase-Mutante mit verbesserter Stereoselektivität hinsichtlich chiraler Carbonsäuren oder COOH-funktionalisierter Verbindungen als die in Fig. 2 gezeigte Ausgangslipase aus dem Stamm *P. aeruginosa,* erhältlich durch Mutagenisierung der Ausgangslipase mit einem Verfahren gemäß (1);
(3) eine DNA-Sequenz, die für die unter (2) definierte Lipasemutante kodiert;
(4) ein Vektor, umfassend die unter (3) definierte DNA-Sequenz;
(5) eine Transformante, umfassend eine DNA-Sequenz, wie unter (3) definiert und/oder einen Vektor, wie unter (4) definiert und
(6) ein Verfahren zur Herstellung der in (2) definierten Lipasemutanten, umfassend das Kultivieren einer Transformanten gemäß (5).

Die Herstellung der neuen Biokatalysatoren beginnt in der Regel mit der Isolierung eines Lipase- bzw. Esterase-Gens aus dem Ursprungsorganismus. Hierfür kommen alle mikrobiellen, pflanzlichen und tierischen Organismen in Frage, die Träger eines Lipase- bzw. Esterase-Gens sind. Die Genisolierung kann nach den literaturbekannten Methoden vorgenommen werden (J. Sambrook, E.F. Fritsch, T. Maniatis, *Molecular Cloning: A Laboratory Manual*, Cold Spring Harbor Laboratory Press, 1989, New York). In der Regel wird dazu die genomische DNA mit Hilfe von Restriktionsendonucleasen fragmentiert und die erhaltenen Genfragmente in einem Wirtsorganismus (z.B. *E. coli*) kloniert. Unter Verwendung von Oligonucleotiden, die sequenzhomolog zu einem Abschnitt des Lipase- bzw. Esterase-Gens sind, wird dann in Hybridisierungsexperimenten das Gen in der Genbank identifiziert und nachfolgend isoliert.
Überraschend wurde im Rahmen der Erfindung gefunden, daß durch eine modifizierte Polymerase-Kettenreaktion (PCR) unter Veränderung bestimmter Reaktionsparameter natürlich vorkommende Hydrolasegene derart mutagenisiert werden können, daß eine umfangreiche Mutantenbibliothek erhalten wird, die mit Hilfe eines neuartigen Testverfahrens nach Mutanten mit verbesserter Enantioselektivität durchsucht werden kann.

Die Neuartigkeit des Verfahrens besteht darin, daß ausgehend von einem natürlich vorkommenden Lipase- bzw. Esterase-Gen (dem sogenannten Wildtyp-Gen) unter Verwendung einer modifizierten PCR (im folgenden als *mutagenisierende PCR* bezeichnet) eine umfangreiche, randomisierte Mutantenbibliothek angelegt werden kann. Dabei wurde gefunden, daß durch Veränderung der PCR-Reaktionskomponenten die Mutationsfrequenz während der PCR gezielt eingestellt werden kann. Durch die Variation der $Mg^{2+}$- und/oder der Desoxynucleotid-Konzentrationen und/oder der Zugabe von $Mn^{2+}$-Ionen läßt sich die Anzahl der Mutationen im betrachteten Lipase-Gen (Mutationsfrequenz) steuern. Vorzugsweise werden hierzu folgende Konzentrationen in Abhängigkeit der verwendeten DNA-Polymerase benutzt:

$Mg^{2+}$ : 1,5 mM - 8,0 mM
dNTP : 0,05 mM - 1,0 mM
$Mn^{2+}$ : 0,0 mM - 3.0 mM

Außerdem wurde gefunden, daß die Zyklenzahl der PCR-Reaktion mit der Anzahl der Mutationen korreliert: je höher die Zyklenzahl gewählt wird, desto höher die Gesamtanzahl der Mutationen. Mit Hilfe dieses Parameters läßt sich die Diversität der Mutantenbibliothek einstellen.

Zur Bestimmung der Mutationsfrequenz werden die aufgereinigten PCR-Produkte sequenziert. Durch Vergleich der erhaltenen Sequenzen mit der Sequenz des Wildtyp-Gens läßt sich die Mutationsfrequenz bestimmen.

Tabelle 1 zeigt die Abhängigkeit der Mutationsfrequenz von der Konzentration obengenannter PCR-Reaktionskomponenten bei der Amplifizierung des Lipase-Gens aus *P. aeruginosa (lipA)*.

Tabelle 1

| Versuch | $Mg^{2+}$ (mM) | $Mn^{2+}$ (mM) | dATP/ dGTP (mM) | dTTP/ dCTP (mM) | Mutationsfrequenz (Mutationen /1000 bp[1)]) |
|---|---|---|---|---|---|
| 1 | 6,1 | - | 0,2 | 0,2 | 1-2 |
| 2 | 7,0 | 0,5 | 0,2 | 1,0 | 15-20 |

[1)]bp = Basenpaare

Aufgrund der Resultate der Sequenzierung ergibt sich weiterhin, daß als Mutationsarten Transitionen und Transversionen statistisch in etwa gleicher Häufigkeit auftreten. Deletionen und Insertionen werden hingegen nur selten beobachtet. Zudem liegen die Mutationen über das ganze Lipase-Gen gleich verteilt vor. Somit läßt sich mit der beschriebenen Methode eine Mutantenbank mit statistisch gleichverteilten Mutationen erzeugen. Als vorteilhaft hat sich eine Mutationsfrequenz von 1-2 Mutationen / Hydrolasegen erwiesen. Dadurch wird verhindert, daß beim Auftreten mehrerer Mutationen pro Hydrolasegen eine negative Mutation eine Mutation mit positivem Effekt maskiert. Um eine komplette Mutantenbibliothek mit jeweils einem Aminosäureaustausch pro Enzymmolekül zu erhalten, müssen bei einer Lipase, die aus 285 Aminosäuren (hier: Lipase aus *P. aeruginosa)* besteht, theoretisch 5415 Mutanten erzeugt werden. Dieser Wert ergibt sich gemäß folgendem Algorithmus:

$$N = 19 \times M \times 285! / [(285 - M)! \times M!]$$

mit N = Anzahl der Mutanten und M = Anzahl der Aminosäure-Austausche pro Lipase-Molekül. Im Rahmen der Erfindung konnte überraschend gezeigt werden, daß bereits bei weitaus geringeren Bibliotheksgrößen positive Mutanten gefunden werden, wobei mit einer Mutationsfrequenz von 1-2 gearbeitet wurde.

Die nach dem beschriebenen Verfahren erhaltenen mutierten Lipase- bzw. Esterase-Gene werden in einen geeigneten Expressionsvektor ligiert und dann nach einem Wirtsorganismus, z.B. *E. coli,* transformiert. Anschließend werden die transformierten Zellen auf Agarplatten ausgestrichen und kultiviert. Sofern eine genügend hohe Expressionsrate vorliegt, können die erhaltenen Kolonien in mit Flüssigmedium versehene Mikrotiterplatten überführt werden und nach dem Anwachsen direkt zum Screeningtest eingesetzt werden. Für den Fall, daß bei der Expression des Lipasegens nur wenig Enzym gebildet wird, bzw. das Genprodukt im verwendeten Wirtsorganismus nicht korrekt gefaltet (Inklusionskörper) oder unvollständig in das Kulturmedium sekretiert wird, ist es vorteilhaft, die mutierten Gene in einen anderen Wirtsorganismus, vorzugsweise den Ursprungsorganismus, umzuklonieren.

Um genügend hohe Enzymaktivitäten zu erhalten, werden die einzelnen Bakterienklone, die ein mutiertes Lipase- bzw. Esterase-Gen enthalten, von den Agarplatten in die Tröge von handelsüblichen Mikrotiterplatten überführt

und dort in Flüssigmedium angezogen. Vorzugsweise werden hierfür Mikrotiterplatten mit 96 Trögen pro Platte benutzt. Das Wachstum der Bakterien kann durch Messung der Zelldichte ($OD_{600}$-Wert) kontrolliert werden. Es ist vorteilhaft, parallel eine zweite Mikrotiterplatte auf diese Weise anzuimpfen, um so eine Referenz für die spätere Identifizierung positiver Klone zu haben. Diese wird zweckmäßigerweise nach dem Anwachsen der Bakterien mit Gycerin versetzt und bis zur Identifizierung bei -80°C gelagert. Sofern die Bakterien das Enzym in den Extrazellularraum sekretieren (z.B. bei der Lipase aus *P. aeruginosa),* werden die Zellen in den Mikrotiterplatten abzentrifugiert und der Überstand mit der Lipase- bzw. Esterase-Aktivität zum Screeningtest eingesetzt. Für den Fall, daß die Bakterien (z.B. *E. coli*) das Enzym in das Periplasma sezernieren, muß vorher eine Zellwand-Lyse durchgeführt werden, wobei literaturbekannte Methoden wie z.B. eine Lysozym-Behandlung angewendet werden können.

Durch Anzucht der zugehörigen Klone von der Referenzplatte läßt sich ausreichend Plasmid-DNA isolieren, die zur Charakterisierung des mutierten Lipase- bzw. Esterase-Gens eingesetzt werden kann. Durch Sequenzierung werden die Mutationen im Gen lokalisiert. Ein Vorteil der Erfindung betrifft die Tatsache, daß auch ohne Kenntnisse der exakten Position der Mutationen in einem positiven Klon, das mutierte Gen in weiteren Mutations-Zyklen nach dem beschriebenen Verfahren hinsichtlich seiner Eigenschaften weiter optimiert werden kann. Dazu wird das isolierte Lipase- bzw. Esterase-Gen erneut in einer nach den oben angebenen Bedingungen modifizierten PCR (*mutagenisierende PCR*) eingesetzt. Diese Prozedur kann so oft wiederholt werden, bis die Eigenschaften der Lipase- bzw. Esterase-Mutante den Anforderungen an die stereoselektive Umsetzung genügen.

Zur weiteren Optimierung der identifizierten positiven Mutanten läßt sich das beschriebene Verfahren dergestalt erweitern, daß die DNA mehrerer Positiv-Mutanten zunächst fragmentiert wird und dann in einem kombinatorischen Prozeß nach W.P.C. Stemmer *(Nature,* 1994, 370, 389) zu funktionalen Lipase- bzw. Esterase-Genen reassembliert werden kann. Die so erhaltene *in vitro*-Rekombinantenbibliothek wird anschließend exprimiert und die rekombinanten Genprodukte mit Hilfe der erfindungsgemäßen Testverfahren auf verbesserte Enantioselektivität hin untersucht. Der Vorteil dieses Verfahrens besteht darin, daß sich aufgrund der Rekombination die positiven Eigenschaften verschiedener Lipase- bzw. Esterase-Mutanten in einem neuen rekombinanten Gen addieren können, was letztlich zu einer weiteren Verbesserung der Lipase bzw. Esterase führen kann. Der Ablauf des beschriebenen Verfahrens ist wie folgt:

Die Lipase- bzw. Esterase-Gene werden zunächst mit Hilfe des Enzyms DNAse I (z.B. aus Rinder-Pankreas) in Fragmente gespalten mit einer vorzugsweisen Länge zwischen 25 Bp und 100 Bp. Die Größe der Fragmente läßt sich durch Auftrennung mittels einer Agarose-Gelelektrophorese und Vergleich mit entsprechenden DNA-Längenmarkern kontrollieren. Die so erhaltenen DNA-Fragmente werden aufgereinigt, um sie von anhaftender DNAse zu befreien. Die *in vitro*-Rekombination wird unter den Bedingungen einer konventionellen PCR durchgeführt, wobei keine PCR-Primer zugesetzt werden. Analog zur konventionellen PCR unterteilt sich ein Zyklus in drei Schritte: a) Denaturierung, b) Annealing und c) Elongation. Während des Annealings kommt es zu einer Hybridisierung sequenzhomologer Fragmente, die aus unterschiedlichen mutierten Lipase- bzw. Esterase-Genen stammen können. Im nachfolgenden Elongationsschritt werden die Stränge durch die DNA-Polymerase vervollständigt, so daß schließlich neue, rekombinante Lipasegene erhalten werden. Die optimale Anzahl der Zyklen wird in einem Vorversuch bestimmt. Dazu trennt man nach jeweils 5 Zyklen eine geringe Probe des Reaktionsansatzes mittels Agarose-Gelelektrophorese auf und ermittelt daraus den Zyklus, bei dem das Maximum der Größenverteilung der Rekombinanten im Bereich der Größe des Enzym-Gens liegt. Vorzugsweise wird eine Zyklenzahl zwischen 30 und 45 gewählt. Die erhaltene Bande im Agarosegel, die der Größe nach dem Lipase- bzw. Esterase-Gen entspricht, wird aufgereinigt und durch eine konventionelle PCR amplifiziert. Das PCR-Produkt wird aufgereinigt und nach Ligation in einen geeigneten Vektor (Plasmid) nach *E. coli* transformiert. Wie bereits im Abschnitt über die *mutagenisierende PCR* besprochen, kann es erforderlich sein, in einen anderen Wirtsorganismus umzuklonieren, falls die Lipaseaktivität nach der Expression in *E. coli zu* gering sein sollte. Die erhaltenen Rekombinanten werden für den Test auf Enantioselektivität in Mikrotiterplatten angezogen.

In einer Variante der Erfindung können die beschriebenen Verfahren der *mutagenisierenden PCR* und der *in vitro*-Rekombination zur Erzeugung von Mutations- bzw. Rekombinantenbibliotheken nacheinander in beliebiger Reihenfolge und Häufigkeit durchgeführt bzw. wiederholt werden, um die Enantioselektivität der Lipase bzw. Esterase zu optimieren. Vorzugsweise wird zu Beginn mindestens ein Mutationszyklus mittels *mutagenisierender PCR* durchgeführt. Im Anschluß daran kann dann ein *in vitro*-Rekombinations-Zyklus erfolgen, wobei die jeweils besten positiven Mutantenklone eingesetzt werden. Durch Kontrolle der Enantioselektivität der erhaltenen Enzym-Mutanten läßt sich der Optimierungsprozeß verfolgen. bibliothek kann dann exprimiert werden und nach verbesserter Enantioselektivität gescreent werden.

Positive Lipase- bzw. Esterase-Mutanten, die durch das Screening von Mutanten- oder Rekombinantenbibliotheken identifiziert wurden, können mit Hilfe der ortsgerichteten Sättigungsmutagenese weiter optimiert werden. Dazu wird die positive Mutation in dem Lipase- bzw. Esterase-Gen zunächst durch Sequenzierung lokalisiert. Anschließend wird dieses Gen mittels einer beliebigen Methode zur ortsgerichteten Mutagenese, die einen mehrfachen Basenaustausch zuläßt, so verändert, daß alle möglichen Codons an der Stelle des Gens entstehen, die für die zu optimierende Position kodiert. Auf diese Weise erhält man eine begrenzt große Bibliothek von Mutanten, bei denen an

der zu optimierenden Aminosäure-Position die ursprünglich vorhandene Aminosäure durch die restlichen 19 Aminosäuren ersetzt ist. Die so erhaltene, begrenzt große Mutantenbibliothek kann dann exprimiert werden und nach verbesserter Enantioselektivität gescreent werden.

In einer Variante des beschriebenen Verfahrens wird das Lipase- bzw. Esterase-Gen des Wildtyp-Enzyms zusammen mit den gefundenen Positiv-Mutanten zur *in vitro*-Rekombination eingesetzt. Dadurch kann es zu Rückkreuzungen kommen, bei denen Mutationen mit neutralen oder negativen Eigenschaften eliminiert werden können. Die erhaltene Rekombinantenbibliothek kann nach der Expression auf verbesserte Enantioselektivität untersucht werden.

In einer weiteren Variante des beschriebenen Verfahrens werden Hydrolasegene aus unterschiedlichen Organismen zur *in vitro*-Rekombination eingesetzt, sofern sie eine genügende Sequenzhomologie zu dem ursprünglich eingesetzten Hydrolasegen besitzen.

In einer Variante des Verfahrens wird die *in* vitro-Rekombination unter den Bedingungen der beschriebenen modifizierten PCR durchgeführt. Dazu wird die Konzentration der $Mg^{2+}$- bzw. $Mn^{2+}$-Ionen sowie die der Desoxynucleotide (dNTPs) verändert, um gezielt die Mutationsfrequenz während der *in vitro*-Rekombination zu einzustellen.

Gegenstand der Erfindung sind weiterhin Testverfahren, die die Identifizierung von Enzym-Mutanten mit verbesserter Stereoselektivität oder Regioselektivität aus umfangreichen Mutantenbibliotheken ermöglichen. Dazu werden zwei gleiche Aliquots des enzymhaltigen Überstandes nach der Zentrifugation der Bakterienzellen in jeweils benachbarte Tröge einer neuen Mikrotiterplatte überführt. Nach Zugabe der beiden enantiomeren Substrate in jeweils einen der Tröge wird die Aktivität der Lipase bzw. Esterase spektrophotometrisch bestimmt. Die Messungen werden in einem handelsüblichen Spektralphotometer für Mikrotiterplatten durchgeführt. Dadurch wird ein hoher Probendurchsatz ermöglicht. Die Wahl des Substrates richtet sich nach der Art der chiralen Verbindung, für die eine Optimierung der Lipase bzw. Esterase vorgenommen werden soll. Insbesondere eignet sich das Verfahren für chirale Carbonsäuren, Alkohole und Amine.

Im Fall von chiralen Carbonsäuren bzw. chiralen COOH-funktionalisierten Verbindungen werden die beiden entsprechenden p-Nitrophenylester der (R)- bzw. (S)-Säure als Testsubstrat eingesetzt. Formel 1 zeigt das Prinzip des Testverfahrens, wobei R einen beliebigen organischen Rest mit mindestens einem asymmetrischen Zentrum symbolisiert.

<u>Formel 1</u>

Schema für das Testverfahren auf Stereoselektivität bei chiralen Carbonsäuren bzw. COOH-funktionalisierten Verbindungen

<u>**Reaktion 1:**</u>

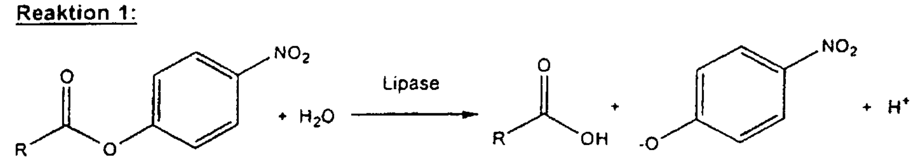


(R)-Enantiomer

<u>**Reaktion 2:**</u>

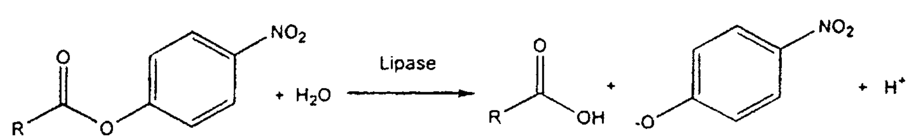


(S)-Enantiomer

Aufgrund der hohen Extinktion des bei der Hydrolase-katalysierten Esterhydrolyse freigesetzten p-Nitrophenolat-Anions ($\lambda_{max}$=405 nm, $E_{max}$=14.000) ergibt sich ein hochempfindliches Testverfahren, mit dem eine Aktivitätsbestimmung auch bei niedrigen Substratkonzentrationen durchgeführt werden kann. Die Enantioselektivität der Hydrolase-Mutanten läßt sich aus dem Quotienten der Hydrolysegeschwindigkeiten $V_{app(R)}$ und $V_{app(S)}$ für den (R)- und

den (S)-Ester mit ausreichender Genauigkeit bestimmen. Da die beiden Testansätze jeweils nur ein Enantiomer enthalten (entweder den R- oder S-Ester), muß bei der Bestimmung der Enantioselektivität die fehlende Konkurrenzreaktion mit dem anderen Enantiomer berücksichtigt werden. Dieser kinetische Effekt kann zwar zur Berechnung fehlerbehafteter Enantioselektivitäten führen, es hat sich jedoch gezeigt, daß die nach der vorgestellten Methode erhaltenen apparenten Enantioselektivitäten ($E_{app}$) genügend Aussage-kraft hinsichtlich der Enantioselektivität der mutierten Lipasen haben. $E_{app}$ ergibt sich als $V_{app(R)}$ / $V_{app(S)}$. Ein weiterer Vorteil liegt in der einfachen Durchführung und guten Reproduzierbarkeit des Tests, der sich auch für ein Screening mit hohem Probendurchsatz eignet.

Im Fall von chiralen Alkoholen bzw. von chiralen OH-funktionalisierten Verbindungen werden Fettsäureester der beiden enantiomerenreinen Alkohole für den Test auf Stereoselektivität eingesetzt. Die Kettenlänge der Fettsäuren liegt im Bereich von $C_2$ bis. Als Alkohol-Komponente können primäre, sekundäre und tertiäre Alkohole sowie deren Derivate mit mindestens einem Asymmetriezentrum eingesetzt werden. Lösungen der Ester der (R)- und (S)-Alkohole werden in jeweils benachbarten Trögen einer Mikrotiterplatte mit Kulturüberständen der Hydrolase-Mutanten hydrolysiert. Die Hydrolysegeschwindigkeiten $V_{app(R)}$ und $V_{app(S)}$ der (R)- und (S)-Ester sind ein Maß für die Enantioselektivität der untersuchten Enzym-Mutante. Die Detektion erfolgt über eine gekoppelte Enzymreaktion (H.U. Bergmeyer, *Grundlagen der enzymatischen Analyse,* Verlag Chemie, Weinheim, 1977), bei der die kontinuierliche Freisetzung der Fettsäure verfolgt wird. Der gebildete Farbstoff wird colorimetrisch bei 546 nm ($\varepsilon$ = 19,3 l x $mmol^{-1}$ x $cm^{-1}$) bestimmt. Die Konzentrationen der Enzyme, Cofaktoren und Coenzyme der Hilfsreaktionen 2 und 3 (siehe Formel 2) sowie der Indikatorreaktion 4 müssen so gewählt werden, daß die zu bestimmende Lipase- bzw. Esterase-katalysierte Reaktion geschwindigkeitsbestimmend ist. Der Quotient der Hydrolysegeschwindigkeiten des (R)- und (S)-Esters entspricht der apparenten Enantioselektivität ($E_{app}$). In einer Variante werden anstelle der enantiomerenreinen Ester die Fettsäureamide chiraler Amine bzw. $NH_2$- oder NHR-funktionalisierter Verbindungen eingesetzt. Formel 2 zeigt das Schema des Testsystems.

Formel 2

Schema für das Testverfahren auf Stereoselektivität bei chiralen Alkoholen; R symbolisiert einen beliebigen organischen Rest mit mindestens einem asymmetrischen Zentrum; Abkürzungen: CoA (Coenzym A), ATP (Adenosin-5'-triphosphat), AMP (Adenosin-5'-monophosphat)

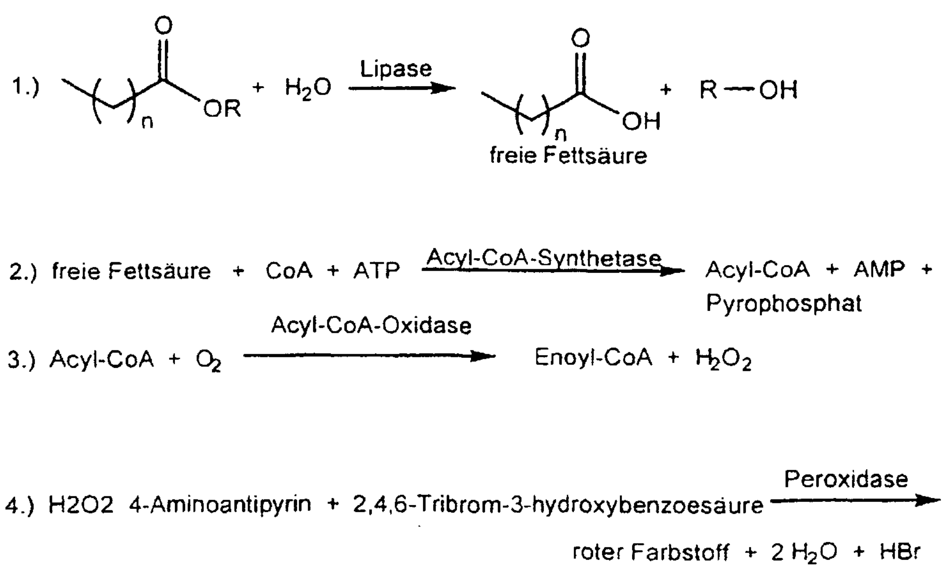


In einer Variante des Verfahrens können anstelle der Fettsäureester bzw. -amide die entsprechenden Ester und Amide der Bernsteinsäure eingesetzt werden. Diese haben gegenüber den Fettsäuren den Vorteil der besseren Löslichkeit in wäßrigen Lösungen bzw. wäßrig-organischen Solventien. Die Messung erfolgt UV-spektrometrisch bei 340 nm ($\varepsilon$ = 6,3 l $\times$ $mmol^{-1}$ x $cm^{-1}$). Auch bei diesem Testverfahren ist zu beachten, daß die Hydrolase-katalysierte Reaktion 1 geschwindigkeitsbestimmend ist. Der Quotient der Hydrolysegeschwindigkeiten $V_{app(R)}$ und $V_{app(S)}$ des (R)- und (S)-Esters entspricht der apparenten Enantioselektivität ($E_{app}$). In einer Variante werden anstelle der enantiomerenreinen Ester die Fettsäureamide chiraler Amine eingesetzt. Als Amin-Komponente können sowohl primäre als auch sekundäre Amine eingesetzt werden. Das Schema des Testverfahrens ist in Formel 3 wiedergegeben.

Formel 3

Schema für das Testverfahren auf Stereoselektivität bei chiralen Alkoholen; R symbolisiert einen beliebigen organischen Rest mit mindestens einem asymmetrischen Zentrum; Abkürzungen: CoA (Coenzym A), ITP (Inosin-5'-triphosphat), IDP (Inosin-5'-diphosphat), NADH / NAD⁺ (reduziertes bzw. oxidiertes Nicotinamid-adenin-dinucleotid)

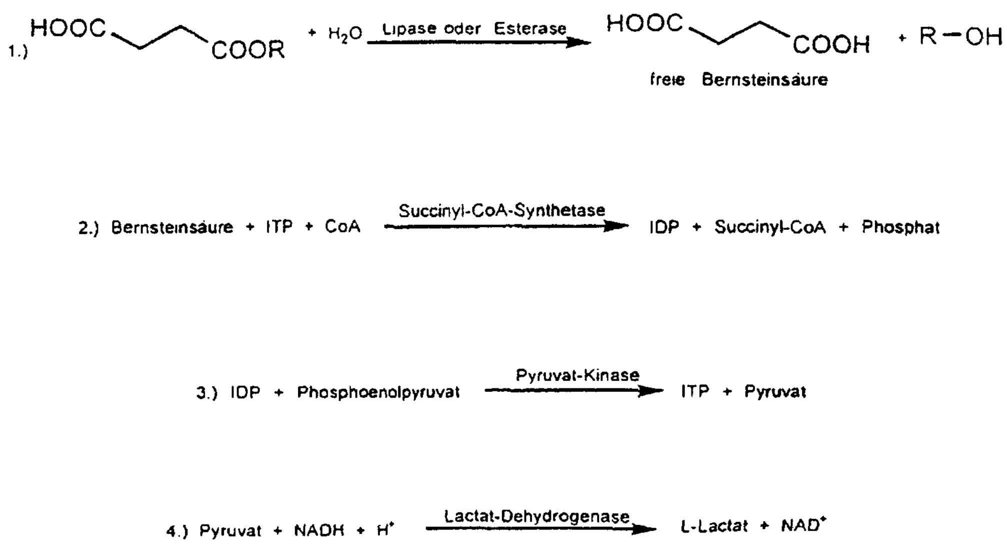


Der Test zur Identifizierung von Hydrolase-Mutanten mit verbesserter Stereoselektivität kann weiterhin derart ausgeführt werden, daß beide Stereoisomere im Testansatz enthalten sind. Dadurch kann auf die getrennte Messung des (R)- und (S)-Enantiomers verzichtet werden. Das Testprinzip geht von einer Anbindung eines racemischen Gemisches des chiralen Substrates an einer Festphase aus. Über eine Ester- bzw. Amidbindung an dieser chiralen Verbindung wird ein radioaktiv markierter organischer Rest gebunden. Zwei Fälle lassen sich unterscheiden:

a) Festphasen-gebundene chirale Carbonsäure: die Carboxylfunktion wird mit einem radioaktiv markierten Alkohol verestert

b) Festphasen-gebundener chiraler Alkohol oder chirales Amin, bzw. OH- oder $NH_2$- (NHR-)funktionalisierte Verbindungen: die Hydroxyl- bzw. Aminfunktion wird mit einer radioaktiv markierten Carbonsäure markiert.

Entscheidend ist dabei, daß die beiden Enantiomeren des an der Festphase gebundenen racemischen Gemisches mit unterschiedlichen Isotopen markiert sind. Vorzugsweise werden $^3H$ und $^{14}C$-markierte Verbindungen eingesetzt. Als Festphase können sowohl alle gängigen organischen, funktionalisierten Polymere sowie anorganische, funktionalisierte Träger eingesetzt werden. Vorzugsweise werden Fest-phasen auf Polystyrol-Basis und Kieselgel-Träger eingesetzt. Anschließend werden die chiralen, radioaktiv markierten Verbindungen an die Festphase gebunden, wobei die Kupplung an die Festphase an die chemische Beschaffenheit des chiralen Substrates angepaßt werden muß. Formel 4 zeigt das Schema der modifizierten Festphase und das Prinzip des Testverfahrens.

Formel 4

Schema des Festphasen-Screeningtests auf Stereoselektivität mit doppelt radioaktiv markiertem Sustrat; (X = O, NH; R ist ein radioaktiv markierter, organischer Rest)

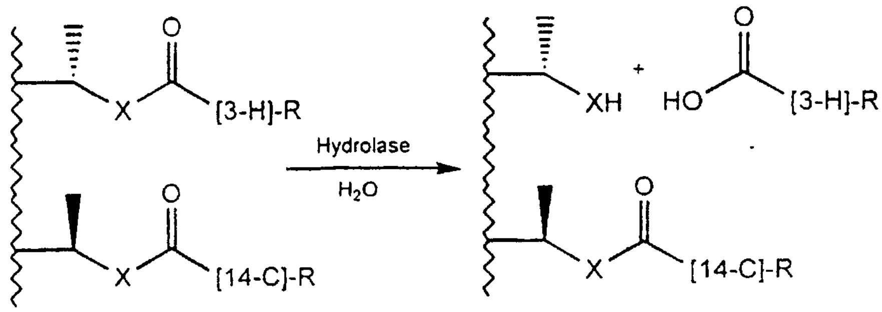


Etwa gleiche Mengen des so modifizierten Trägers können in kleine Reaktionsgefäße verteilt werden (z.B. in die Tröge von Mikrotiterplatten) und dann mit den Kulturüberständen der Hydrolase-Mutanten versetzt werden. Bei der anschließenden Reaktion werden die radioaktiv markierten Komponenten (Carbonsäure oder Alkohol) von der Festphase hydrolysiert und in das flüssige Medium abgegeben. Ein Aliquot des Mediums wird dann entnommen und in einem Szintillationsmeßgerät auf die Menge an Radioaktivität hin untersucht. Aus dem Verhältnis der beiden unterschiedlichen Isotope zueinander kann der Enantiomerenüberschuß und der Umsatz der Reaktion und somit die Enantioselektivität der mutierten Esterase bzw. Lipase berechnet werden. Durch Verwendung von regioisomeren Testverbindungen lassen sich die beschriebenen Tests auch zur Identifizierung von Hydrolase-Mutanten mit verbesserter Regioselektivität einsetzen. Anstelle von Hydrolase-Mutanten können auch andere Katalysatoren eingesetzt werden, um die Stereo- oder Regioselektivität zu bestimmen.

Der Test auf Enantioselektivität der nach dem beschriebenen Verfahren hergestellten Hydrolase-Mutanten kann auch durch kapillar-elektrophoretische Trennung erfolgen unter Verwendung von chiral modifizierten Kapillaren, die eine direkte Trennung der enantiomeren Substrate bzw. Produkte der Hydrolase-katalysierten Testreaktion ermöglichen. Hierzu können die Testsubstrate als Racemat eingesetzt werden. Die Trennung kann sowohl in Kapillaren erfolgen als auch unter Verwendung von präparierten Mikrochips, die eine elektrophoretische Trennung und Parallelisierung der Analysen zum Zwecke eines hohen Probendurchsatzes ermöglichen. Vorraussetzung ist in beiden Fällen, daß die Enantiomeren kapillarelektrophoretisch trennbar sind.

Die Erfindung wird durch die nachfolgenden Beispiele und Figuren näher erläutert.

Fig. 1 zeigt die experimentell erhaltenen Meßkurven zur Bestimmung der apparenten Enantioselektivität ($E_{app}$) bei der Hydrolyse von (R)- bzw. (S)-2-Methyldecansäure-p-nitrophenylester mit Kulturüberständen der Lipase-Mutanten P1B 01-E4, P2B 08-H3, P3B 13-D10, P4B 04-H3, P5B 14-C11, P4BSF 03-G10 und der Wildtyp-Lipase aus *P. aeruginosa* (die Steigerungen haben die Einheit [mOD/min]).

Fig. 2: Vergleich der DNA-Seqenzen der Lipase-Mutanten P1B 01-H1, P1B 01-E4, P2B 08-H3, P3B 13-D10, P4B 04-H3, P5B 14-C11 und P4BSF 03-G10 S155F mit der Sequenz des Wildtyps von Lipase aus *P. aeruginosa* (die mutierten Basen relativ zum Wildtyp sind eingerahmt, der Start der reifen Lipase-Mutanten liegt bei Base 163 bzw. bei Base 162 beim Wildtyp).

## Beispiel 1

Im folgenden Beispiel wurde das Gen der Lipase aus *P. aeruginosa* (Isolierung nach K.-E. Jäger, Ruhr-Universität Bochum) für eine Optimierung herangezogen. Das Substrat, auf das hin die Enantioselektivität der Lipase verbessert werden sollte, war *(R,S)*-2-Methyldecansäure. Es sollte eine Lipase-Mutante entwickelt werden mit einer Präferenz für das (S)-Enantiomer. Der Screening-Test wurde mit (R)- bzw. *(S)*-2-Methyl-decansäure-p-nitrophenylester durchgeführt.

Formel 5

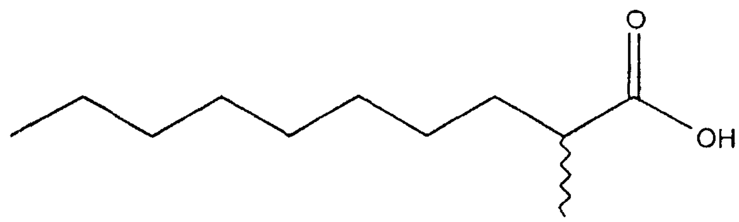

(R,S)-2-Methyldecansäure

Bakterienstämme

| | |
|---|---|
| *E. coli* JM109: | e14-(McrA), *rec*A1, *end*A1, *gyr*A96, *thi*-1, *hsd*R17($r_K$-$m_K$+), *sup*E44, *rel*A1, Δ(*lac-pro*AB), [F' *tra*Δ36 *pro*AB *lac*I$^q$ ZΔM15] (Fa. Stratagene) |
| *P. aeruginosa* PABST7.1: | lacUV5/lacI$^q$ reguliertes T7-Polymerasegen stabil in das Chromosom des Stamm *P. aeruginosa* PABS integriert, der eine Deletion im Strukturgen der Lipase *lipA* trägt (K.-E. Jaeger *et al. J. Mol. Cat. Part B,* 1997, in press) |

Plasmide

| | |
|---|---|
| pMut5: | *BamHI*/*ApaI* Fragment (1046 Bp) des *P. aeruginosa* Lipa-segens *lipA* im Vektor pBluescript KSII (Fa. Stratagene) |
| pUCPL6A: | *BamHI*/*HindIII* Fragment (2,8 KBp), welches das *P. aeru-ginosa* Lipase-Operon umfaßt, im Vektor pUCPKS (Watson *et al*., Gene 1996, 172, 163) unter der Kontrolle des T7-Promotors |

Kultivierung von Bakterien

*E. coli* JM109 wird über Nacht (16h) bei 37°C in 5 ml LB Medium auf einem Reagenzglasroller angezogen. Für *P. aeruginosa* PABST7.1 wird dem Medium 1mM IPTG zugesetzt. Für den Screening-Test wird *P. aeruginosa* PABST7.1 in Mikrotiterplatten auf einem Rundschüttler angezogen, wobei das Kulturvolumen 200 µl beträgt und die Inkubation auf 36-48h verlängert wird. Antibiotika werden in folgenden Konzentrationen zugegeben:
*E.coli* JM109: Ampicillin 100 µg/ml; *P.aeruginosa* PABST7.1: Carbenicillin 200 µg/ml, Tetrazyklin 50 µg/ml

Mutagenisierende PCR

Das Lipasegen *lipA* wird unter Verwendung des durch Endonuclease *Xmn* I linearisierten Plasmids pMut5 als Matrize und folgender PCR-Primer amplifiziert:

A :5'-GCGCAATTAACCCTCACTAAAGGGAACAAA-3';
B :5'-GCGTAATACGACTCACTATAGGGCGAA-3'

Nach Reinigung des PCR-Produktes mittels einer Qiagen Qiaquick Column® dient es als Template in einer mutagenen PCR-Reaktion. Die Reaktionsbedingungen sind wie folgt: ein 100 µl Reaktionsvolumen enthält 16,6 mM $(NH_4)_2SO_4$; 67 mM Tris-HCl (pH 8.8); 6,1 mM $MgCl_2$; 6,7 µM EDTA (pH 8.0); 0,2 mM dNTPs; 10 mM Mercaptoethanol; 10 µl DMSO; je 10 pmol der Primer; 0,1 ng Template-DNA und 1U Taq-Polymerase (Goldstar, Fa. Eurogentec). Das Reaktionsvolumen wird mit 100 µl Paraffin überschichtet. Es wurden 10 parallele Reaktionen durchgeführt, die nach Reaktionsende

vereinigt wurden. Das Zyklen-Protokol ist wie folgt: Nach einer 2 min Denaturierung bei 98°C, folgen 25 Zyklen mit 1 min 94°C, 2 min 64°C 1 min 72°C auf einem Robocycler 40 (Fa. Stratagen), gefolgt von einer 7 min Inkubation bei 72°C. Die Taq-Polymerase wird nach der Denaturierung des 1. Zyklus zugesetzt. Die Sequenzierung der PCR-Produkte ergibt eine Fehlerrate von ca. 1-2 Basenaustauschen pro 1000 Bp.

Klonierung der PCR-Produkte

Die PCR-Produkte werden mit Ethanol gefällt und in A. dest resuspendiert. Nach Restriktion mit *ApaI* und *BamHI* wird das entstandene 1046 Bp Fragment mittels einer Qiagen Qiaquick Column® gereinigt und unter Verwendung von T4-DNA Ligase (Fa. MBI Fermentas) für 2h bei RT in den entsprechend vorbereiteten Vektor pUCPL6A ligiert. Das Reaktionsvolumen wird 1:5 verdünnt und in 200 µl kompetenter Zellen von *E. coli* JM109, die nach der Methode von *Hanahan* (*J. Mol. Biol.* 1983, 166, 557) präpariert werden, transformiert. Dazu werden DNA und Zellen für 1h auf Eis gelagert, 2 min bei 42°C und nach Zugabe von 700 µl LB-Medium 45 min bei 37°C unter Schütteln inkubiert. Die Zellsuspension wird anschließend auf LB (Ampicillin 100 µg/ml) Platten ausplattiert. 60 ng des PCR-Produktes, die in die Ligationsreaktion eingesetzt werden, erbringen ca. 1500 Kolonien. Alle Kolonien werden in sterilem LB-Medium resuspendiert, die Plasmid DNA gereinigt und durch Elektroporation nach der Methode von *Farinha* und *Kropinski* (*FEMS Microbiol. Lett.* 1990, 70, 221) in *P. aeruginosa* PABST7.1 transformiert. Die 96 Tröge der Mikrotiterplatten werden mit jeweils einer Kolonie inokuliert und wie im Abschnitt "Kultivierung von Bakterien" beschrieben behandelt. Zur Gewinnung des Kulturüberstandes, der anschließend im Test auf Stereoselektivität eingesetzt wird, werden die Mikrotiterplatten 30 min bei 4000 Upm zentrifugiert.

Test auf Stereoselektivität

Die durch Zentrifugation gewonnenen Lipase-haltigen Kulturüberstände werden in jeweils zwei gleichen Aliquots in benachbarte Tröge einer Mikrotiterplatte pipettiert. Das Testvolumen beträgt 100 µl und setzt sich aus folgenden Komponenten zusammen (Tabelle 2):

Tabelle 2

| Zusammensetzung des Reaktionsansatzes für den Test auf verbesserte Enantioselektivität von Lipase-Mutanten | |
|---|---|
| (R)-Ansatz | (S)-Ansatz |
| 50 µl Kulturüberstand<br>40 µl 10 mM Tris/HCl-Puffer, pH 7,5<br>10 µl Substratlösung [10 mg / ml (R)-2-Methyldecansäure-p-nitrophenylester in DMF] | 50 µl Kulturüberstand<br>40 µl 10 mM Tris/HCl-Puffer, pH 7,5<br>10 µl Substratlösung [10 mg / ml (S)-2-Methyldecansäure-p-nitrophenylester in DMF] |

Nach der Zugabe des Tris/HCl-Puffers zu den Überständen wird die Mikrotiterplatte ca. 5 min bei 30 °C inkubiert. Die Reaktion wird nach Addition der Substratlösung spektrophotometrisch bei 410 nm und 30 °C kontinuierlich 10 min lang verfolgt. Aus dem linearen Anstieg der Absorptionskurve, die ein Maß für die konstante Anfangsgeschwindigkeit der Hydrolyse ist, wird die apparente Enantioselektivität ($E_{app}$) bestimmt. Dazu werden die gemessenen Steigungen im linearen Bereich der Anfangsgeschwindigkeiten der Reaktionen des Enantiomerenpaares dividiert und man erhält den Wert für die apparente Enantioselektivität der entsprechenden Lipase-Mutante.

Bestimmung der Stereoselektivität durch Gaschromatoqraphie

Ausgewählte positive Klone werden in 5 ml -Flüssigkulturen (LB-Medium) angezogen und der Lipase-haltige Überstand nach Zentrifugation und Entfernung des Bakterien-Pellets zur Reaktion eingesetzt. Als Substrat werden 100 µl einer Lösung von racemischem *(R,S)*-2-Methyldecansäurep-nitrophenylester (10 mg/ml in Dimethylformamid) eingesetzt. Diese wird mit 700 µl 10 mM Tris/HCl-Puffer pH 7,5 versetzt. Die Reaktion wird durch Zugabe von 100 µl Kulturüberstand gestartet und bei 30°C und 1000 rpm in Eppendorf-Reaktionsgefäßen durchgeführt. Nach 2,5 h werden jeweils 200 µl Proben entnommen und in ein mit 200 µl Dichlormethan gefülltes Eppendorf-Gefäß überführt. Nach Zugabe von 25 µl 20 %iger Salzsäure werden Produkte und Edukt extrahiert (Vortex-Schüttler, 1 min). Die organische Phase wird schließlich zur gaschromatographischen Analyse (GC) eingesetzt. Dabei wird eine Trennung der Enantiomere der freien 2-Methyldecansäure erzielt.

| Trennbedingungen der GC: | |
|---|---|
| Instrument | Hewlett Packard 5890 |
| Säule | 25 m 2,6 DM 3 Pent β-CD / 80% SE 54 |
| Detector | FID |
| Temperatur | 230°C Inlet; 80-190°C mit 2°C / min |
| Gas | 0,6 bar $H_2$ |
| Probenmenge | 0,1 ml |

Ergebnisse (1. Zyklus)

Von ca. 1000 untersuchten Klonen, die durch *mutagenisierende PCR* mit der Ausgangs-DNA (Wildtyp-Gen von Lipase aus *P. aeruginosa*) erhalten wurden, wurden 12 mit verbesserter Enantioselektivität gegenüber dem entsprechenden Wildtyp-Enzym identifiziert. 3 Klone wurden schließlich ausgewählt und deren Enantioselektivität durch GC-Analyse bestimmt.

Tabelle 3

| Ausgewählte Lipase-Mutanten mit verbesserter Enantioselektivität (1. Zyklus) | | | | | |
|---|---|---|---|---|---|
| Mutante | $V_{app}$(S) [mOD/ min] | Vapp(R) [mOD/ min] | $E_{app}$ [1]) | % ee (nachGC)/% Umsatz | E-Wert [2]) (berechnet nach GC) |
| Wildtyp | 21,8 | 14,9 | 1,5 | 2,4 / 15,3 | 1,1 |
| P1B 01-E4 | 128,4 | 43,2 | 3,0 | 36,1 / 23,2 | 2,4 |
| P1B 01-F12 | 78,8 | 35,7 | 2,2 | 14,1 / 30,5 | 1,4 |
| P1B 01-H1 | 158,7 | 56,2 | 2,8 | 37,6 / 4,5 | 2,2 |

[1]) $E_{app} = V_{app}(S) / V_{app}(R)$

[2]) $E = \ln[1-c(1+ee_p)] / \ln[1-c(1-ee_p)]$ mit c = Umsatz, $ee_p$ = ee-Wert des Produktes

Die DNA des Klons P1B 01-E4 diente als Ausgangspunkt für eine neue PCR-Mutagenisierungsrunde. Dazu wurde das Plasmid pUCPL6A aus dem Klon isoliert und wie oben beschrieben nach *E. coli* JM109 transformiert. Nach Präparation der Plasmid-DNA wurde das 1046 Bp große Fragment durch Restriktion mit *Apal* und *BamHI* und anschließender Reinigung gewonnen und in das entsprechend vorbereitete Plasmid pMut5 ligiert. Nach Transformation und Plasmidisolierung diente dieses Plasmid als template-DNA in einer *mutagenisierenden PCR*-Reaktion unter den oben beschriebenen Bedingungen. Die gewonnene DNA der *mutagenisierenden PCR* diente zur Herstellung einer neuen Mutantenbibliothek (2. Generation).

Ergebnisse (2. Zyklus)

Aus der Mutantenbibliothek der 2. Generation wurden ca. 2200 Klone zum Screening-Test herangezogen. Dabei wurden 10 Mutanten mit einer gegenüber der Mutante P1B 01-E4 verbesserten Enantioselektivität identifiziert. 2 Mutanten (P2B 04-G11 und P2B 08-H3) wurden per GC-Analyse genauer untersucht.

Tabelle 4

| Ausgewählte Lipase-Mutanten mit verbesserter Enantioselektivität (2. Zyklus) | | | | | |
|---|---|---|---|---|---|
| Mutante | $V_{app}$(S) (mOD/ min] | Vapp(R) [mOD/ min] | $E_{app}$ [1]) | % ee (nachGC) / % Umsatz | E-Wert [2]) (berechnet nach GC) |
| P2B 04-G11 | 224,9 | 52,3 | 4,3 | 47,8 / 30,0 | 3,4 |
| P2B 08-H3 | 310,8 | 67,4 | 4,6 | 56,6 / 19,3 | 4,1 |

[1]) $E_{app}$ $V_{app}(S) / V_{app}(R)$

[2]) $E = \ln[1-c(1+ee_p)] / \ln[1-c(1-ee_p)]$ mit c = Umsatz, $ee_p$ = ee-Wert des Produktes

Der Klon P2B 08-H3 wurde für die nächste Mutationsrunde (3. Generation) eingesetzt.

Ergebnisse (3. Zyklus)

Aus der Mutantenbibliothek der 3. Generation wurden ca. 2400 Klone zum Screening-Test herangezogen. Dabei wurde 1 Mutante (P3B 13-D10) mit einer gegenüber der Mutante P2B 08-H3 verbesserten Enantioselektivität identifiziert. Diese wurde durch GC-Analyse weiter untersucht.

Tabelle 5

| Ausgewählte Lipase-Mutanten mit verbesserter Enantioselektivität (3. Zyklus) | | | | | |
|---|---|---|---|---|---|
| Mutante | $V_{app}$(S) [mOD/min] | Vapp(R) [mOD/min] | $E_{app}$ 1) | % ee (nachGC)/% Umsatz | E-Wert 2) (berechnet nach GC) |
| P3B 13-D10 | 240,0 | 35,2 | 6,9 | 74,8 / 34,6 | 10,2 |

1) $E_{app} = V_{app}(S) / V_{app}(R)$
2) $E = \ln[1-c(1+ee_p)] / \ln[1-c(1-ee_p)]$ mit c = Umsatz, $ee_p$ = ee-Wert des Produktes

Ergebnisse (4. Zyklus)

Aus der Mutantenbibliothek der 4. Generation wurden ca. 2000 Klone zum Screening-Test herangezogen. Dabei wurden 4 Mutanten mit einer gegenüber der Mutante P3B 13-D10 verbesserten Enantioselektivität identifiziert. Diese wurde durch GC-Analyse weiter untersucht.

Tabelle 6

| Ausgewählte Lipase-Mutanten mit verbesserter Enantioselektivität (4. Zyklus) | | | | | |
|---|---|---|---|---|---|
| Mutante | $V_{app}$(S) [mOD/min] | Vapp(R) [mOD/min] | $E_{app}$ 1) | % ee (nachGC) /% Umsatz | E-Wert 2) (berechnet nach GC) |
| P4B 04-H3 | 355,6 | 26,5 | 13,4 | 81,0 / 20,0 | 11,2 |
| P4B 01-F2 | 162,4 | 13,8 | 11,7 | 82,1 / 5,0 | 10,6 |
| P4B 15-G1 | 315,4 | 28,1 | 11,2 | 80,0 / 18,0 | 10,7 |
| P4B 15-H7 | 288,0 | 25,1 | 11,5 | 78,4 / 22,0 | 10,2 |

1) $E_{app} = V_{app}(S) / V_{app}(R)$
2) $E = \ln[1-c(1+ee_p)] / \ln[1-c(1-ee_p)]$ mit c = Umsatz, $ee_p$ = ee-Wert des Produktes

Der Klon P4B 04-H3 wurde für die nächste Mutationsrunde (5. Generation) eingesetzt.

Ergebnisse (5. Zyklus)

Aus der Mutantenbibliothek der 5. Generation wurden ca. 5200 Klone zum Screening-Test herangezogen. Dabei wurden 2 Mutanten mit einer gegenüber der Mutante P4B 04-H3 verbesserten Enantioselektivität identifiziert. Diese wurde durch GC-Analyse weiter untersucht.

Tabelle 7

| Ausgewählte Lipase-Mutanten mit verbesserter Enantioselektivität (5. Zyklus) | | | | | |
|---|---|---|---|---|---|
| Mutante | $V_{app}$(S) [mOD/min] | Vapp(R) [mOD/min] | $E_{app}$ 1) | % ee (nachGC) /% Umsatz | E-Wert 2) (berechnet nach GC) |
| P5B 14-C11 | 275,9 | 17,3 | 15,9 | 77,0 / 43,0 | 13,7 |
| P5B 08-F2 | 124,0 | 8,7 | 14,3 | 79,7 / 40,3 | 15,1 |

1) $E_{app} = V_{app}(S) / V_{app}(R)$
2) $E = \ln[1-c(1+ee_p)] / \ln[1-c(1-ee_p)]$ mit c = Umsatz, $ee_p$ = ee-Wert des Produktes

Sequenzierung der positiven Mutanten

Durch Sequenzierung der positiven Mutanten konnten die Mutationen in den Lipase-Genen lokalisiert werden (siehe Figur 2). Nach Zuordnung der Basentripletts zu den entsprechenden Aminosäuren ergeben sich gegenüber der Wildtyp-Lipase aus *P. aeruginosa* folgende Aminosäure-Austausche :

| | |
|---|---|
| P1B 01-H1 | T103I ($Thr_{103} \rightarrow Ile_{103}$), S149G ($Sen_{149} \rightarrow Gly_{149}$) |
| P1B 01-E4 | S149G ($Ser_{149} \rightarrow Gly_{149}$) |
| P2B 08-H3 | S149G ($Ser_{149} \rightarrow Gly_{149}$), S155L ($Ser_{155} \rightarrow Leu_{155}$) |
| P3B 13-D10 | S149G ($Ser_{149} \rightarrow Gly_{149}$), S155L ($Ser_{155} \rightarrow Leu_{155}$), V47G ($Val_{47} \rightarrow Gly_{47}$) |
| P4B 04-H3 | S149G ($Ser_{149} \rightarrow Gly_{149}$), S155L ($Ser_{155} \rightarrow Leu_{155}$), V47G ($Val_{47} \rightarrow Gly_{47}$), S33N ($Ser_{33} \rightarrow Asn_{33}$), F259L ($Phe_{259} \rightarrow Leu_{259}$) |
| P5B 14-C11 | S149G ($Ser_{149} \rightarrow Gly_{149}$), S155L ($Ser_{155} \rightarrow Leu_{155}$), V47G ($Val_{47} \rightarrow Gly_{47}$), S33N ($Ser_{33} \rightarrow Asn_{33}$), F259L ($Phe_{259} \rightarrow Leu_{259}$), K110R ($Lys_{110} \rightarrow Arg_{110}$) |

Die Mutanten P1B 01-E4, P2B 08-H3 und P3B 13-D10 wurden unter den Bezeichnungen DSM 11 658, DSM 11 659 und DSM 11 659 am 16.07.1997 bei der DSMZ - Deutsche Sammlung von Mikroorganismen und Zellkulturen GmbH, D-38124 Braunschweig, Mascheroder Weg 1b, hinterlegt.

Die Mutanten P5B 14-C11 und P4B 04-H3 wurden unter den Bezeichnungen DSM 12 320 und DSM 12 322 am 20.07.1998 bei der DSMZ - Deutsche Sammlung von Mikroorganismen und Zellkulturen GmbH, D-38124 Braunschweig, Mascheroder Weg 1b, hinterlegt.

Beispiel 2

Die Vorschriften zur Kultivierung der Bakterien, der *mutagenisierenden PCR* sowie des Testverfahrens auf Enantioselektivität sind analog zu Beispiel 1. Die Herstellung umfangreicher Mutantenbibliotheken erfolgt in diesem Beispiel jedoch durch *in vitro*-Rekombination.

Die für die *in* vitro-Rekombination verwendete DNA wird entweder durch *mutagenisierende PCR* generiert oder durch Vereinigung der DNA aus einer beliebigen Anzahl von Klonen aus einer oder mehreren, durch wiederholte *mutagenisierende PCR* entstandenen Generationen an Klonen gewonnen. Wenn die PCR-Produkte einer *mutagenisierenden PCR* Ausgangspunkt zur Gewinnung von DNA für die *in* vitro-Rekombination waren, wird wie folgt verfahren: Die PCR-Produkte der *mutagenisierenden PCR* (siehe Beispiel 1) werden gereinigt, mit den Restriktionsendonucleasen *Apa* I und *BamH* I geschnitten, in den entsprechend geschnittenen Vektor pMUTS ligiert und anschließend nach *E. coli* JM 109 transformiert. Die Plasmid-DNA aus allen Transformationsklonen wird isoliert. War eine beliebige Anzahl ausgewählter Klone einer oder verschiedener Generationen an Mutantenklonen Ausgangspunkt für die Gewinnung von DNA für die *in vitro*-Rekombination, so wird die Plasmid-DNA des Vektors pMUT5 mit den jeweiligen Varianten des Lipasegens von P. *aeruginosa* isoliert und vereinigt. In beiden Fällen wird wie folgt weiter verfahren: Durch Restriktion mit der Endonuclease *Pvu* II wird ein 1430 Bp großes Fragment gewonnen, das neben dem Strukturgen für die Lipase von *P. aeruginosa* die Bindestellen der schon in der *mutagenisierenden PCR* verwendeten Primer A und B umfaßt. Dieses Fragment wird gereinigt und durch Inkubation mit Desoxyribonuclease I (DNAse I aus *Rinder-Pankreas*) in zufällig generierte Fragmente geteilt. Dabei kann die Größe der Fragmente sowie die Fehlerrate der sich anschließenden Reassemblierung durch Wahl der Inkubationsbedingungen beeinflußt werden.

DNAse I Behandung:

In einem Gesamtvolumen von 100 µl werden 3 µg *Pvu* II-Fragmente in 50 mM Tris/HCl pH 7.5, 10 mM $MgCl_2$ bzw. 10 mM $MnCl_2$ und 50 µg/ml BSA für 10-25 min bzw. 1-10 min bei 23 °C mit 0,075 U DNAse I inkubiert. Die Reaktion wird durch 10 minütige Inkubation bei 93 °C terminiert. In Abhängigkeit von der Reaktiondauer entstehen hierbei Fragmente von kleiner 500 Bp bis kleiner 10 Bp. Für den Fall, daß nur ein bestimmter Größenbereich vewendet wird, können diese Fragmente durch selektiven Elektrotransfer auf DEAE-Membran aus Agarosegelen gewonnen werden (nach F.M. Ausubel, *et al*., eds., *Current Protocols in Molecular Biology*, John Wiley and Sons, 1989). Nach Reinigung der Fragmente durch das Qiagen Nucleotide Removal Kit® (Fa. Qiagen) wird die nachfolgende Reassemblierungsreaktion durchgeführt.

Reassemblierungsreaktion

10-30 ng der aus der DNAse I-Restriktion stammenden Fragmente werden in 75 mM Tris/HCl pH 9 0, 20 mM

$(NH_4)_2SO_4$, 0.01% (w/v) Tween® 20, 1,5 mM $MgCl_2$, 0,2 mM dNTPs mit 2U Goldstar Taq-Polymerase (Fa. Eurogentec) in einem Gesamtvolumen von 50 µl folgendem PCR-Programm unterzogen: 2 min 94 °C, 40 Zyklen mit 1 min 94 °C, 2 min 52 °C und 1 min 72 °C abschließend 7 min 72 °C. Die Taq-Polymerase wird nach dem 1 minütigen Denaturierungsschritt des 1. Zyklus zugegeben.

PCR

1 µl aus der Reassemblierungsreaktion wird in eine sich anschließende PCR-Reaktion eingesetzt, die sich mit folgenden Unterschieden, wie für die Reassemblierungsreaktion beschrieben, zusammensetzt: anstelle der DNAse I-generierten Fragmente wird 1 µl der Reassemblierungsreaktion als Matrizen-DNA eingesetzt. Zusätzlich werden die Primer A und B in einer Konzentration von 0,2 mM sowie 10 % Dimethylsulfoxid zugesetzt. Das Zyklenprotokoll lautet wie folgt:
2 min 98 °C, 30 Zyklen zu 1 min 94 °C, 2 min 64 °C, 1 min 72 °C und abschließend 7 min 72 °C; Es werden Parallelansätze durchgeführt. Die in diesen Reaktionen entstandenen PCR-Produkte werden gereinigt, mit den Restriktionendonukleasen Apa I und Bam HI restringiert und wie im Abschnitt "mutagenisierende PCR" von Beispiel 1 beschrieben kloniert.

Ergebnisse (*in vitro*-Rekombination):

Es wurden 12 Klone aus der 1. Generation der durch *mutagenisierende PCR* erhaltenen Mutantenbibliothek (siehe Beispiel 1) für die *in vitro-R*ekombination eingesetzt. Folgende Klone, die im Screening-Test verbesserte Enantioselektivität gezeigt hatten, wurden benutzt:
P1B 01-A2, P1B 01-A6, P1B 01-D2, P1B 01-D5, P1B 01-E1, P1B 01-E4, P1B 01-F3, P1B 01-F11, P1B 01-H1, P1B 01-H3, P1B 01-F12.

Die nach der oben beschriebenen Vorgehensweise rekombinierte DNA dieser Klone wird wie im Abschnitt "mutagenisierende PCR" angegeben kloniert und die Kulturüberstände zum Test auf Enantioselektivität eingesetzt. Es wurden ca. 1000 rekombinante Klone getestet. Die beiden identifizierten Rekombinanten S2A 01-E11 und S2A 02-G3 zeigen gegenüber der besten Mutante der 1. Generation (P1B 01-E4) aus Beispiel 1 eine signifikante Verbesserung der Enantioselektivität.

Tabelle 8

Ausgewählte Lipase-Mutanten mit verbesserter Enantioselektivität (*in vitro*-Rekombination)

| Mutante | $V_{app}$(S) [mOD/ min] | Vapp(R) [mOD/ min] | $E_{app}$ 1) | % ee (nachGC)/% Umsatz | E-Wert 2) (ber. nach GC) |
|---|---|---|---|---|---|
| S2A 01-E11 | 145,6 | 41,6 | 3,5 | 41,0 / 27,0 | 2,8 |
| S2A 02-G3 | 210,8 | 62,0 | 3,4 | 38,0 / 23,0 | 2,5 |

1) $E_{app} = V_{app}(S) / V_{app}(R)$
2) $E = \ln[1-c(1+ee_p)] / \ln[1-c(1-ee_p)]$ mit c = Umsatz, $ee_p$ = ee-Wert des Produktes

Beispiel 3

Ortsgerichtete Sättigungsmutagenese an der Aminosäure-Position 155 der Lipase-Mutante P3B 13-D10:

Plasmide:

| | |
|---|---|
| pMut5 13D10: | BamHI/Apal Fragment (1046Bp) des Gens der Mutante P3B 13D10 der Lipase von P. aeruginosa in pBluescript KS II |
| pMut5ΔAK 13D10: | Deletion des AflIII/Kpnl Fragmentes in pMut5 13D10 |

Ein Fragment des Gens der Lipase der Mutante P3B 13D10 wird unter Verwendung des durch die Endonuclease Xmnl linearisierten Plasmids pMut5 13D10 und folgender PCR-Primer amplifiziert:

A: 5'-GCGCAATTAACCCTCACTAAAGGGAACAAA-3'
M: 5'-GGTACGCAGAATNNNCTGGGCTCGC-3'

N steht dabei für A oder C oder G oder T.

Die Reaktionsbedingungen sind dabei wie folgt: ein 50 µl Reaktionsvolumen enthält 75 mM Tris/HCl pH9.0 (bei 25 °C); 20 mM $(NH_4)_2SO_4$ ;1,5 mM $MgCl_2$; 0,01%(w/v) Tween® 20; 10% (v/v) DMSO; je 10 pmol der Primer ; 0,1 ng der Template-DNA und 2U Taq-Polymerase (Goldstar, Fa. Eurogentec). Das Zyklenprotokol ist wie folgt: Nach 2 min Denaturierung bei 98 °C folgen 30 Zyklen mit 1 min 94 °C, 2 min 64°C, 1 min 72 °C auf einem Robocycler 40 (Fa. Stratagen), gefolgt von 7 min Inkubation bei 72 °C. Die Taq-Polymerase wird nach der Denaturierung des 1. Zyklus zugesetzt. Nach Reinigung der PCR-Produkte mittels Agarosegelelektrophorese und Elution der DNA aus dem Agarosegel unter Verwendung des Nucleospin Extrakt-Kits (Macherey & Nagel) diente diese als Primer (so genannter Megaprimer) in einer nachfolgenden PCR. Dazu wird das Lipasegen auf dem durch die Endonuclease XmnI linearisierten Plasmid pMut5ΔAK 13D10 unter Verwendung folgender PCR primer und den oben beschriebenen Reaktionsbedingungen amplifiziert:

A: 5'-GCGCAATTAACCCTCACTAAAGGGAACAAA-3'
B (Megaprimer): 5'GCGTAATACGACTCACTATAGGGCGAA-3'

Die Reaktionbedingungen und das Zyklenprotokoll sind wie oben beschrieben mit dem Unterschied, daß dem Reaktionansatz 1-10 ng des Megaprimer zugesetzt werden. Die Klonierung der PCR-Produkte erfolgt wie unter Klonierung der PCR-Produkte beschrieben.

Ergebnisse (Sättigungsmutagenese 3. Generation. P3B13-D10)

Aus der Mutantenbibliothek der Sättigungsmutagenese (3. Generation, P3B 13-D10) wurden ca. 900 Klone zum Screening-Test herangezogen. Dabei wurde 1 Mutante (P4BSF 03-G10) mit einer gegenüber der Mutante P3B 13-D10 verbesserten Enantioselektivität identifiziert. Diese wurde durch GC-Analyse weiter untersucht.

Tabelle 9

| Ausgewählte Lipase-Mutante mit verbesserter Enantioselektivität (3. Generation, P3B 13-D10) | | | | | |
|---|---|---|---|---|---|
| Mutante | $V_{app}$(S) [mOD/min] | Vapp(R) [mOD/min] | $E_{app}$ [1]) | % ee (nachGC)/% Umsatz | E-Wert [2]) (berechnet nach GC) |
| P4BSF 03-G10 | 3 84,7 | 25,3 | 15,2 | 87,3 / 19,0 | 20,4 |

[1]) $E_{app} = V_{app}(S) / V_{app}(R)$
[2]) $E = \ln[1-c(1+ee_p)] / \ln[1-c(1-ee_p)]$ mit c = Umsatz, $ee_p$ = ee-Wert des Produktes

Sequenzierung der positiven Mutanten

Durch Sequenzierung der positiven Mutanten konnten die Mutationen im Lipase-Gen lokalisiert werden (siehe Figur 2). Nach Zuordnung der Basentripletts zu der entsprechenden Aminosäure ergab sich gegenüber der Mutante P3B 13-D10 folgender Aminosäure-Austausch :

P4BSF 03-G10: L155F ($Leu_{155} \rightarrow Phe_{155}$)

Die Mutante P4BSF 03-G10 wurde unter der Bezeichnung DSM 12 321 am 20.07.1998 bei der DSMZ - Deutsche Sammlung von Mikroorganismen und Zellkulturen GmbH, D-38124 Braunschweig, Mascheroder Weg 1b, hinterlegt.

## Patentansprüche

1. Verfahren zur Herstellung und Identifizierung von Hydrolase-Mutanten mit verbesserten Eigenschaften hinsichtlich Stereo- oder Regioselektivität, **dadurch gekennzeichnet, dass**

   a) ein Ausgangs-Hydrolasegen mittels einer modifizierten Polymerase-Kettenreaktion (PCR) mutagenisiert wird, wobei durch Einstellen der $Mg^{2+}$-, $Mn^{2+}$- und der Desoxynucleotid-Konzentrationen eine durchschnittliche Mutationsfrequenz von 1-2 Basenaustauschen bezogen auf das zu mutagenisierende Hydrolasegen eingestellt wird, sowie durch Einstellung der Zyklenanzahl die Gesamtanzahl der Mutationen in der amplifizierten DNA eingestellt wird,
   b) gegebenenfalls ein oder mehrere gemäß Schritt a) mutierte Hydrolasegene oder Gemische von einem oder mehreren Ausgangs-Hydrolasegenen und einem oder mehreren gemäß Schritt a) mutierten Hydrolasegenen durch enzymatische Fragmentierung dieser Gene und nachfolgender enzymatischer Reassemblierung der entstandenen Fragmente zu vollständig rekombinierten Hydrolasegenen mutagenisiert werden,

c) die gemäß Schritt a) oder b) erhaltenen mutierten Hydrolasegene in einen Wirtsorganismus transformiert werden und

d) Hydrolase-Mutanten mit verbesserten Eigenschaften hinsichtlich Stereo- oder Regioselektivität, die von in Schritt c) erhaltenen Transformanten exprimiert werden, durch ein Testverfahren identifiziert werden.

**2.** Verfahren gemäß Anspruch 1, wobei in Schritt a) als Ausgangs-Hydrolasegen ein in einer zuvor gemäß Anspruch 1 durchgeführten PCR mutagenisiertes Hydrolasegen eingesetzt wird.

**3.** Verfahren gemäß Anspruch 1, wobei in Schritt b) die enzymatische Fragmentierung der Hydrolasegene mit einer Desoxyribonuclease durchgeführt wird.

**4.** Verfahren gemäß Anspruch 1, wobei in Schritt b) die Reassemblierung der Fragmente enzymatisch mit Hilfe einer thermostabilen DNA-Polymerase unter Verwendung eines zyklischen Temperaturprogrammes, in welchem die Parameter Temperatur und Zyklendauer eingestellt werden, durchgeführt wird.

**5.** Verfahren gemäß Anspruch 1, wobei in Schritt b) während der enzymatischen Reassemblierung durch Einstellen der $Mg^{2+}$-, $Mn^{2+}$- und der Desoxynucleotidkonzentration die Mutationsfrequenz eingestellt wird.

**6.** Verfahren gemäß Anspruch 1, wobei in Schritt b) die vollständig rekombinierten Hydrolasegene nach Beeendigung der Reassemblierungsreaktion durch eine Polymerase-Kettenreaktion amplifiziert werden.

**7.** Verfahren gemäß Anspruch 1, wobei in Schritt b) entweder aus Schritt a) gemäß Anspruch 1 erhaltene modifizierte Hydrolasegene oder mehrere gemäß Anspruch 2 mutagenisierte Hydrolasegene der Fragmentierung und Reassemblierung unterworfen werden.

**8.** Verfahren gemäß Anspruch 1, wobei in Schritt b) zur Reassemblierung zusätzlich synthetisch hergestellte Genfragmente eingesetzt werden.

**9.** Verfahren gemäß Anspruch 1, wobei in Schritt b) zur Reassemblierung Hydrolasegen-Fragmente aus unterschiedlichen Organismen eingesetzt werden können, die eine Sequenzhomologie von mindestens 60% zueinander besitzen.

**10.** Verfahren gemäß Anspruch 1 oder 5, wobei die Hydrolase-Mutanten Lipase- oder Esterasemutanten sind und die Konzentration der Magnesium-Ionen 1,5 bis 8,0 mM, bevorzugt 5,8 bis 6,4 mM ist und die Konzentration der Mangan-Ionen 0,0 bis 3,0 mM, bevorzugt < 0,3 mM ist.

**11.** Verfahren gemäß Anspruch 1 oder 5, wobei die Hydrolase-Mutanten Lipase- oder Esterasemutanten sind und die Konzentration der Desoxynucleotidtriphosphate 0,05 mM bis 1,0 mM, bevorzugt 0,2 mM beträgt.

**12.** Verfahren gemäß Anspruch 1, wobei in Schritt d) der Test auf Stereo- oder Regioselektivität über die Bestimmung der zeitlichen Konzentrationsänderung von freien Fettsäuren oder Bernsteinsäure erfolgt, wobei die entsprechenden stereo- oder regioisomeren Carbonsäureester oder -amide mit Hilfe der Hydrolase-Mutanten in getrennten Gefäßen zu freien Fettsäuren oder Bernsteinsäure hydrolysiert werden.

**13.** Verfahren gemäß Anspruch 1, wobei in Schritt d) der Test auf Stereo- oder Regioselektivität über die Messung der Radioaktivität erfolgt, wobei die Hydrolase-Mutanten mit in einer funktionellen Gruppe unterschiedlich radioaktiv markierten Stereo- oder Regioisomeren umgesetzt werden und wobei das Gemisch der Stereo- oder Regioisomeren trägerfixiert ist.

**14.** Verfahren gemäß Anspruch 13, wobei eines der Stereo- oder Regioisomere des an den Träger gebundenen Gemisches der isomeren Verbindung mit dem Radioisotop $^{3}H$ und das andere Stereo- oder Regioisomer mit dem Radioisotop $^{14}C$ markiert ist.

**15.** Verfahren gemäß Anspruch 1, wobei in Schritt d) der Test auf Stereoselektivität über die kapillarelektrophoretische Bestimmung der Reaktionsprodukte und -edukte einer Testreaktion erfolgt, wobei die Trennung der stereoisomeren Reaktionsprodukte und -edukte in chiral-modifizierten Kapillaren durchgeführt wird.

**16.** Verfahren gemäß Anspruch 12 bis 15, wobei mehrere Reaktionen parallel in Mikrotiterplatten durchgeführt werden.

**17.** Verfahren nach Anspruch 1, wobei in den in Schritt d) identifizierten Mutanten mit verbesserten Eigenschaften durch Sequenzierung die Position des Codons, das für die veränderte Aminosäure codiert, lokalisiert wird, nachfolgend mittels ortsgerichteter Sättigungsmutagenese einen Satz Hydrolasegene mit allen für diese Position möglichen Codons erzeugt wird, und die so erhaltenen mutierten Hydrolasegene analog Schritten c) und d) von Anspruch 1 weiterbehandelt werden.

**18.** Verfahren nach Anspruch 17, wobei die Lokalisierung der Position des Codons, das für die veränderte Aminosäure codiert, durch DNA-Sequenzierung erfolgt.

**19.** Lipase-Mutante mit verbesserter Stereoselektivität hinsichtlich chiraler Carbonsäuren oder chiral. COOH-funktionalisierten Verbindungen als die in Fig. 2 gezeigte Ausgangslipase aus dem Stamm *P. aeruginosa*, erhältlich durch Mutagenisierung der Ausgangslipase mit einem Verfahren gemäß einen oder mehreren der Ansprüche 1 bis 18.

**20.** Lipase-Mutante gemäß Anspruch 19, die durch Exprimieren der Transformanten P1B 01-E4 (DSM 11 658), P2B 08-H3 (DSM 11 659), P3B 13-D10 (DSM 11 660), P4B 04-H3 (DSM 12 322), P5B 14-C11 (DSM 12 320) oder P4BSF 03-G10 (DSM 12 321) erhältlich ist.

**21.** Lipase-Mutante gemäß Anspruch 19, die die Aminosäuresequenz der in SEQ ID NOs 4, 6, 8, 12, 14, 16 oder 18 gezeigten reifen Proteine aufweist.

**22.** DNA-Sequenz, die für eine Lipase-Mutante gemäß einem oder mehreren der Ansprüche 19 bis 21 kodiert.

**23.** DNA-Sequenz gemäß Anspruch 22, die eine in SEQ ID NOs 3, 5, 7, 11, 13, 15 oder 17 gezeigte DNA-Sequenz umfasst.

**24.** Vektor, umfassend eine DNA-Sequenz gemäß Anspruch 22 oder 23.

**25.** Transformante, umfassend eine DNA-Sequenz gemäß Anspruch 22 oder 23 und/oder einen Vektor gemäß Anspruch 25.

**26.** Transformante gemäß Anspruch 25, die Transformante P1B 01-E4 (DSM 11 658), P2B 08-H3 (DSM 11 659), P3B 13-D10 (DSM 11 660), P4B 04-H3 (DSM 12 322), P5B 14-C11 (DSM 12 320) oder P4BSF 03-G10 (DSM 12 321) ist.

**27.** Verfahren zur Herstellung der Lipasemutanten gemäß Anspruch 19, umfassend das Kultivieren einer Transformanten gemäß Anspruch 25 oder 26.

## Claims

**1.** A process for the preparation and identification of hydrolase mutants having improved properties with respect to stereo- or regioselectivity, **characterized in that**

a) a starting hydrolase gene is mutagenized by a modified polymerase chain reaction (PCR), wherein an average mutation rate of 1-2 base exchanges, based on the hydrolase gene to be mutagenized, is adjusted by adjusting the concentrations of $Mg^{2+}$, $Mn^{2+}$ and of the deoxynucleotides, and the total number of mutations in the amplified DNA is adjusted by adjusting the number of cycles;

b) optionally one or more starting hydrolase genes mutated according to step a) or mixtures of one or more starting hydrolase genes and one or more hydrolase genes mutated according to step a) are mutagenized by enzymatically fragmenting said genes followed by enzymatic reassembly of the fragments produced to give complete recombinant hydrolase genes;

c) the mutated hydrolase genes obtained according to step a) or b) are transformed into a host organism; and

d) hydrolase mutants having improved properties with respect to stereo- or regioselectivity, expressed by transformants obtained in step c), are identified by a test method.

**2.** The process according to claim 1, wherein a hydrolase gene mutagenized in a PCR previously performed according

to claim 1 is used as the starting hydrolase gene in step a).

**3.** The process according to claim 1, wherein the enzymatic fragmentation of the hydrolase genes in step b) is performed using a deoxyribonuclease.

**4.** The process according to claim 1, wherein the reassembly of the fragments in step b) is effected enzymatically by means of a thermostable DNA polymerase using temperature cycles in which the parameters of temperature and duration of cycles are adjusted.

**5.** The process according to claim 1, wherein the mutation rate is adjusted during the enzymatic reassembly in step b) by adjusting the concentrations of $Mg^{2+}$, $Mn^{2+}$ and of the deoxynucleotides.

**6.** The process according to claim 1, wherein the completely recombined hydrolase genes are amplified by a polymerase chain reaction in step b) after completion of the reassembly reaction.

**7.** The process according to claim 1, wherein in step b) either modified hydrolase genes obtained from step a) according to claim 1 or several hydrolase genes mutagenized according to claim 2 are subjected to fragmentation and reassembly.

**8.** The process according to claim 1, wherein synthetically prepared gene fragments are additionally used for the reassembly in step b).

**9.** The process according to claim 1, wherein hydrolase gene fragments from different organisms sharing a sequence homology of at least 60% can be used for the reassembly in step b).

**10.** The process according to claim 1 or 5, wherein the hydrolase mutants are lipase or esterase mutants, and the concentration of the magnesium ions is from 1.5 to 8.0 mM, preferably from 5.8 to 6.4 mM, and the concentration of the manganese ions is from 0.0 to 3.0 mM, preferably $< 0.3$ mM.

**11.** The process according to claim 1 or 5, wherein the hydrolase mutants are lipase or esterase mutants, and the concentration of the deoxynucleotide triphosphates is from 0.05 to 1.0 mM, preferably 0.2 mM.

**12.** The process according to claim 1, wherein the test for stereo- or regioselectivity in step d) is effected through determination of the change in time of concentrations of free fatty acids or succinic acid, wherein the corresponding stereo- or regioisomeric carboxylic acid esters or amides are hydrolyzed in separate vessels by means of the hydrolase mutants to form free fatty acids or succinic acid.

**13.** The process according to claim 1, wherein the test for stereo- or regioselectivity in step d) is effected through measuring the radioactivity, wherein the hydrolase mutants are reacted with stereo- or regioisomers having different radioactive labels in one functional group, and wherein the mixture of the stereo- or regioisomers is fixed on a support.

**14.** The process according to claim 13, wherein one of the stereo- or regioisomers of the support-bound mixture of isomeric compounds is labeled with the radioisotope $^{3}H$, and the other stereo- or regioisomer is labeled with the radioisotope $^{14}C$.

**15.** The process according to claim 1, wherein the test for stereoselectivity in step d) is effected through the capillary-electrophoretic determination of the reaction products and educts of a test reaction, the separation of the stereoisomeric reaction products and educts being performed in chirally modified capillaries.

**16.** The process according to claims 12 to 15, wherein several reactions are performed in parallel in microtitration plates.

**17.** The process according to claim 1, wherein the position of the codon coding for the changed amino acid is localized by sequencing in the mutants having improved properties identified in step d), followed by generating a set of hydrolase genes with all possible codons for this position by means of site-directed saturation mutagenesis, and the mutated hydrolase genes thus obtained are further treated by analogy with steps c) and d) of claim 1.

**18.** The process according to claim 17, wherein the localization of the position of the codon coding for the changed amino acid is effected through DNA sequencing.

**19.** A lipase mutant with improved stereoselectivity with respect to chiral carboxylic acids or chiral COOH-functionalized compounds as compared to the starting lipase shown in Figure 2 from the strain *P. aeruginosa*, obtainable by mutagenization of the starting lipase by a process according to one or more of claims 1 to 18.

**20.** The lipase mutant according to claim 19 which is obtainable by expression from the transformants P1B 01-E4 (DSM 11 658), P2B 08-H3 (DSM 11 659), P3B 13-D10 (DSM 11 660), P4B 04-H3 (DSM 12 322), P5B 14-C11 (DSM 12 320) or P4BSF 03-G10 (DSM 12 321).

**21.** The lipase mutant according to claim 19 which has the amino acid sequence of the mature proteins shown in SEQ ID NOs. 4, 6, 8, 12, 14, 16 or 18.

**22.** A DNA sequence coding for a lipase mutant according to one or more of claims 19 to 21.

**23.** The DNA sequence according to claim 22 which comprises a DNA sequence as shown in SEQ ID NOs. 3, 5, 7, 11, 13, 15 or 17.

**24.** A vector comprising a DNA sequence according to claim 22 or 23.

**25.** A transformant comprising a DNA sequence according to claim 22 or 23 and/or a vector according to claim 25.

**26.** The transformant according to claim 25 which is transformant P1B 01-E4 (DSM 11 658), P2B 08-H3 (DSM 11 659), P3B 13-D10 (DSM 11 660), P4B 04-H3 (DSM 12 322), P5B 14-C11 (DSM 12 320) or P4BSF 03-G10 (DSM 12 321).

**27.** A process for the preparation of the lipase mutants according to claim 19, comprising the culturing of a transformant according to claim 25 or 26.

## Revendications

**1.** Procédé de fabrication et d'identification de mutants d'hydrolase présentant des propriétés améliorées pour ce qui concerne la stéréo- et la régio-sélectivité, **caractérisé en ce que**

a) un gène d'hydrolase de départ est soumis à une mutagénèse au moyen d'une réaction en chaîne par la polymérase (PCR) modifiée, où, par ajustement des concentrations en $Mg^{2+}$, $Mn^{2+}$ et désoxynucléotide, on règle une fréquence de mutation moyenne de 1-2 échanges de base, par rapport au gène d'hydrolase à soumettre à mutagénèse, et on règle, par ajustement du nombre de cycles, le nombre total des mutations dans l'ADN amplifié,
b) éventuellement un ou plusieurs gènes d'hydrolase ayant subi une mutation selon l'étape a) ou des mélanges d'un ou de plusieurs gènes d'hydrolase de départ et d'un ou de plusieurs gènes d'hydrolase ayant subi une mutation selon l'étape a) subissent une mutagénèse en gènes d'hydrolase entièrement recombinés par fragmentation enzymatique de ces gènes et ré-assemblage enzymatique ultérieur des fragments obtenus,
c) les gènes d'hydrolase ayant subi une mutation, obtenus selon l'étape a) ou b), sont transformés dans un organisme hôte, et
b) les mutants d'hydrolase présentant des propriétés améliorées pour ce qui concerne la stéréo- ou la régio-sélectivité, qui sont exprimés par les transformants obtenus à l'étape c), sont identifiés par une méthode d'essai.

**2.** Procédé selon la revendication 1, dans lequel, à l'étape a), on utilise en tant que gène d'hydrolase de départ un gène d'hydrolase ayant subi une mutagénèse lors d'une PCR préalable réalisée selon la revendication 1.

**3.** Procédé selon la revendication 1, dans lequel, à l'étape b), la fragmentation enzymatique des gènes d'hydrolase est réalisée avec une désoxyribonucléase.

**4.** Procédé selon la revendication 1, dans lequel, à l'étape b), le ré-assemblage des fragments est réalisé par voie enzymatique à l'aide d'une ADN-polymérase thermostable en utilisant un programme de température cyclique,

dans lequel les paramètres de température et de durée de cycle sont ajustés.

**5.** Procédé selon la revendication 1, dans lequel, à l'étape b), pendant le ré-assemblage enzymatique, on ajuste la fréquence de mutation en réglant la concentration en $Mg^{2+}$, $Mn^{2+}$ et désoxynucléotide.

**6.** Procédé selon la revendication 1, dans lequel, à l'étape b), les gènes d'hydrolase entièrement recombinés sont amplifiés après la fin de la réaction de ré-assemblage, par une réaction en chaîne par la polymérase.

**7.** Procédé selon la revendication 1, dans lequel, à l'étape b), les gènes d'hydrolase modifiés, obtenus à partir de l'étape a) selon la revendication 1, ou plusieurs gènes d'hydrolase ayant subi une mutagénèse selon la revendication 2 sont soumis à fragmentation et ré-assemblage.

**8.** Procédé selon la revendication 1, dans lequel, à l'étape b), on utilise de plus pour le ré-assemblage des fragments de gène fabriqués par voie synthétique.

**9.** Procédé selon la revendication 1, dans lequel, à l'étape b), pour le ré-assemblage, on utilise des fragments de gènes d'hydrolase de différents organismes, qui possèdent les uns par rapport aux autres une homologie de séquence d'au moins 60 %.

**10.** Procédé selon la revendication 1 ou 5, dans lequel les mutants d'hydrolase sont des mutants de lipase ou d'estérase, et la concentration en ions magnésium est de 1,5 à 8,0 mM, de préférence de 5,8 à 6,4 mM, et la concentration en ions manganèse est de 0,0 à 3,0 mM, et de préférence inférieure à 0,3 mM.

**11.** Procédé selon la revendication 1 ou 5, dans lequel les mutants d'hydrolase sont des mutants de lipase ou d'estérase et la concentration en désoxynucléotide-triphosphate va de 0,05 mM à 1,0 mM, et est de préférence égale à 0,2 mM.

**12.** Procédé selon la revendication 1, dans lequel, à l'étape d), l'essai de stéréo- ou de régio-sélectivité est réalisé en déterminant la modification de concentration en acides gras libres ou en acide succinique en fonction du temps, les esters ou amides d'acides carboxyliques stéréo- ou régio-isomères correspondants étant hydrolysés à l'aide des mutants d'hydrolase dans des récipients séparés en acides gras libres ou en acide succinique.

**13.** Procédé selon la revendication 1, dans lequel, à l'étape d), l'essai de stéréo- ou de régio-sélectivité est réalisé en mesurant la radioactivité, les mutants d'hydrolase étant mis à réagir avec des stéréo- ou régio-isomères différemment radiomarqués dans un groupe fonctionnel, et le mélange de stéréo- ou de régio-isomères étant fixé sur un support.

**14.** Procédé selon la revendication 13, dans lequel un des stéréo- ou régio-isomères du mélange du composé isomère lié au support est marqué avec le radio-isotope $^{3}H$ et l'autre stéréo- ou régio-isomère avec le radio-isotope $^{14}C$.

**15.** Procédé selon la revendication 1, dans lequel, à l'étape d), l'essai de stéréo-sélectivité est réalisé en déterminant par électrophorèse capillaire les produits et éduits de la réaction d'une réaction d'essai, la séparation des produits et éduits stéréo-isomères de la réaction étant réalisée dans des capillaires modifiés de façon chirale.

**16.** Procédé selon la revendication 12 à 15, dans lequel plusieurs réactions sont conduites en parallèle dans des plaques de microtitration.

**17.** Procédé selon la revendication 1, dans lequel, dans les mutants identifiés à l'étape d) présentant des propriétés améliorées, on localise par séquençage la position du codon qui code pour l'acide aminé modifié, après quoi, au moyen d'une mutagénèse de saturation dirigée, on produit un jeu de gènes d'hydrolase avec tous les codons possibles pour cette position, et les gènes d'hydrolase mutés ainsi obtenus sont complémentairement traités de façon analogue aux étapes c) et d) de la revendication 1.

**18.** Procédé selon la revendication 17, dans lequel la localisation de la position du codon qui code pour l'acide aminé modifié est réalisée par séquençage d'ADN.

**19.** Mutants de lipase présentant une stéréo-sélectivité améliorée pour ce qui concerne les acides carboxyliques chiraux ou les composés chiraux à fonctionnalité COOH par rapport à la lipase de départ montrée à la fig. 2, issue

de la souche *P. aeruginosa*, pouvant être obtenus par mutagénèse de la lipase de départ avec un procédé selon une ou plusieurs des revendications 1 à 18.

**20.** Mutants de lipase selon la revendication 19, qui peuvent être obtenus par expression des transformants P1B 01-E4 (DSM 11 658), P2B 08-H3 (DSM 11 659), P3B 13-D10 (DSM 11 660), P4B 04-H3 (DSM 12 322), P5B 14-C11 (DSM 12 320) ou P4BSF 03-G10 (DSM 12 321).

**21.** Mutants de lipase selon la revendication 19, qui présentent la séquence d'acides aminés des protéines matures montrées dans les ID SEQ N° 4, 6, 8, 12, 14, 16 ou 18.

**22.** Séquence d'ADN qui code pour un mutant de lipase selon une ou plusieurs des revendications 19 à 21.

**23.** Séquence d'ADN selon la revendication 22, qui comporte une séquence d'ADN montrée dans les ID SEQ N° 3, 5, 7,11, 13, 15 ou 17.

**24.** Vecteur comprenant une séquence d'ADN selon la revendication 22 ou 23.

**25.** Transformant comprenant une séquence d'ADN selon 1a revendication 22 ou 23 et/ou un vecteur selon la revendication 25.

**26.** Transformant selon la revendication 25, qui est le transformant P1B 01-E4 (DSM 11 658), P2B 08-H3 (DSM 11 659), P3B 13-D10 (DSM 11 660), P4B 04-H3 (DSM 12 322), P5B 14-C11 (DSM 12 320) ou P4BSF 03-G10 (DSM 12 321).

**27.** Procédé de fabrication de mutants de lipase selon la revendication 19, comprenant la culture d'un transformant selon la revendication 25 ou 26.

Fig. 1

Wildtyp

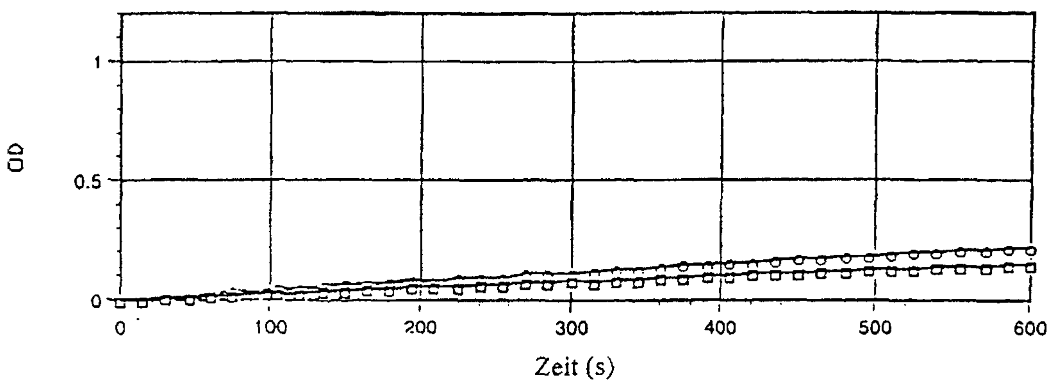


$V_{app}$ (S) = 21,8 $V_{app}$ (R) = 14,9 $E_{app}$ = 1,5

PlB 01-E4

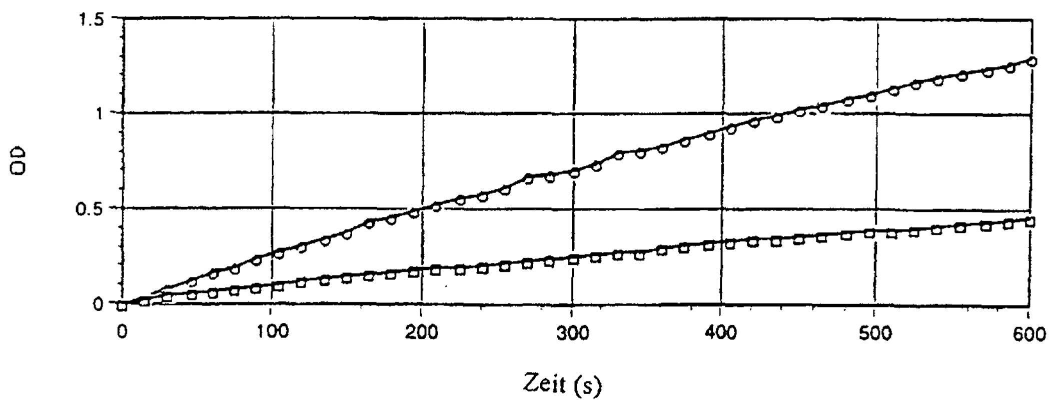


$V_{app}$ (S) = 128,4 $V_{app}$ (R) = 43,2 $E_{app}$ = 3,0

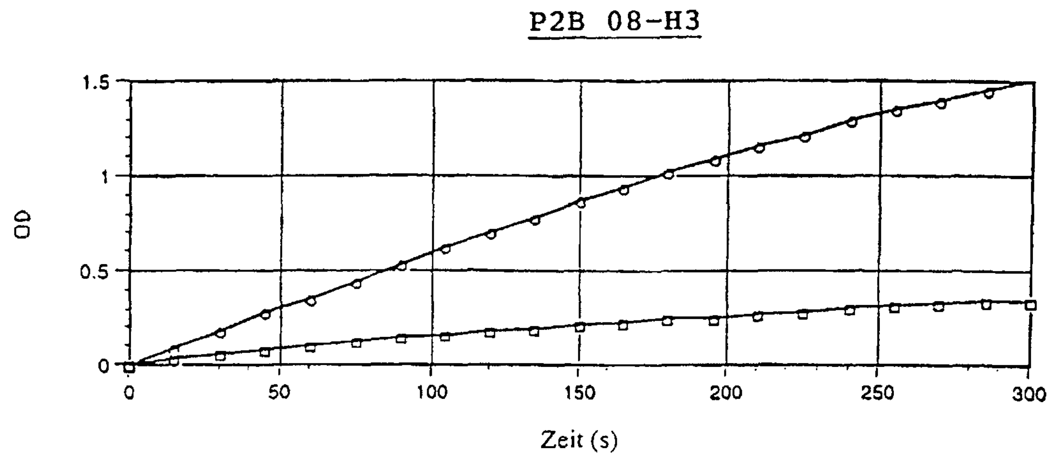


$V_{app}$ (S) = 310,8 $V_{app}$ (R) = 67,4 $E_{app}$ = 4,6

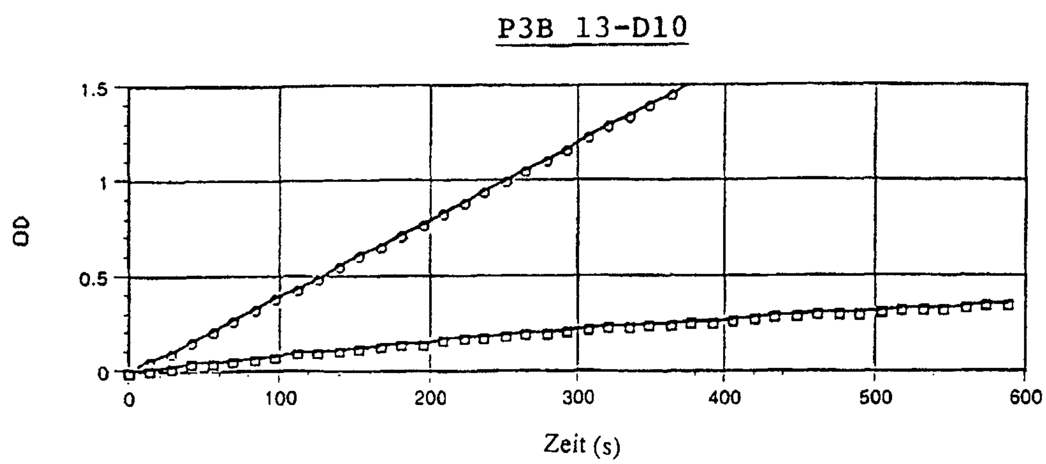


$V_{app}$ (S) = 241,1 $V_{app}$ (R) = 35,2 $E_{app}$ = 6,9

P4B 04-H3

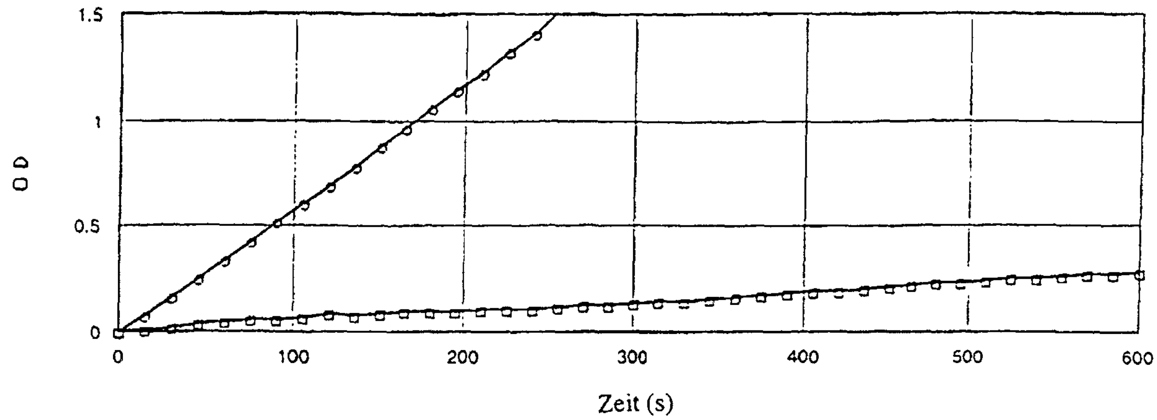


$V_{app}$ (S) = 355,6  $V_{app}$ (R) = 26,5  $E_{app}$ = 13,4

P5B 14-C11

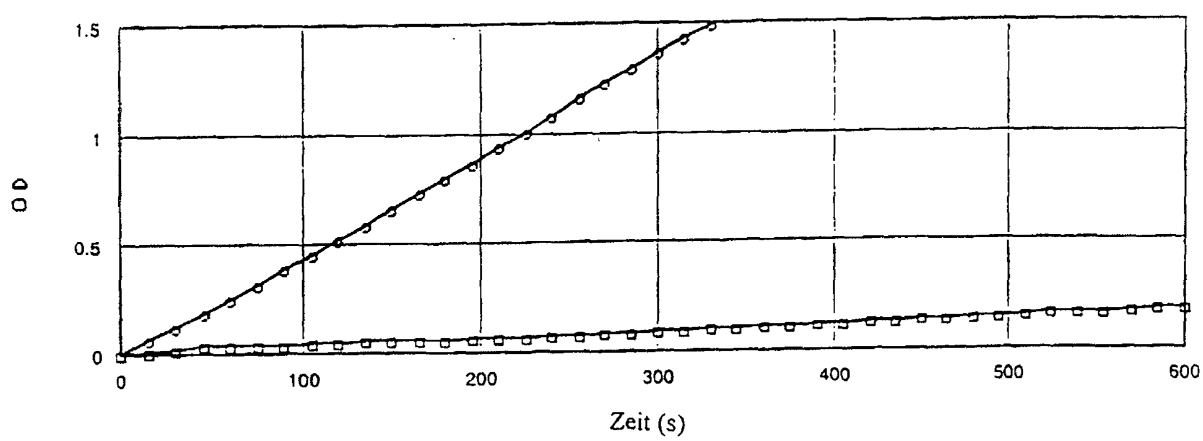


$V_{app}$ (S) = 275,9  $V_{app}$ (R) = 17,3  $E_{app}$ = 15,9

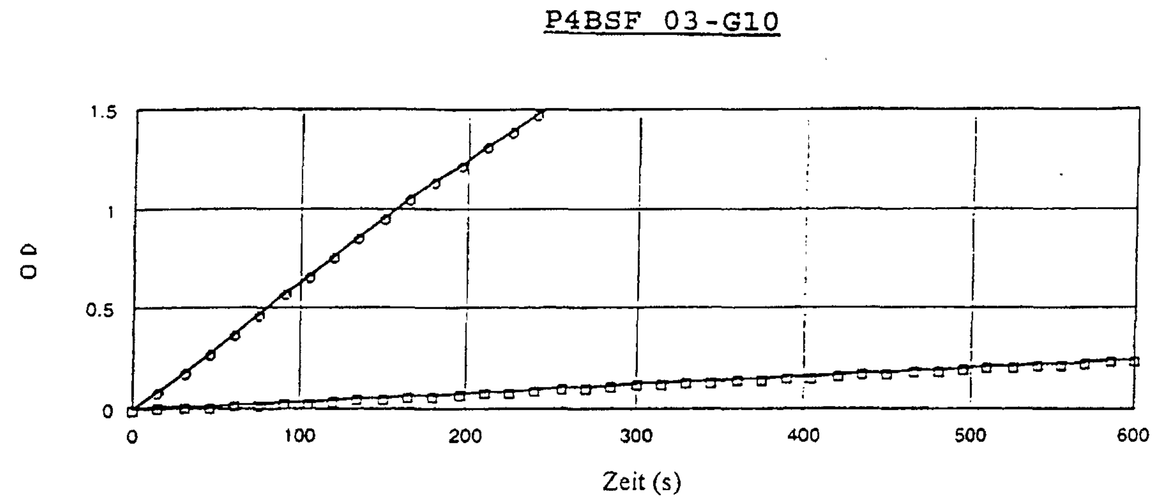


$V_{app}$ (S) = 384,7 $V_{app}$ (R) = 25,3 $E_{app}$ = 15.2

## Fig. 2

| | 1–19/20 (10) | |
|---|---|---|
| Wildtyp | G G A T C C C C [-] G G T T C T C C C G G | 19 |
| P1B 01-H1 | G G A T C C C C C G G T T C T C C C G G | 20 |
| P1B 01-E4 | G G A T C C C C C G G T T C T C C C G G | 20 |
| P2B 08-H3 | G G A T C C C C C G G T T C T C C C G G | 20 |
| P3B 13-D10 | G G A T C C C C C G G T T C T C C C G G | 20 |
| P4B 04-H3 | G G A T C C C C C G G T T C T C C C G G | 20 |
| P5B 14-C11 | G G A T C C C C C G G T T C T C C C G G | 20 |
| P4BSF 03-G10 | G G A T C C C C C G G T T C T C C C G G | 20 |

| | 20 … 30 | |
|---|---|---|
| Wildtyp | A A G G A T T C G G G C G A T G G C T G | 39 |
| P1B 01-H1 | A A G G A T T C G G G C G A T G G C T G | 40 |
| P1B 01-E4 | A A G G A T T C G G G C G A T G G C T G | 40 |
| P2B 08-H3 | A A G G A T T C G G G C G A T G G C T G | 40 |
| P3B 13-D10 | A A G G A T T C G G G C G A T G G C T G | 40 |
| P4B 04-H3 | A A G G A T T C G G G C G A T G G C T G | 40 |
| P5B 14-C11 | A A G G A T T C G G G C G A T G G C T G | 40 |
| P4BSF 03-G10 | A A G G A T T C G G G C G A T G G C T G | 40 |

| | 40 … 50 | |
|---|---|---|
| Wildtyp | G C A G G A C G C G C C C C T C G G C C | 59 |
| P1B 01-H1 | G C A G G A C G C G C C C C T C G G C C | 60 |
| P1B 01-E4 | G C A G G A C G C G C C C C T C G G C C | 60 |
| P2B 08-H3 | G C A G G A C G C G C C C C T C G G C C | 60 |
| P3B 13-D10 | G C A G G A C G C G C C C C T C G G C C | 60 |
| P4B 04-H3 | G C A G G A C G C G C C C C T C G G C C | 60 |
| P5B 14-C11 | G C A G G A C G C G C C C C T C G G C C | 60 |
| P4BSF 03-G10 | G C A G G A C G C G C C C C T C G G C C | 60 |

| | 60 … 70 | |
|---|---|---|
| Wildtyp | C C A T C A A C C T G A G A T G A G A A | 79 |
| P1B 01-H1 | C C A T C A A C C T G A G A T G A G A A | 80 |
| P1B 01-E4 | C C A T C A A C C T G A G A T G A G A A | 80 |
| P2B 08-H3 | C C A T C A A C C T G A G A T G A G A A | 80 |
| P3B 13-D10 | C C A T C A A C C T G A G A T G A G A A | 80 |
| P4B 04-H3 | C C A T C A A C C T G A G A T G A G A A | 80 |
| P5B 14-C11 | C C A T C A A C C T G A G A T G A G A A | 80 |
| P4BSF 03-G10 | C C A T C A A C C T G A G A T G A G A A | 80 |

| | 80 … 90 | |
|---|---|---|
| Wildtyp | C A A C A T G A A G A A G A A G T C T C | 99 |
| P1B 01-H1 | C A A C A T G A A G A A G A A G T [A] T C | 100 |
| P1B 01-E4 | C A A C A T G A A G A A G A A G T C T C | 100 |
| P2B 08-H3 | C A A C A T G A A G A A G A A G T C T C | 100 |
| P3B 13-D10 | C A A C A T G A A G A A G A A G T C T C | 100 |
| P4B 04-H3 | C A A C A T G A A G A A G A A G T C T C | 100 |
| P5B 14-C11 | C A A C A T G A A G A A G A A G T C T C | 100 |
| P4BSF 03-G10 | C A A C A T G A A G A A G A A G T C T C | 100 |

| | 100 … 110 | |
|---|---|---|
| Wildtyp | T G C T C C C C C T C G G C C T G G C C | 119 |
| P1B 01-H1 | T G C T C C C C C T C G G C C T G G C C | 120 |
| P1B 01-E4 | T G C T C C C C C T C G G C C T G G C C | 120 |
| P2B 08-H3 | T G C T C C C C C T C G G C C T G G C C | 120 |
| P3B 13-D10 | T G C T C C C C C T C G G C C T G G C C | 120 |
| P4B 04-H3 | T G C T C C C C C T C G G C C T G G C C | 120 |
| P5B 14-C11 | T G C T C C C C C T C G G C C T G G C C | 120 |
| P4BSF 03-G10 | T G C T C C C C C T C G G C C T G G C C | 120 |

| | 120 | | 130 | |
|---|---|---|---|---|
| Wildtyp | A T C G G T C T C G C C T C T C T C G C | | | 139 |
| P1B 01-H1 | A T C G G T C T C G C C T C T C T C G C | | | 140 |
| P1B 01-E4 | A T C G G T C T C G C C T C T C T C G C | | | 140 |
| P2B 08-H3 | A T C G G T C T C G C C T C T C T C G C | | | 140 |
| P3B 13-D10 | A T C G G T C T C G C C T C T C T C G C | | | 140 |
| P4B 04-H3 | A T C G G T C T C G C C T C T C T C G C | | | 140 |
| P5B 14-C11 | A T C G G T C T C G C C T C T C T C G C | | | 140 |
| P4BSF 03-G10 | A T C G G T C T C G C C T C T C T C G C | | | 140 |

| | 140 | | 150 | |
|---|---|---|---|---|
| Wildtyp | T G C C A G C C C T C T G A T C C A G G | | | 159 |
| P1B 01-H1 | T G C C A G C C C T C T G A T C C A G G | | | 160 |
| P1B 01-E4 | T G C C A G C C C T C T G A T C C A G G | | | 160 |
| P2B 08-H3 | T G C C A G C C C T C T G A T C C A G G | | | 160 |
| P3B 13-D10 | T G C C A G C C C T C T G A T C C A G G | | | 160 |
| P4B 04-H3 | T G C C A G C C C T C T G A T C C A G G | | | 160 |
| P5B 14-C11 | T G C C A G C C C T C T G A T C C A G G | | | 160 |
| P4BSF 03-G10 | T G C C A G C C C T C T G A T C C A G G | | | 160 |

| | 160 | | 170 | |
|---|---|---|---|---|
| Wildtyp | C C A G C A C C T A C A C C C A G A C C | | | 179 |
| P1B 01-H1 | C C A G C A C C T A C A C C C A G A C C | | | 180 |
| P1B 01-E4 | C C A G C A C C T A C A C C C A G A C C | | | 180 |
| P2B 08-H3 | C C A G C A C C T A C A C C C A G A C C | | | 180 |
| P3B 13-D10 | C C A G C A C C T A C A C C C A G A C C | | | 180 |
| P4B 04-H3 | C C A G C A C C T A C A C C C A G A C C | | | 180 |
| P5B 14-C11 | C C A G C A C C T A C A C C C A G A C C | | | 180 |
| P4BSF 03-G10 | C C A G C A C C T A C A C C C A G A C C | | | 180 |

| | 180 | | 190 | |
|---|---|---|---|---|
| Wildtyp | A A A T A C C C C A T C G T G C T G G C | | | 199 |
| P1B 01-H1 | A A A T A C C C C A T C G T G C T G G C | | | 200 |
| P1B 01-E4 | A A A T A C C C C A T C G T G C T G G C | | | 200 |
| P2B 08-H3 | A A A T A C C C C A T C G T G C T G G C | | | 200 |
| P3B 13-D10 | A A A T A C C C C A T C G T G C T G G C | | | 200 |
| P4B 04-H3 | A A A T A C C C C A T C G T G C T G G C | | | 200 |
| P5B 14-C11 | A A A T A C C C C A T C G T G C T G G C | | | 200 |
| P4BSF 03-G10 | A A A T A C C C C A T C G T G C T G G C | | | 200 |

| | 200 | | 210 | |
|---|---|---|---|---|
| Wildtyp | C C A C G G C A T G C T C G G C T T C G | | | 219 |
| P1B 01-H1 | C C A C G G C A T G C T C G G C T T C G | | | 220 |
| P1B 01-E4 | C C A C G G C A T G C T C G G C T T C G | | | 220 |
| P2B 08-H3 | C C A C G G C A T G C T C G G C T T C G | | | 220 |
| P3B 13-D10 | C C A C G G C A T G C T C G G C T T C G | | | 220 |
| P4B 04-H3 | C C A C G G C A T G C T C G G C T T C G | | | 220 |
| P5B 14-C11 | C C A C G G C A T G C T C G G C T T C G | | | 220 |
| P4BSF 03-G10 | C C A C G G C A T G C T C G G C T T C G | | | 220 |

| | 220 | | 230 | |
|---|---|---|---|---|
| Wildtyp | A C A A C A T C C T C G G G G T C G A C | | | 239 |
| P1B 01-H1 | A C A A C A T C C T C G G G G T C G A C | | | 240 |
| P1B 01-E4 | A C A A C A T C C T C G G G G T C G A C | | | 240 |
| P2B 08-H3 | A C A A C A T C C T T G G G G T C G A C | | | 240 |
| P3B 13-D10 | A C A A C A T C C T T G G G G T C G A C | | | 240 |
| P4B 04-H3 | A C A A C A T C C T T G G G G T C G A C | | | 240 |
| P5B 14-C11 | A C A A C A T C C T T G G G G T C G A C | | | 240 |
| P4BSF 03-G10 | A C A A C A T C C T T G G G G T C G A C | | | 240 |

| | 240 | | | | | | | | | | 250 | | | | | | | | | | |
|---|---|---|---|---|---|---|---|---|---|---|---|---|---|---|---|---|---|---|---|---|---|
| Wildtyp | T | A | C | T | G | G | T | T | C | G | G | C | A | T | T | C | C | C | A | G | 259 |
| P1B 01-H1 | T | A | C | T | G | G | T | T | C | G | G | C | A | T | T | C | C | C | A | G | 260 |
| P1B 01-E4 | T | A | C | T | G | G | T | T | C | G | G | C | A | T | T | C | C | C | A | G | 260 |
| P2B 08-H3 | T | A | C | T | G | G | T | T | C | G | G | C | A | T | T | C | C | C | A | G | 260 |
| P3B 13-D10 | T | A | C | T | G | G | T | T | C | G | G | C | A | T | T | C | C | C | A | G | 260 |
| P4B 04-H3 | T | A | C | T | G | G | T | T | C | G | G | C | A | T | T | C | C | C | A | A | 260 |
| P5B 14-C11 | T | A | C | T | G | G | T | T | C | G | G | C | A | T | T | C | C | C | A | G | 260 |
| P4BSF 03-G10 | T | A | C | T | G | G | T | T | C | G | G | C | A | T | T | C | C | C | A | G | 260 |

| | 260 | | | | | | | | | | 270 | | | | | | | | | | |
|---|---|---|---|---|---|---|---|---|---|---|---|---|---|---|---|---|---|---|---|---|---|
| Wildtyp | C | G | C | C | T | T | G | C | G | C | C | G | T | G | A | C | G | G | T | G | 279 |
| P1B 01-H1 | C | G | C | C | T | T | G | C | G | C | C | G | T | G | A | C | G | G | T | G | 280 |
| P1B 01-E4 | C | G | C | C | T | T | G | C | G | C | C | G | T | G | A | C | G | G | T | G | 280 |
| P2B 08-H3 | C | G | C | C | T | T | G | C | G | C | C | G | T | G | A | C | G | G | T | G | 280 |
| P3B 13-D10 | C | G | C | C | T | T | G | C | G | C | C | G | T | G | A | C | G | G | T | G | 280 |
| P4B 04-H3 | C | G | C | C | T | T | G | C | G | C | C | G | T | G | A | C | G | G | T | G | 280 |
| P5B 14-C11 | C | G | C | C | T | T | G | C | G | C | C | G | T | G | A | C | G | G | T | G | 280 |
| P4BSF 03-G10 | C | G | C | C | T | T | G | C | G | C | C | G | T | G | A | C | G | G | T | G | 280 |

| | 280 | | | | | | | | | | 290 | | | | | | | | | | |
|---|---|---|---|---|---|---|---|---|---|---|---|---|---|---|---|---|---|---|---|---|---|
| Wildtyp | C | C | C | A | G | G | T | C | T | A | C | G | T | C | A | C | C | G | A | A | 299 |
| P1B 01-H1 | C | C | C | A | G | G | T | C | T | A | C | G | T | C | A | C | C | G | A | A | 300 |
| P1B 01-E4 | C | C | C | A | G | G | T | C | T | A | C | G | T | C | A | C | C | G | A | A | 300 |
| P2B 08-H3 | C | C | C | A | G | G | T | C | T | A | C | G | T | C | A | C | C | G | A | A | 300 |
| P3B 13-D10 | C | C | C | A | G | G | T | C | T | A | C | G | T | C | A | C | C | G | A | A | 300 |
| P4B 04-H3 | C | C | C | A | G | G | T | C | T | A | C | G | T | C | A | C | C | G | A | A | 300 |
| P5B 14-C11 | C | C | C | A | G | G | T | C | T | A | C | G | T | C | A | C | C | G | A | A | 300 |
| P4BSF 03-G10 | C | C | C | A | G | G | T | C | T | A | C | G | T | C | A | C | C | G | A | A | 300 |

| | 300 | | | | | | | | | | 310 | | | | | | | | | | |
|---|---|---|---|---|---|---|---|---|---|---|---|---|---|---|---|---|---|---|---|---|---|
| Wildtyp | G | T | C | A | G | C | C | A | G | T | T | G | G | A | C | A | C | C | T | C | 319 |
| P1B 01-H1 | G | T | C | A | G | C | C | A | G | T | T | G | G | A | C | A | C | C | T | C | 320 |
| P1B 01-E4 | G | T | C | A | G | C | C | A | G | T | T | G | G | A | C | A | C | C | T | C | 320 |
| P2B 08-H3 | G | T | C | A | G | C | C | A | G | T | T | G | G | A | C | A | C | C | T | C | 320 |
| P3B 13-D10 | G | G | C | A | G | C | C | A | G | T | T | G | G | A | C | A | C | C | T | C | 320 |
| P4B 04-H3 | G | G | C | A | G | C | C | A | G | T | T | G | G | A | C | A | C | C | T | C | 320 |
| P5B 14-C11 | G | G | C | A | G | C | C | A | G | T | T | G | G | A | C | A | C | C | T | C | 320 |
| P4BSF 03-G10 | G | G | C | A | G | C | C | A | G | T | T | G | G | A | C | A | C | C | T | C | 320 |

| | 320 | | | | | | | | | | 330 | | | | | | | | | | |
|---|---|---|---|---|---|---|---|---|---|---|---|---|---|---|---|---|---|---|---|---|---|
| Wildtyp | G | G | A | A | G | T | C | C | G | C | G | G | C | G | A | G | C | A | G | T | 339 |
| P1B 01-H1 | G | G | A | A | G | T | C | C | G | C | G | G | C | G | A | G | C | A | G | T | 340 |
| P1B 01-E4 | G | G | A | A | G | T | C | C | G | C | G | G | C | G | A | G | C | A | G | T | 340 |
| P2B 08-H3 | G | G | A | A | G | T | C | C | G | C | G | G | C | G | A | G | C | A | G | T | 340 |
| P3B 13-D10 | G | G | A | A | G | T | C | C | G | C | G | G | C | G | A | G | C | A | G | T | 340 |
| P4B 04-H3 | G | G | A | A | G | T | C | C | G | C | G | G | C | G | A | G | C | A | G | T | 340 |
| P5B 14-C11 | G | G | A | A | G | T | C | C | G | C | G | G | C | G | A | G | C | A | G | T | 340 |
| P4BSF 03-G10 | G | G | A | A | G | T | C | C | G | C | G | G | C | G | A | G | C | A | G | T | 340 |

| | 340 | | | | | | | | | | 350 | | | | | | | | | | |
|---|---|---|---|---|---|---|---|---|---|---|---|---|---|---|---|---|---|---|---|---|---|
| Wildtyp | T | G | C | T | G | C | A | A | C | A | G | G | T | G | G | A | G | G | A | A | 359 |
| P1B 01-H1 | T | G | C | T | G | C | A | A | C | A | G | G | T | G | G | A | G | G | A | A | 360 |
| P1B 01-E4 | T | G | C | T | G | C | A | A | C | A | G | G | T | G | G | A | G | G | A | A | 360 |
| P2B 08-H3 | T | G | C | T | G | C | A | A | C | A | G | G | T | G | G | A | G | G | A | A | 360 |
| P3B 13-D10 | T | G | C | T | G | C | A | A | C | A | G | G | T | G | G | A | G | G | A | A | 360 |
| P4B 04-H3 | T | G | C | T | G | C | A | A | C | A | G | G | T | G | G | A | G | G | A | A | 360 |
| P5B 14-C11 | T | G | C | T | G | C | A | A | C | A | G | G | T | G | G | A | G | G | A | A | 360 |
| P4BSF 03-G10 | T | G | C | T | G | C | A | A | C | A | G | G | T | G | G | A | G | G | A | A | 360 |

| | 360 | | | | | | | | | | 370 | | | | | | | | | | |
|---|---|---|---|---|---|---|---|---|---|---|---|---|---|---|---|---|---|---|---|---|---|
| Wildtyp | A | T | C | G | T | C | G | C | C | C | T | C | A | G | C | G | G | C | C | A | 379 |
| P1B 01-H1 | A | T | C | G | T | C | G | C | C | C | T | C | A | G | C | G | G | C | C | A | 380 |
| P1B 01-E4 | A | T | C | G | T | C | G | C | C | C | T | C | A | G | C | G | G | C | C | A | 380 |
| P2B 08-H3 | A | T | C | G | T | C | G | C | C | C | T | C | A | G | C | G | G | C | C | A | 380 |
| P3B 13-D10 | A | T | C | G | T | C | G | C | C | C | T | C | A | G | C | G | G | C | C | A | 380 |
| P4B 04-H3 | A | T | C | G | T | C | G | C | C | C | T | C | A | G | C | G | G | C | C | A | 380 |
| P5B 14-C11 | A | T | C | G | T | C | G | C | C | C | T | C | A | G | C | G | G | C | C | A | 380 |
| P4BSF 03-G10 | A | T | C | G | T | C | G | C | C | C | T | C | A | G | C | G | G | C | C | A | 380 |

| | 380 | | | | | | | | | | 390 | | | | | | | | | | |
|---|---|---|---|---|---|---|---|---|---|---|---|---|---|---|---|---|---|---|---|---|---|
| Wildtyp | G | C | C | C | A | A | G | G | T | C | A | A | C | C | T | G | A | T | C | G | 399 |
| P1B 01-H1 | G | C | C | C | A | A | G | G | T | C | A | A | C | C | T | G | A | T | C | G | 400 |
| P1B 01-E4 | G | C | C | C | A | A | G | G | T | C | A | A | C | C | T | G | A | T | C | G | 400 |
| P2B 08-H3 | G | C | C | C | A | A | G | G | T | C | A | A | C | C | T | G | A | T | C | G | 400 |
| P3B 13-D10 | G | C | C | C | A | A | G | G | T | C | A | A | C | C | T | G | A | T | C | G | 400 |
| P4B 04-H3 | G | C | C | C | A | A | G | G | T | C | A | A | C | C | T | G | A | T | C | G | 400 |
| P5B 14-C11 | G | C | C | C | A | A | G | G | T | C | A | A | C | C | T | G | A | T | C | G | 400 |
| P4BSF 03-G10 | G | C | C | C | A | A | G | G | T | C | A | A | C | C | T | G | A | T | C | G | 400 |

| | 400 | | | | | | | | | | 410 | | | | | | | | | | |
|---|---|---|---|---|---|---|---|---|---|---|---|---|---|---|---|---|---|---|---|---|---|
| Wildtyp | G | C | C | A | C | A | G | C | C | A | C | G | G | C | G | G | G | C | C | G | 419 |
| P1B 01-H1 | G | C | C | A | C | A | G | C | C | A | C | G | G | C | G | G | G | C | C | G | 420 |
| P1B 01-E4 | G | C | C | A | C | A | G | C | C | A | C | G | G | C | G | G | G | C | C | G | 420 |
| P2B 08-H3 | G | C | C | A | C | A | G | C | C | A | C | G | G | C | G | G | G | C | C | G | 420 |
| P3B 13-D10 | G | C | C | A | C | A | G | C | C | A | C | G | G | C | G | G | G | C | C | G | 420 |
| P4B 04-H3 | G | C | C | A | C | A | G | C | C | A | C | G | G | C | G | G | G | C | C | G | 420 |
| P5B 14-C11 | G | C | C | A | C | A | G | C | C | A | C | G | G | C | G | G | G | C | C | G | 420 |
| P4BSF 03-G10 | G | C | C | A | C | A | G | C | C | A | C | G | G | C | G | G | G | C | C | G | 420 |

| | 420 | | | | | | | | | | 430 | | | | | | | | | | |
|---|---|---|---|---|---|---|---|---|---|---|---|---|---|---|---|---|---|---|---|---|---|
| Wildtyp | A | C | C | A | T | C | C | G | C | T | A | C | G | T | C | G | C | C | G | C | 439 |
| P1B 01-H1 | A | C | C | A | T | C | C | G | C | T | A | C | G | T | C | G | C | C | G | C | 440 |
| P1B 01-E4 | A | C | C | A | T | C | C | G | C | T | A | C | G | T | C | G | C | C | G | C | 440 |
| P2B 08-H3 | A | C | C | A | T | C | C | G | C | T | A | C | G | T | C | G | C | C | G | C | 440 |
| P3B 13-D10 | A | C | C | A | T | C | C | G | C | T | A | C | G | T | C | G | C | C | G | C | 440 |
| P4B 04-H3 | A | C | C | A | T | C | C | G | C | T | A | C | G | T | C | G | C | C | G | C | 440 |
| P5B 14-C11 | A | C | C | A | T | C | C | G | C | T | A | C | G | T | C | G | C | C | G | C | 440 |
| P4BSF 03-G10 | A | C | C | A | T | C | C | G | C | T | A | C | G | T | C | G | C | C | G | C | 440 |

| | 440 | | | | | | | | | | 450 | | | | | | | | | | |
|---|---|---|---|---|---|---|---|---|---|---|---|---|---|---|---|---|---|---|---|---|---|
| Wildtyp | C | G | T | A | C | G | T | C | C | C | G | A | C | C | T | G | A | T | C | G | 459 |
| P1B 01-H1 | C | G | T | A | C | G | T | C | C | C | G | A | C | C | T | G | A | T | C | G | 460 |
| P1B 01-E4 | C | G | T | A | C | G | T | C | C | C | G | A | C | C | T | G | A | T | C | G | 460 |
| P2B 08-H3 | C | G | T | A | C | G | T | C | C | C | G | A | C | C | T | G | A | T | C | G | 460 |
| P3B 13-D10 | C | G | T | A | C | G | T | C | C | C | G | A | C | C | T | G | A | T | C | G | 460 |
| P4B 04-H3 | C | G | T | A | C | G | T | C | C | C | G | A | C | C | T | G | A | T | C | G | 460 |
| P5B 14-C11 | C | G | T | A | C | G | T | C | C | C | G | A | C | C | T | G | A | T | C | G | 460 |
| P4BSF 03-G10 | C | G | T | A | C | G | T | C | C | C | G | A | C | C | T | G | A | T | C | G | 460 |

| | 460 | | | | | | | | | | 470 | | | | | | | | | | |
|---|---|---|---|---|---|---|---|---|---|---|---|---|---|---|---|---|---|---|---|---|---|
| Wildtyp | C | T | T | C | C | G | C | C | A | C | C | A | G | C | G | T | C | G | G | C | 479 |
| P1B 01-H1 | C | T | T | C | C | G | C | C | A | T | C | A | G | C | G | T | C | G | G | C | 480 |
| P1B 01-E4 | C | T | T | C | C | G | C | C | A | C | C | A | G | C | G | T | C | G | G | C | 480 |
| P2B 08-H3 | C | T | T | C | C | G | C | C | A | C | C | A | G | C | G | T | C | G | G | C | 480 |
| P3B 13-D10 | C | T | T | C | C | G | C | C | A | C | C | A | G | C | G | T | C | G | G | C | 480 |
| P4B 04-H3 | C | T | T | C | C | G | C | C | A | C | C | A | G | C | G | T | C | G | G | C | 480 |
| P5B 14-C11 | C | T | T | C | C | G | C | C | A | C | C | A | G | C | G | T | C | G | G | C | 480 |
| P4BSF 03-G10 | C | T | T | C | C | G | C | C | A | C | C | A | G | C | G | T | C | G | G | C | 480 |

| | 480 490 | |
|---|---|---|
| Wildtyp | G C C C C G C A C A A G G G T T C G G A | 499 |
| P1B 01-H1 | G C C C C G C A C A A G G G T T C G G A | 500 |
| P1B 01-E4 | G C C C C G C A C A A G G G T T C G G A | 500 |
| P2B 08-H3 | G C C C C G C A C A A G G G T T C G G A | 500 |
| P3B 13-D10 | G C C C C G C A C A A G G G T T C G G A | 500 |
| P4B 04-H3 | G C C C C G C A C A A G G G T T C G G A | 500 |
| P5B 14-C11 | G C C C C G C A C A G G G G T T C G G A | 500 |
| P4BSF 03-G10 | G C C C C G C A C A A G G G T T C G G A | 500 |

| | 500 510 | |
|---|---|---|
| Wildtyp | C A C C G C C G A C T T C C T G C G C C | 519 |
| P1B 01-H1 | C A C C G C C G A C T T C C T G C G C C | 520 |
| P1B 01-E4 | C A C C G C C G A C T T C C T G C G C C | 520 |
| P2B 08-H3 | C A C C G C C G A C T T C C T G C G C C | 520 |
| P3B 13-D10 | C A C C G C C G A C T T C C T G C G C C | 520 |
| P4B 04-H3 | C A C C G C C G A C T T C C T G C G C C | 520 |
| P5B 14-C11 | C A C C G C C G A C T T C C T G C G C C | 520 |
| P4BSF 03-G10 | C A C C G C C G A C T T C C T G C G C C | 520 |

| | 520 530 | |
|---|---|---|
| Wildtyp | A G A T C C C A C C G G G T T C G G C C | 539 |
| P1B 01-H1 | A G A T C C C A C C G G G T T C G G C C | 540 |
| P1B 01-E4 | A G A T C C C A C C G G G T T C G G C C | 540 |
| P2B 08-H3 | A G A T C C C A C C G G G T T C G G C C | 540 |
| P3B 13-D10 | A G A T C C C A C C G G G T T C G G C C | 540 |
| P4B 04-H3 | A G A T C C C A C C G G G T T C G G C C | 540 |
| P5B 14-C11 | A G A T C C C A C C G G G T T C G G C C | 540 |
| P4BSF 03-G10 | A G A T C C C A C C G G G T T C G G C C | 540 |

| | 540 550 | |
|---|---|---|
| Wildtyp | G G C G A G G C A G T C C T C T C C G G | 559 |
| P1B 01-H1 | G G C G A G G C A G T C C T C T C C G G | 560 |
| P1B 01-E4 | G G C G A G G C A G T C C T C T C C G G | 560 |
| P2B 08-H3 | G G C G A G G C A G T C C T C T C C G G | 560 |
| P3B 13-D10 | G G C G A G G C A G T C C T C T C C G G | 560 |
| P4B 04-H3 | G G C G A G G C A G T C C T C T C C G G | 560 |
| P5B 14-C11 | G G C G A G G C A G T C C T C T C C G G | 560 |
| P4BSF 03-G10 | G G C G A G G C A G T C C T C T C C G G | 560 |

| | 560 570 | |
|---|---|---|
| Wildtyp | G C T G G T C A A C A G C C T C G G C G | 579 |
| P1B 01-H1 | G C T G G T C A A C A G C C T C G G C G | 580 |
| P1B 01-E4 | G C T G G T C A A C A G C C T C G G C G | 580 |
| P2B 08-H3 | G C T G G T C A A C A G C C T C G G C G | 580 |
| P3B 13-D10 | G C T G G T C A A C A G C C T C G G C G | 580 |
| P4B 04-H3 | G C T G G T C A A C A G C C T C G G C G | 580 |
| P5B 14-C11 | G C T G G T C A A C A G C C T C G G C G | 580 |
| P4BSF 03-G10 | G C T G G T C A A C A G C C T C G G C G | 580 |

| | 580 590 | |
|---|---|---|
| Wildtyp | C G C T G A T C A G C T T C C T T T C C | 599 |
| P1B 01-H1 | C G C T G A T C A G C T T C C T T T C C | 600 |
| P1B 01-E4 | C G C T G A T C A G C T T C C T T T C C | 600 |
| P2B 08-H3 | C G C T G A T C A G C T T C C T T T C C | 600 |
| P3B 13-D10 | C G C T G A T C A G C T T C C T T T C C | 600 |
| P4B 04-H3 | C G C T G A T C A G C T T C C T T T C C | 600 |
| P5B 14-C11 | C G C T G A T C A G C T T C C T T T C C | 600 |
| P4BSF 03-G10 | C G C T G A T C A G C T T C C T T T C C | 600 |

| | 600 | | | | | | | | | | 610 | | | | | | | | | | |
|---|---|---|---|---|---|---|---|---|---|---|---|---|---|---|---|---|---|---|---|---|---|
| Wildtyp | A | G | C | G | G | C | A | G | C | A | C | C | G | G | T | A | C | G | C | A | 619 |
| P1B 01-H1 | A | G | C | G | G | C | G | G | C | A | C | C | G | G | T | A | C | G | C | A | 620 |
| P1B 01-E4 | A | G | C | G | G | C | G | G | C | A | C | C | G | G | T | A | C | G | C | A | 620 |
| P2B 08-H3 | A | G | C | G | G | C | G | G | C | A | C | C | G | G | T | A | C | G | C | A | 620 |
| P3B 13-D10 | A | G | C | G | G | C | G | G | C | A | C | C | G | G | T | A | C | G | C | A | 620 |
| P4B 04-H3 | A | G | C | G | G | C | G | G | C | A | C | C | G | G | T | A | C | G | C | A | 620 |
| P5B 14-C11 | A | G | C | G | G | C | G | G | C | A | C | C | G | G | T | A | C | G | C | A | 620 |
| P4BSF 03-G10 | A | G | C | G | G | C | G | G | C | A | T | C | G | G | T | A | C | G | C | A | 620 |

| | 620 | | | | | | | | | | 630 | | | | | | | | | | |
|---|---|---|---|---|---|---|---|---|---|---|---|---|---|---|---|---|---|---|---|---|---|
| Wildtyp | G | A | A | T | T | C | A | C | T | G | G | G | C | T | C | G | C | T | G | G | 639 |
| P1B 01-H1 | G | A | A | T | T | C | A | C | T | G | G | G | C | T | C | G | C | T | G | G | 640 |
| P1B 01-E4 | G | A | A | T | T | C | A | C | T | G | G | G | C | T | C | G | C | T | G | G | 640 |
| P2B 08-H3 | G | A | A | T | T | T | A | C | T | G | G | G | C | T | C | G | C | T | G | G | 640 |
| P3B 13-D10 | G | A | A | T | T | T | A | C | T | G | G | G | C | T | C | G | C | T | G | G | 640 |
| P4B 04-H3 | G | A | A | T | T | T | A | C | T | G | G | G | C | T | C | G | C | T | G | G | 640 |
| P5B 14-C11 | G | A | A | T | T | T | A | C | T | G | G | G | C | T | C | G | C | T | G | G | 640 |
| P4BSF 03-G10 | G | A | A | T | T | T | T | C | T | G | G | G | C | T | C | G | C | T | G | G | 640 |

| | 640 | | | | | | | | | | 650 | | | | | | | | | | |
|---|---|---|---|---|---|---|---|---|---|---|---|---|---|---|---|---|---|---|---|---|---|
| Wildtyp | A | G | T | C | G | C | T | G | A | A | C | A | G | C | G | A | G | G | G | T | 659 |
| P1B 01-H1 | A | G | T | C | G | C | T | G | A | A | C | A | G | C | G | A | G | G | G | T | 660 |
| P1B 01-E4 | A | G | T | C | G | C | T | G | A | A | C | A | G | C | G | A | G | G | G | T | 660 |
| P2B 08-H3 | A | G | T | C | G | C | T | G | A | A | C | A | G | C | G | A | G | G | G | T | 660 |
| P3B 13-D10 | A | G | T | C | G | C | T | G | A | A | C | A | G | C | G | A | G | G | G | T | 660 |
| P4B 04-H3 | A | G | T | C | G | C | T | G | A | A | C | A | G | C | G | A | G | G | G | T | 660 |
| P5B 14-C11 | A | G | T | C | G | C | T | G | A | A | C | A | G | T | G | A | G | G | G | T | 660 |
| P4BSF 03-G10 | A | G | T | C | G | C | T | G | A | A | C | A | G | C | G | A | G | G | G | T | 660 |

| | 660 | | | | | | | | | | 670 | | | | | | | | | | |
|---|---|---|---|---|---|---|---|---|---|---|---|---|---|---|---|---|---|---|---|---|---|
| Wildtyp | G | C | C | G | C | G | C | G | C | T | T | C | A | A | C | G | C | C | A | A | 679 |
| P1B 01-H1 | G | C | C | G | C | G | C | G | C | T | T | C | A | A | C | G | C | C | A | A | 680 |
| P1B 01-E4 | G | C | C | G | C | G | C | G | C | T | T | C | A | A | C | G | C | C | A | A | 680 |
| P2B 08-H3 | G | C | C | G | C | G | C | G | C | T | T | C | A | A | C | G | C | C | A | A | 680 |
| P3B 13-D10 | G | C | C | G | C | G | C | G | C | T | T | C | A | A | C | G | C | C | A | A | 680 |
| P4B 04-H3 | G | C | C | G | C | G | C | G | C | T | T | C | A | A | C | G | C | C | A | A | 680 |
| P5B 14-C11 | G | C | C | G | C | G | C | G | C | T | T | C | A | A | C | G | C | C | A | A | 680 |
| P4BSF 03-G10 | G | C | C | G | C | G | C | G | C | T | T | C | A | A | C | G | C | C | A | A | 680 |

| | 680 | | | | | | | | | | 690 | | | | | | | | | | |
|---|---|---|---|---|---|---|---|---|---|---|---|---|---|---|---|---|---|---|---|---|---|
| Wildtyp | G | T | A | C | C | C | G | C | A | G | G | G | C | A | T | C | C | C | C | A | 699 |
| P1B 01-H1 | G | T | A | C | C | C | G | C | A | G | G | G | C | A | T | C | C | C | C | A | 700 |
| P1B 01-E4 | G | T | A | C | C | C | G | C | A | G | G | G | C | A | T | C | C | C | C | A | 700 |
| P2B 08-H3 | G | T | A | C | C | C | G | C | A | G | G | G | C | A | T | C | C | C | C | A | 700 |
| P3B 13-D10 | G | T | A | C | C | C | G | C | A | G | G | G | C | A | T | C | C | C | C | A | 700 |
| P4B 04-H3 | G | T | A | C | C | C | G | C | A | G | G | G | C | A | T | C | C | C | C | A | 700 |
| P5B 14-C11 | G | T | A | C | C | C | G | C | A | G | G | G | C | A | T | C | C | C | C | A | 700 |
| P4BSF 03-G10 | G | T | A | C | C | C | G | C | A | G | G | G | C | A | T | C | C | C | C | A | 700 |

| | 700 | | | | | | | | | | 710 | | | | | | | | | | |
|---|---|---|---|---|---|---|---|---|---|---|---|---|---|---|---|---|---|---|---|---|---|
| Wildtyp | C | C | T | C | G | G | C | C | T | G | C | G | G | C | G | A | A | G | G | C | 719 |
| P1B 01-H1 | C | C | T | C | G | G | C | C | T | G | C | G | G | C | G | A | A | G | G | C | 720 |
| P1B 01-E4 | C | C | T | C | G | G | C | C | T | G | C | G | G | C | G | A | A | G | G | C | 720 |
| P2B 08-H3 | C | C | T | C | G | G | C | C | T | G | C | G | G | C | G | A | A | G | G | C | 720 |
| P3B 13-D10 | C | C | T | C | G | G | C | C | T | G | C | G | G | C | G | A | A | G | G | C | 720 |
| P4B 04-H3 | C | C | T | C | G | G | C | C | T | G | C | G | G | C | G | A | A | G | G | C | 720 |
| P5B 14-C11 | C | C | T | C | G | G | C | C | T | G | C | G | G | C | G | A | A | G | G | C | 720 |
| P4BSF 03-G10 | C | C | T | C | G | G | C | C | T | G | C | G | G | C | G | A | A | G | G | C | 720 |

| | 720 ... 730 | |
|---|---|---|
| Wildtyp | G C C T A C A A G G T C A A C G G C G T | 739 |
| P1B 01-H1 | G C C T A C A A G G T C A A C G G C G T | 740 |
| P1B 01-E4 | G C C T A C A A G G T C A A C G G C G T | 740 |
| P2B 08-H3 | G C C T A C A A G G T C A A C G G C G T | 740 |
| P3B 13-D10 | G C C T A C A A G G T C A A C G G C G T | 740 |
| P4B 04-H3 | G C T T A C A A G G T C A A C G G C G T | 740 |
| P5B 14-C11 | G C T T A C A A G G T C A A C G G C G T | 740 |
| P4BSF 03-G10 | G C C T A C A A G G T C A A C G G C G T | 740 |

| | 740 ... 750 | |
|---|---|---|
| Wildtyp | G A G C T A T T A C T C C T G G A G C G | 759 |
| P1B 01-H1 | G A G C T A T T A C T C C T G G A G C G | 760 |
| P1B 01-E4 | G A G C T A T T A C T C C T G G A G C G | 760 |
| P2B 08-H3 | G A G C T A T T A C T C C T G G A G C G | 760 |
| P3B 13-D10 | G A G C T A T T A C T C C T G G A G C G | 760 |
| P4B 04-H3 | G A G C T A T T A C T C C T G G A G C G | 760 |
| P5B 14-C11 | G A G C T A T T A C T C C T G G A G C G | 760 |
| P4BSF 03-G10 | G A G C T A T T A C T C C T G G A G C G | 760 |

| | 760 ... 770 | |
|---|---|---|
| Wildtyp | G T T C C T C G C C G C T G A C C A A C | 779 |
| P1B 01-H1 | G T T C C T C G C C G C T G A C C A A C | 780 |
| P1B 01-E4 | G T T C C T C G C C G C T G A C C A A C | 780 |
| P2B 08-H3 | G T T C C T C G C C G C T G A C C A A C | 780 |
| P3B 13-D10 | G T T C C T C G C C G C T G A C C A A C | 780 |
| P4B 04-H3 | G T T C C T C G C C G C T G A C C A A C | 780 |
| P5B 14-C11 | G T T C C T C G C C G C T G A C C A A C | 780 |
| P4BSF 03-G10 | G T T C C T C G C C G C T G A C C A A C | 780 |

| | 780 ... 790 | |
|---|---|---|
| Wildtyp | T T C C T C G A T C C G A G C G A C G C | 799 |
| P1B 01-H1 | T T C C T C G A T C C G A G C G A C G C | 800 |
| P1B 01-E4 | T T C C T C G A T C C G A G C G A C G C | 800 |
| P2B 08-H3 | T T C C T C G A T C C G A G C G A C G C | 800 |
| P3B 13-D10 | T T C C T C G A T C C G A G C G A C G C | 800 |
| P4B 04-H3 | T T C C T C G A T C C G A G C G A C G C | 800 |
| P5B 14-C11 | T T C C T C G A T C C G A G C G A C G C | 800 |
| P4BSF 03-G10 | T T C C T C G A T C C G A G C G A C G C | 800 |

| | 800 ... 810 | |
|---|---|---|
| Wildtyp | C T T C C T C G G C G C C T C G T C G C | 819 |
| P1B 01-H1 | C T T C C T C G G C G C C T C G T C G C | 820 |
| P1B 01-E4 | C T T C C T C G G C G C C T C G T C G C | 820 |
| P2B 08-H3 | C T T C C T C G G C G C C T C G T C G C | 820 |
| P3B 13-D10 | C T T C C T C G G C G C C T C G T C G C | 820 |
| P4B 04-H3 | C T T C C T C G G C G C C T C G T C G C | 820 |
| P5B 14-C11 | C T T C C T C G G C G C C T C G T C G C | 820 |
| P4BSF 03-G10 | C T T C C T C G G C G C C T C G T C G C | 820 |

| | 820 ... 830 | |
|---|---|---|
| Wildtyp | T G A C C T T C A A G A A C G G C A C C | 839 |
| P1B 01-H1 | T G A C C T T C A A G A A C G G C A C C | 840 |
| P1B 01-E4 | T G A C C T T C A A G A A C G G C A C C | 840 |
| P2B 08-H3 | T G A C C T T C A A G A A C G G C A C C | 840 |
| P3B 13-D10 | T G A C C T T C A A G A A C G G C A C C | 840 |
| P4B 04-H3 | T G A C C T T C A A G A A C G G C A C C | 840 |
| P5B 14-C11 | T G A C C T T C A A G A A C G G C A C C | 840 |
| P4BSF 03-G10 | T G A C C T T C A A G A A C G G C A C C | 840 |

```
              840                 850
Wildtyp       G C C A A C G A C G G C C T G G T C G G  859
P1B 01-H1     G C C A A C G A C G G C C T G G T C G G  860
P1B 01-E4     G C C A A C G A C G G C C T G G T C G G  860
P2B 08-H3     G C C A A C G A C G G C C T G G T C G G  860
P3B 13-D10    G C C A A C G A C G G C C T G G T C G G  860
P4B 04-H3     G C C A A C G A C G G C C T G G T C G G  860
P5B 14-C11    G C C A A C G A C G G C C T G G T C G G  860
P4BSF 03-G10  G C C A A C G A C G G C C T G G T C G G  860

              860                 870
Wildtyp       C A C C T G C A G T T C G C A C C T G G  879
P1B 01-H1     C A C C T G C A G T T C G C A C C T G G  880
P1B 01-E4     C A C C T G C A G T T C G C A C C T G G  880
P2B 08-H3     C A C C T G C A G T T C G C A C C T G G  880
P3B 13-D10    C A C C T G C A G T T C G C A C C T G G  880
P4B 04-H3     C A C C T G C A G T T C G C A C C T G G  880
P5B 14-C11    C A C C T G C A G T T C G C A C C T G G  880
P4BSF 03-G10  C A C C T G C A G T T C G C A C C T G G  880

              880                 890
Wildtyp       G C A T G G T G A T C C G C G A C A A C  899
P1B 01-H1     G C A T G G T G A T C C G C G A C A A C  900
P1B 01-E4     G C A T G G T G A T C C G C G A C A A C  900
P2B 08-H3     G C A T G G T G A T C C G C G A C A A C  900
P3B 13-D10    G C A T G G T G A T C C G C G A C A A C  900
P4B 04-H3     G C A T G G T G A T C C G C G A C A A C  900
P5B 14-C11    G C A T G G T G A T C C G C G A C A A C  900
P4BSF 03-G10  G C A T G G T G A T C C G C G A C A A C  900

              900                 910
Wildtyp       T A C C G G A T G A A C C A C C T G G A  919
P1B 01-H1     T A C C G G A T G A A C C A C C T G G A  920
P1B 01-E4     T A C C G G A T G A A C C A C C T G G A  920
P2B 08-H3     T A C C G G A T G A A C C A C C T G G A  920
P3B 13-D10    T A C C G G A T G A A C C A C C T G G A  920
P4B 04-H3     T A C C G G A T G A A C C A C C T G G A  920
P5B 14-C11    T A C C G G A T G A A C C A C C T G G A  920
P4BSF 03-G10  T A C C G G A T G A A C C A C C T G G A  920

              920                 930
Wildtyp       C G A G G T G A A C C A G G T C T T C G  939
P1B 01-H1     C G A G G T G A A C C A G G T C T T C G  940
P1B 01-E4     C G A G G T G A A C C A G G T C T T C G  940
P2B 08-H3     C G A G G T G A A C C A G G T C T T C G  940
P3B 13-D10    C G A G G T G A A C C A G G T C T T C G  940
P4B 04-H3     C G A G G T G A A C C A G G T C[C]T C G  940
P5B 14-C11    C G A G G T G A A C C A G G T C[C]T C G  940
P4BSF 03-G10  C G A G G T G A A C C A G G T C T T C G  940

              940                 950
Wildtyp       G C C T C A C C A G C C T G T T C G A G  959
P1B 01-H1     G C C T C A C C A G C C T G T T C G A G  960
P1B 01-E4     G C C T C A C C A G C C T G T T C G A G  960
P2B 08-H3     G C C T C A C C A G C C T G T T C G A G  960
P3B 13-D10    G C C T C A C C A G C C T G T T C G A G  960
P4B 04-H3     G C C T C A C C A G C C T G T T C G A G  960
P5B 14-C11    G C C T C A C C A G C C T G T T C G A G  960
P4BSF 03-G10  G C C T C A C C A G C C T G T T C G A G  960
```

| | 960 | | | | | | | | | | 970 | | | | | | | | | | |
|---|---|---|---|---|---|---|---|---|---|---|---|---|---|---|---|---|---|---|---|---|---|
| Wildtyp | A | C | C | A | G | C | C | C | G | G | T | C | A | G | C | G | T | C | T | A | 979 |
| P1B 01-H1 | A | C | C | A | G | C | C | C | G | G | T | C | A | G | C | G | T | C | T | A | 980 |
| P1B 01-E4 | A | C | C | A | G | C | C | C | G | G | T | C | A | G | C | G | T | C | T | A | 980 |
| P2B 08-H3 | A | C | C | A | G | C | C | C | G | G | T | C | A | G | C | G | T | C | T | A | 980 |
| P3B 13-D10 | A | C | C | A | G | C | C | C | G | G | T | C | A | G | C | G | T | C | T | A | 980 |
| P4B 04-H3 | A | C | C | A | G | C | C | C | G | G | T | C | A | G | C | G | T | C | T | A | 980 |
| P5B 14-C11 | A | C | C | A | G | C | C | C | G | G | T | C | A | G | C | G | T | C | T | A | 980 |
| P4BSF 03-G10 | A | C | C | A | G | C | C | C | G | G | T | C | A | G | C | G | T | C | T | A | 980 |

| | 980 | | | | | | | | | | 990 | | | | | | | | | | |
|---|---|---|---|---|---|---|---|---|---|---|---|---|---|---|---|---|---|---|---|---|---|
| Wildtyp | C | C | G | C | C | A | G | C | A | C | G | C | C | A | A | C | C | G | C | C | 999 |
| P1B 01-H1 | C | C | G | C | C | A | G | C | A | C | G | C | C | A | A | C | C | G | C | C | 1000 |
| P1B 01-E4 | C | C | G | C | C | A | G | C | A | C | G | C | C | A | A | C | C | G | C | C | 1000 |
| P2B 08-H3 | C | C | G | C | C | A | G | C | A | C | G | C | C | A | A | C | C | G | C | C | 1000 |
| P3B 13-D10 | C | C | G | C | C | A | G | C | A | C | G | C | C | A | A | C | C | G | C | C | 1000 |
| P4B 04-H3 | C | C | G | C | C | A | G | C | A | C | G | C | C | A | A | C | C | G | C | C | 1000 |
| P5B 14-C11 | C | C | G | C | C | A | G | C | A | C | G | C | C | A | A | C | C | G | C | C | 1000 |
| P4BSF 03-G10 | C | C | G | C | C | A | G | C | A | C | G | C | C | A | A | C | C | G | C | C | 1000 |

| | 1000 | | | | | | | | | | 1010 | | | | | | | | | | |
|---|---|---|---|---|---|---|---|---|---|---|---|---|---|---|---|---|---|---|---|---|---|
| Wildtyp | T | G | A | A | G | A | A | C | G | C | C | A | G | C | C | T | G | T | A | G | 1019 |
| P1B 01-H1 | T | G | A | A | G | A | A | C | G | C | C | A | G | C | C | T | G | T | A | G | 1020 |
| P1B 01-E4 | T | G | A | A | G | A | A | C | G | C | C | A | G | C | C | T | G | T | A | G | 1020 |
| P2B 08-H3 | T | G | A | A | G | A | A | C | G | C | C | A | G | C | C | T | G | T | A | G | 1020 |
| P3B 13-D10 | T | G | A | A | G | A | A | C | G | C | C | A | G | C | C | T | G | T | A | G | 1020 |
| P4B 04-H3 | T | G | A | A | G | A | A | C | G | C | C | A | G | C | C | T | G | T | A | G | 1020 |
| P5B 14-C11 | T | G | A | A | G | A | A | C | G | C | C | A | G | C | C | T | G | T | A | G | 1020 |
| P4BSF 03-G10 | T | G | A | A | G | A | A | C | G | C | C | A | G | C | C | T | G | T | A | G | 1020 |

| | 1020 | | | | | | | | | | 1030 | | | | | | | | | | |
|---|---|---|---|---|---|---|---|---|---|---|---|---|---|---|---|---|---|---|---|---|---|
| Wildtyp | G | A | C | C | C | C | G | G | C | C | G | G | G | G | C | C | T | C | G | G | 1039 |
| P1B 01-H1 | G | A | C | C | C | C | G | G | C | C | G | G | G | G | C | C | T | C | G | G | 1040 |
| P1B 01-E4 | G | A | C | C | C | C | G | G | C | C | G | G | G | G | C | C | T | C | G | G | 1040 |
| P2B 08-H3 | G | A | C | C | C | C | G | G | C | C | G | G | G | G | C | C | T | C | G | G | 1040 |
| P3B 13-D10 | G | A | C | C | C | C | G | G | C | C | G | G | G | G | C | C | T | C | G | G | 1040 |
| P4B 04-H3 | G | A | C | C | C | C | G | G | C | C | G | G | G | G | C | C | T | C | G | G | 1040 |
| P5B 14-C11 | G | A | C | C | C | C | G | G | C | C | G | G | G | G | C | C | T | C | G | G | 1040 |
| P4BSF 03-G10 | G | A | C | C | C | C | G | G | C | C | G | G | G | G | C | C | T | C | G | G | 1040 |

| | 1040 | | | | | | | | | | |
|---|---|---|---|---|---|---|---|---|---|---|---|
| Wildtyp | C | C | C | G | G | G | C | C | | | 1047 |
| P1B 01-H1 | C | C | C | G | G | G | C | C | C | | 1049 |
| P1B 01-E4 | C | C | C | G | G | G | C | C | C | | 1049 |
| P2B 08-H3 | C | C | C | G | G | G | C | C | C | | 1049 |
| P3B 13-D10 | C | C | C | G | G | G | C | C | C | | 1049 |
| P4B 04-H3 | C | C | C | G | G | G | C | C | C | G | 1050 |
| P5B 14-C11 | C | C | C | G | G | G | C | C | C | | 1049 |
| P4BSF 03-G10 | C | C | C | G | G | G | C | C | C | | 1049 |

SEQUENZPROTOKOLL

(1) ALLGEMEINE ANGABEN:

(i) ANMELDER:

(A) NAME: Studiengesellschaft Kohle mbH
(B) STRASSE: Kaiser-Wilhelm-Platz 1
(C) ORT: Muelheim an der Ruhr
(E) LAND: Deutschland
(F) POSTLEITZAHL: 45470

(ii) BEZEICHNUNG DER ERFINDUNG: Verfahren zur Herstellung und Identifizierung neuer Hydrolasen mit verbesserten Eigenschaften

(iii) ANZAHL DER SEQUENZEN: 21

(iv) COMPUTER-LESBARE FASSUNG:

(A) DATENTRÄGER: Floppy disk
(B) COMPUTER: IBM PC compatible
(C) BETRIEBSSYSTEM: PC-DOS/MS-DOS
(D) SOFTWARE: PatentIn Release #1.0, Version #1.30 (EPA)

(2) ANGABEN ZU SEQ ID NO: 1:

(i) SEQUENZKENNZEICHEN:

(A) LÄNGE: 30 Basenpaare
(B) ART: Nucleotid
(C) STRANGFORM: nicht bekannt
(D) TOPOLOGIE: linear

(ii) ART DES MOLEKÜLS: Sonstige Nucleinsäure

(A) BESCHREIBUNG: /desc = "synthetische DNA"

(xi) SEQUENZBESCHREIBUNG: SEQ ID NO: 1:

```
GCGCAATTAA CCCTCACTAA AGGGAACAAA                                            30
```

(2) ANGABEN ZU SEQ ID NO: 2:

(i) SEQUENZKENNZEICHEN:

(A) LÄNGE: 27 Basenpaare
(B) ART: Nucleotid
(C) STRANGFORM: nicht bekannt
(D) TOPOLOGIE: linear
(ii) ART DES MOLEKÜLS: Sonstige Nucleinsäure

(A) BESCHREIBUNG: /desc = "synthetische DNA"

(xi) SEQUENZBESCHREIBUNG: SEQ ID NO: 2:

GCGTAATACG ACTCACTATA GGGCGAA 27

(2) ANGABEN ZU SEQ ID NO: 3:

(i) SEQUENZKENNZEICHEN:

(A) LÄNGE: 1049 Basenpaare
(B) ART: Nucleotid
(C) STRANGFORM: nicht bekannt
(D) TOPOLOGIE: nicht bekannt

(ii.) ART DES MOLEKÜLS: Genom-DNA

(ix) MERKMAL:

(A) NAME/SCHLÜSSEL: CDS
(B) LAGE:85..1017

(ix) MERKMAL:

(A) NAME/SCHLÜSSEL: mat_peptide
(B) LAGE:163..1017

(xi) SEQUENZBESCHREIBUNG: SEQ ID NO: 3:

```
GGATCCCCCG GTTCTCCCGG AAGGATTCGG GCGATGGCTG GCAGGACGCG CCCCTCGGCC    60

CCATCAACCT GAGATGAGAA CAAC ATG AAG AAG AAG TAT CTG CTC CCC CTC      111
                           Met Lys Lys Lys Tyr Leu Leu Pro Leu
                           -26 -25                 -20

GGC CTG GCC ATC GGT CTC GCC TCT CTC GCT GCC AGC CCT CTG ATC CAG     159
Gly Leu Ala Ile Gly Leu Ala Ser Leu Ala Ala Ser Pro Leu Ile Gln
        -15                 -10                 -5

GCC AGC ACC TAC ACC CAG ACC AAA TAC CCC ATC GTG CTG GCC CAC GGC     207
Ala Ser Thr Tyr Thr Gln Thr Lys Tyr Pro Ile Val Leu Ala His Gly
      1               5                  10                  15

ATG CTC GGC TTC GAC AAC ATC CTC GGG GTC GAC TAC TGG TTC GGC ATT     255
Met Leu Gly Phe Asp Asn Ile Leu Gly Val Asp Tyr Trp Phe Gly Ile
                 20                  25                  30

CCC AGC GCC TTG CGC CGT GAC GGT GCC CAG GTC TAC GTC ACC GAA GTC     303
Pro Ser Ala Leu Arg Arg Asp Gly Ala Gln Val Tyr Val Thr Glu Val
             35                  40                  45

AGC CAG TTG GAC ACC TCG GAA GTC CGC GGC GAG CAG TTG CTG CAA CAG     351
Ser Gln Leu Asp Thr Ser Glu Val Arg Gly Glu Gln Leu Leu Gln Gln
         50                  55                  60

GTG GAG GAA ATC GTC GCC CTC AGC GGC CAG CCC AAG GTC AAC CTG ATC     399
Val Glu Glu Ile Val Ala Leu Ser Gly Gln Pro Lys Val Asn Leu Ile
     65                  70                  75

GGC CAC AGC CAC GGC GGG CCG ACC ATC CGC TAC GTC GCC GCC GTA CGT     447
Gly His Ser His Gly Gly Pro Thr Ile Arg Tyr Val Ala Ala Val Arg
 80                  85                  90                  95

CCC GAC CTG ATC GCT TCC GCC ATC AGC GTC GGC GCC CCG CAC AAG GGT     495
Pro Asp Leu Ile Ala Ser Ala Ile Ser Val Gly Ala Pro His Lys Gly
                100                 105                 110

TCG GAC ACC GCC GAC TTC CTG CGC CAG ATC CCA CCG GGT TCG GCC GGC     543
Ser Asp Thr Ala Asp Phe Leu Arg Gln Ile Pro Pro Gly Ser Ala Gly
            115                 120                 125
```

```
GAG GCA GTC CTC TCC GGG CTG GTC AAC AGC CTC GGC GCG CTG ATC AGC      591
Glu Ala Val Leu Ser Gly Leu Val Asn Ser Leu Gly Ala Leu Ile Ser
        130                 135                 140

TTC CTT TCC AGC GGC GGC ACC GGT ACG CAG AAT TCA CTG GGC TCG CTG      639
Phe Leu Ser Ser Gly Gly Thr Gly Thr Gln Asn Ser Leu Gly Ser Leu
    145                 150                 155

GAG TCG CTG AAC AGC GAG GGT GCC GCG CGC TTC AAC GCC AAG TAC CCG      687
Glu Ser Leu Asn Ser Glu Gly Ala Ala Arg Phe Asn Ala Lys Tyr Pro
160                 165                 170                 175

CAG GGC ATC CCC ACC TCG GCC TGC GGC GAA GGC GCC TAC AAG GTC AAC      735
Gln Gly Ile Pro Thr Ser Ala Cys Gly Glu Gly Ala Tyr Lys Val Asn
                180                 185                 190

GGC GTG AGC TAT TAC TCC TGG AGC GGT TCC TCG CCG CTG ACC AAC TTC      783
Gly Val Ser Tyr Tyr Ser Trp Ser Gly Ser Ser Pro Leu Thr Asn Phe
            195                 200                 205

CTC GAT CCG AGC GAC GCC TTC CTC GGC GCC TCG TCG CTG ACC TTC AAG      831
Leu Asp Pro Ser Asp Ala Phe Leu Gly Ala Ser Ser Leu Thr Phe Lys
        210                 215                 220

AAC GGC ACC GCC AAC GAC GGC CTG GTC GGC ACC TGC AGT TCG CAC CTG      879
Asn Gly Thr Ala Asn Asp Gly Leu Val Gly Thr Cys Ser Ser His Leu
    225                 230                 235

GGC ATG GTG ATC CGC GAC AAC TAC CGG ATG AAC CAC CTG GAC GAG GTG      927
Gly Met Val Ile Arg Asp Asn Tyr Arg Met Asn His Leu Asp Glu Val
240                 245                 250                 255

AAC CAG GTC TTC GGC CTC ACC AGC CTG TTC GAG ACC AGC CCG GTC AGC      975
Asn Gln Val Phe Gly Leu Thr Ser Leu Phe Glu Thr Ser Pro Val Ser
                260                 265                 270

GTC TAC CGC CAG CAC GCC AAC CGC CTG AAG AAC GCC AGC CTG             1017
Val Tyr Arg Gln His Ala Asn Arg Leu Lys Asn Ala Ser Leu
            275                 280                 285

TAGGACCCCG GCCGGGGCCT CGGCCCGGGC CC                                 1049
```

(2) ANGABEN ZU SEQ ID NO: 4:

(i) SEQUENZKENNZEICHEN:

(A) LÄNGE: 311 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 4:

```
Met Lys Lys Lys Tyr Leu Leu Pro Leu Gly Leu Ala Ile Gly Leu Ala
-26 -25                 -20                 -15

Ser Leu Ala Ala Ser Pro Leu Ile Gln Ala Ser Thr Tyr Thr Gln Thr
-10                 -5                  1               5
```

```
Lys Tyr Pro Ile Val Leu Ala His Gly Met Leu Gly Phe Asp Asn Ile
            10                  15                  20

Leu Gly Val Asp Tyr Trp Phe Gly Ile Pro Ser Ala Leu Arg Arg Asp
        25                  30                  35

Gly Ala Gln Val Tyr Val Thr Glu Val Ser Gln Leu Asp Thr Ser Glu
    40                  45                  50

Val Arg Gly Glu Gln Leu Leu Gln Gln Val Glu Glu Ile Val Ala Leu
55                  60                  65                  70

Ser Gly Gln Pro Lys Val Asn Leu Ile Gly His Ser His Gly Gly Pro
                75                  80                  85

Thr Ile Arg Tyr Val Ala Ala Val Arg Pro Asp Leu Ile Ala Ser Ala
            90                  95                  100

Ile Ser Val Gly Ala Pro His Lys Gly Ser Asp Thr Ala Asp Phe Leu
        105                 110                 115

Arg Gln Ile Pro Pro Gly Ser Ala Gly Glu Ala Val Leu Ser Gly Leu
    120                 125                 130

Val Asn Ser Leu Gly Ala Leu Ile Ser Phe Leu Ser Ser Gly Gly Thr
135                 140                 145                 150

Gly Thr Gln Asn Ser Leu Gly Ser Leu Glu Ser Leu Asn Ser Glu Gly
                155                 160                 165

Ala Ala Arg Phe Asn Ala Lys Tyr Pro Gln Gly Ile Pro Thr Ser Ala
            170                 175                 180

Cys Gly Glu Gly Ala Tyr Lys Val Asn Gly Val Ser Tyr Tyr Ser Trp
        185                 190                 195

Ser Gly Ser Ser Pro Leu Thr Asn Phe Leu Asp Pro Ser Asp Ala Phe
    200                 205                 210

Leu Gly Ala Ser Ser Leu Thr Phe Lys Asn Gly Thr Ala Asn Asp Gly
215                 220                 225                 230

Leu Val Gly Thr Cys Ser Ser His Leu Gly Met Val Ile Arg Asp Asn
                235                 240                 245

Tyr Arg Met Asn His Leu Asp Glu Val Asn Gln Val Phe Gly Leu Thr
            250                 255                 260

Ser Leu Phe Glu Thr Ser Pro Val Ser Val Tyr Arg Gln His Ala Asn
        265                 270                 275

Arg Leu Lys Asn Ala Ser Leu
    280                 285
```

(2) ANGABEN ZU SEQ ID NO: 5:

(i) SEQUENZKENNZEICHEN:

(A) LÄNGE: 1049 Basenpaare
(B) ART: Nucleotid
(C) STRANGFORM: nicht bekannt

(D) TOPOLOGIE: nicht bekannt

(ii) ART DES MOLEKÜLS: Genom-DNA

(ix) MERKMAL:

(A) NAME/SCHLÜSSEL: CDS
(B) LAGE:85..1017

(ix) MERKMAL:

(A) NAME/SCHLÜSSEL: mat_peptide
(B) LAGE:163..1017

(xi) SEQUENZBESCHREIBUNG: SEQ ID NO: 5:

```
GGATCCCCCG GTTCTCCCGG AAGGATTCGG GCGATGGCTG GCAGGACGCG CCCCTCGGCC     60

CCATCAACCT GAGATGAGAA CAAC ATG AAG AAG AAG TCT CTG CTC CCC CTC       111
                           Met Lys Lys Lys Ser Leu Leu Pro Leu
                           -26 -25                 -20

GGC CTG GCC ATC GGT CTC GCC TCT CTC GCT GCC AGC CCT CTG ATC CAG      159
Gly Leu Ala Ile Gly Leu Ala Ser Leu Ala Ala Ser Pro Leu Ile Gln
        -15                 -10                 -5

GCC AGC ACC TAC ACC CAG ACC AAA TAC CCC ATC GTG CTG GCC CAC GGC      207
Ala Ser Thr Tyr Thr Gln Thr Lys Tyr Pro Ile Val Leu Ala His Gly
      1               5                  10                  15

ATG CTC GGC TTC GAC AAC ATC CTC GGG GTC GAC TAC TGG TTC GGC ATT      255
Met Leu Gly Phe Asp Asn Ile Leu Gly Val Asp Tyr Trp Phe Gly Ile
                 20                  25                  30

CCC AGC GCC TTG CGC CGT GAC GGT GCC CAG GTC TAC GTC ACC GAA GTC      303
Pro Ser Ala Leu Arg Arg Asp Gly Ala Gln Val Tyr Val Thr Glu Val
             35                  40                  45

AGC CAG TTG GAC ACC TCG GAA GTC CGC GGC GAG CAG TTG CTG CAA CAG      351
Ser Gln Leu Asp Thr Ser Glu Val Arg Gly Glu Gln Leu Leu Gln Gln
         50                  55                  60

GTG GAG GAA ATC GTC GCC CTC AGC GGC CAG CCC AAG GTC AAC CTG ATC      399
Val Glu Glu Ile Val Ala Leu Ser Gly Gln Pro Lys Val Asn Leu Ile
     65                  70                  75

GGC CAC AGC CAC GGC GGG CCG ACC ATC CGC TAC GTC GCC GCC GTA CGT      447
Gly His Ser His Gly Gly Pro Thr Ile Arg Tyr Val Ala Ala Val Arg
 80                  85                  90                  95

CCC GAC CTG ATC GCT TCC GCC ACC AGC GTC GGC GCC CCG CAC AAG GGT      495
Pro Asp Leu Ile Ala Ser Ala Thr Ser Val Gly Ala Pro His Lys Gly
                100                 105                 110

TCG GAC ACC GCC GAC TTC CTG CGC CAG ATC CCA CCG GGT TCG GCC GGC      543
Ser Asp Thr Ala Asp Phe Leu Arg Gln Ile Pro Pro Gly Ser Ala Gly
            115                 120                 125

GAG GCA GTC CTC TCC GGG CTG GTC AAC AGC CTC GGC GCG CTG ATC AGC      591
Glu Ala Val Leu Ser Gly Leu Val Asn Ser Leu Gly Ala Leu Ile Ser
        130                 135                 140
```

```
TTC CTT TCC AGC GGC GGC ACC GGT ACG CAG AAT TCA CTG GGC TCG CTG      639
Phe Leu Ser Ser Gly Gly Thr Gly Thr Gln Asn Ser Leu Gly Ser Leu
    145                 150                 155

GAG TCG CTG AAC AGC GAG GGT GCC GCG CGC TTC AAC GCC AAG TAC CCG      687
Glu Ser Leu Asn Ser Glu Gly Ala Ala Arg Phe Asn Ala Lys Tyr Pro
160                 165                 170                 175

CAG GGC ATC CCC ACC TCG GCC TGC GGC GAA GGC GCC TAC AAG GTC AAC      735
Gln Gly Ile Pro Thr Ser Ala Cys Gly Glu Gly Ala Tyr Lys Val Asn
                180                 185                 190

GGC GTG AGC TAT TAC TCC TGG AGC GGT TCC TCG CCG CTG ACC AAC TTC      783
Gly Val Ser Tyr Tyr Ser Trp Ser Gly Ser Ser Pro Leu Thr Asn Phe
            195                 200                 205

CTC GAT CCG AGC GAC GCC TTC CTC GGC GCC TCG TCG CTG ACC TTC AAG      831
Leu Asp Pro Ser Asp Ala Phe Leu Gly Ala Ser Ser Leu Thr Phe Lys
        210                 215                 220

AAC GGC ACC GCC AAC GAC GGC CTG GTC GGC ACC TGC AGT TCG CAC CTG      879
Asn Gly Thr Ala Asn Asp Gly Leu Val Gly Thr Cys Ser Ser His Leu
    225                 230                 235

GGC ATG GTG ATC CGC GAC AAC TAC CGG ATG AAC CAC CTG GAC GAG GTG      927
Gly Met Val Ile Arg Asp Asn Tyr Arg Met Asn His Leu Asp Glu Val
240                 245                 250                 255

AAC CAG GTC TTC GGC CTC ACC AGC CTG TTC GAG ACC AGC CCG GTC AGC      975
Asn Gln Val Phe Gly Leu Thr Ser Leu Phe Glu Thr Ser Pro Val Ser
                260                 265                 270

GTC TAC CGC CAG CAC GCC AAC CGC CTG AAG AAC GCC AGC CTG             1017
Val Tyr Arg Gln His Ala Asn Arg Leu Lys Asn Ala Ser Leu
            275                 280                 285

TAGGACCCCG GCCGGGGCCT CGGCCCGGGC CC                                 1049
```

(2) ANGABEN ZU SEQ ID NO: 6:

(i) SEQUENZKENNZEICHEN:

(A) LÄNGE: 311 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 6:

```
Met Lys Lys Lys Ser Leu Leu Pro Leu Gly Leu Ala Ile Gly Leu Ala
-26 -25                 -20                 -15

Ser Leu Ala Ala Ser Pro Leu Ile Gln Ala Ser Thr Tyr Thr Gln Thr
-10                  -5                   1                   5

Lys Tyr Pro Ile Val Leu Ala His Gly Met Leu Gly Phe Asp Asn Ile
                 10                  15                  20
```

```
Leu Gly Val Asp Tyr Trp Phe Gly Ile Pro Ser Ala Leu Arg Arg Asp
        25                  30                  35

Gly Ala Gln Val Tyr Val Thr Glu Val Ser Gln Leu Asp Thr Ser Glu
    40                  45                  50

Val Arg Gly Glu Gln Leu Leu Gln Gln Val Glu Glu Ile Val Ala Leu
 55                 60                  65                      70

Ser Gly Gln Pro Lys Val Asn Leu Ile Gly His Ser His Gly Gly Pro
                75                  80                  85

Thr Ile Arg Tyr Val Ala Ala Val Arg Pro Asp Leu Ile Ala Ser Ala
            90                  95                  100

Thr Ser Val Gly Ala Pro His Lys Gly Ser Asp Thr Ala Asp Phe Leu
        105                 110                 115

Arg Gln Ile Pro Pro Gly Ser Ala Gly Glu Ala Val Leu Ser Gly Leu
    120                 125                 130

Val Asn Ser Leu Gly Ala Leu Ile Ser Phe Leu Ser Ser Gly Gly Thr
135                 140                 145                     150

Gly Thr Gln Asn Ser Leu Gly Ser Leu Glu Ser Leu Asn Ser Glu Gly
                155                 160                 165

Ala Ala Arg Phe Asn Ala Lys Tyr Pro Gln Gly Ile Pro Thr Ser Ala
            170                 175                 180

Cys Gly Glu Gly Ala Tyr Lys Val Asn Gly Val Ser Tyr Tyr Ser Trp
        185                 190                 195

Ser Gly Ser Ser Pro Leu Thr Asn Phe Leu Asp Pro Ser Asp Ala Phe
    200                 205                 210

Leu Gly Ala Ser Ser Leu Thr Phe Lys Asn Gly Thr Ala Asn Asp Gly
215                 220                 225                     230

Leu Val Gly Thr Cys Ser Ser His Leu Gly Met Val Ile Arg Asp Asn
                235                 240                 245

Tyr Arg Met Asn His Leu Asp Glu Val Asn Gln Val Phe Gly Leu Thr
            250                 255                 260

Ser Leu Phe Glu Thr Ser Pro Val Ser Val Tyr Arg Gln His Ala Asn
        265                 270                 275

Arg Leu Lys Asn Ala Ser Leu
    280                 285
```

(2) ANGABEN ZU SEQ ID NO: 7:

(i) SEQUENZKENNZEICHEN:

(A) LÄNGE: 1049 Basenpaare
(B) ART: Nucleotid
(C) STRANGFORM: nicht bekannt
(D) TOPOLOGIE: nicht bekannt

(ii) ART DES MOLEKÜLS: Genom-DNA

(ix) MERKMAL:

(A) NAME/SCHLÜSSEL: CDS
(B) LAGE:85..1017

(ix) MERKMAL:

(A) NAME/SCHLÜSSEL: mat_peptide
(B) LAGE:163..1017

(xi) SEQUENZBESCHREIBUNG: SEQ ID NO: 7:

```
GGATCCCCCG GTTCTCCCGG AAGGATTCGG GCGATGGCTG GCAGGACGCG CCCCTCGGCC      60

CCATCAACCT GAGATGAGAA CAAC ATG AAG AAG AAG TCT CTG CTC CCC CTC        111
                           Met Lys Lys Lys Ser Leu Leu Pro Leu
                           -26 -25                 -20

GGC CTG GCC ATC GGT CTC GCC TCT CTC GCT GCC AGC CCT CTG ATC CAG       159
Gly Leu Ala Ile Gly Leu Ala Ser Leu Ala Ala Ser Pro Leu Ile Gln
        -15                 -10                 -5

GCC AGC ACC TAC ACC CAG ACC AAA TAC CCC ATC GTG CTG GCC CAC GGC       207
Ala Ser Thr Tyr Thr Gln Thr Lys Tyr Pro Ile Val Leu Ala His Gly
    1               5                   10                  15

ATG CTC GGC TTC GAC AAC ATC CTT GGG GTC GAC TAC TGG TTC GGC ATT       255
Met Leu Gly Phe Asp Asn Ile Leu Gly Val Asp Tyr Trp Phe Gly Ile
                20                  25                  30

CCC AGC GCC TTG CGC CGT GAC GGT GCC CAG GTC TAC GTC ACC GAA GTC       303
Pro Ser Ala Leu Arg Arg Asp Gly Ala Gln Val Tyr Val Thr Glu Val
            35                  40                  45

AGC CAG TTG GAC ACC TCG GAA GTC CGC GGC GAG CAG TTG CTG CAA CAG       351
Ser Gln Leu Asp Thr Ser Glu Val Arg Gly Glu Gln Leu Leu Gln Gln
        50                  55                  60

GTG GAG GAA ATC GTC GCC CTC AGC GGC CAG CCC AAG GTC AAC CTG ATC       399
Val Glu Glu Ile Val Ala Leu Ser Gly Gln Pro Lys Val Asn Leu Ile
    65                  70                  75

GGC CAC AGC CAC GGC GGG CCG ACC ATC CGC TAC GTC GCC GCC GTA CGT       447
Gly His Ser His Gly Gly Pro Thr Ile Arg Tyr Val Ala Ala Val Arg
80                  85                  90                  95

CCC GAC CTG ATC GCT TCC GCC ACC AGC GTC GGC GCC CCG CAC AAG GGT       495
Pro Asp Leu Ile Ala Ser Ala Thr Ser Val Gly Ala Pro His Lys Gly
                100                 105                 110

TCG GAC ACC GCC GAC TTC CTG CGC CAG ATC CCA CCG GGT TCG GCC GGC       543
Ser Asp Thr Ala Asp Phe Leu Arg Gln Ile Pro Pro Gly Ser Ala Gly
            115                 120                 125

GAG GCA GTC CTC TCC GGG CTG GTC AAC AGC CTC GGC GCG CTG ATC AGC       591
Glu Ala Val Leu Ser Gly Leu Val Asn Ser Leu Gly Ala Leu Ile Ser
        130                 135                 140
```

```
TTC CTT TCC AGC GGC GGC ACC GGT ACG CAG AAT TTA CTG GGC TCG CTG      639
Phe Leu Ser Ser Gly Gly Thr Gly Thr Gln Asn Leu Leu Gly Ser Leu
    145                 150                 155

GAG TCG CTG AAC AGC GAG GGT GCC GCG CGC TTC AAC GCC AAG TAC CCG      687
Glu Ser Leu Asn Ser Glu Gly Ala Ala Arg Phe Asn Ala Lys Tyr Pro
160                 165                 170                 175

CAG GGC ATC CCC ACC TCG GCC TGC GGC GAA GGC GCC TAC AAG GTC AAC      735
Gln Gly Ile Pro Thr Ser Ala Cys Gly Glu Gly Ala Tyr Lys Val Asn
                180                 185                 190

GGC GTG AGC TAT TAC TCC TGG AGC GGT TCC TCG CCG CTG ACC AAC TTC      783
Gly Val Ser Tyr Tyr Ser Trp Ser Gly Ser Ser Pro Leu Thr Asn Phe
            195                 200                 205

CTC GAT CCG AGC GAC GCC TTC CTC GGC GCC TCG TCG CTG ACC TTC AAG      831
Leu Asp Pro Ser Asp Ala Phe Leu Gly Ala Ser Ser Leu Thr Phe Lys
        210                 215                 220

AAC GGC ACC GCC AAC GAC GGC CTG GTC GGC ACC TGC AGT TCG CAC CTG      879
Asn Gly Thr Ala Asn Asp Gly Leu Val Gly Thr Cys Ser Ser His Leu
    225                 230                 235

GGC ATG GTG ATC CGC GAC AAC TAC CGG ATG AAC CAC CTG GAC GAG GTG      927
Gly Met Val Ile Arg Asp Asn Tyr Arg Met Asn His Leu Asp Glu Val
240                 245                 250                 255

AAC CAG GTC TTC GGC CTC ACC AGC CTG TTC GAG ACC AGC CCG GTC AGC      975
Asn Gln Val Phe Gly Leu Thr Ser Leu Phe Glu Thr Ser Pro Val Ser
                260                 265                 270

GTC TAC CGC CAG CAC GCC AAC CGC CTG AAG AAC GCC AGC CTG             1017
Val Tyr Arg Gln His Ala Asn Arg Leu Lys Asn Ala Ser Leu
            275                 280                 285

TAGGACCCCG GCCGGGGCCT CGGCCCGGGC CC                                 1049
```

(2) ANGABEN ZU SEQ ID NO: 8:

(i) SEQUENZKENNZEICHEN:

(A) LÄNGE: 311 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 8:

```
Met Lys Lys Lys Ser Leu Leu Pro Leu Gly Leu Ala Ile Gly Leu Ala
-26 -25                 -20                 -15

Ser Leu Ala Ala Ser Pro Leu Ile Gln Ala Ser Thr Tyr Thr Gln Thr
-10                  -5                   1               5

Lys Tyr Pro Ile Val Leu Ala His Gly Met Leu Gly Phe Asp Asn Ile
            10                  15                  20

Leu Gly Val Asp Tyr Trp Phe Gly Ile Pro Ser Ala Leu Arg Arg Asp
        25                  30                  35
```

```
Gly Ala Gln Val Tyr Val Thr Glu Val Ser Gln Leu Asp Thr Ser Glu
     40                  45                  50

Val Arg Gly Glu Gln Leu Leu Gln Gln Val Glu Glu Ile Val Ala Leu
 55                  60                  65                  70

Ser Gly Gln Pro Lys Val Asn Leu Ile Gly His Ser His Gly Gly Pro
                 75                  80                  85

Thr Ile Arg Tyr Val Ala Ala Val Arg Pro Asp Leu Ile Ala Ser Ala
             90                  95                 100

Thr Ser Val Gly Ala Pro His Lys Gly Ser Asp Thr Ala Asp Phe Leu
        105                 110                 115

Arg Gln Ile Pro Pro Gly Ser Ala Gly Glu Ala Val Leu Ser Gly Leu
    120                 125                 130

Val Asn Ser Leu Gly Ala Leu Ile Ser Phe Leu Ser Ser Gly Gly Thr
135                 140                 145                 150

Gly Thr Gln Asn Leu Leu Gly Ser Leu Glu Ser Leu Asn Ser Glu Gly
                155                 160                 165

Ala Ala Arg Phe Asn Ala Lys Tyr Pro Gln Gly Ile Pro Thr Ser Ala
            170                 175                 180

Cys Gly Glu Gly Ala Tyr Lys Val Asn Gly Val Ser Tyr Tyr Ser Trp
        185                 190                 195

Ser Gly Ser Ser Pro Leu Thr Asn Phe Leu Asp Pro Ser Asp Ala Phe
    200                 205                 210

Leu Gly Ala Ser Ser Leu Thr Phe Lys Asn Gly Thr Ala Asn Asp Gly
215                 220                 225                 230

Leu Val Gly Thr Cys Ser Ser His Leu Gly Met Val Ile Arg Asp Asn
                235                 240                 245

Tyr Arg Met Asn His Leu Asp Glu Val Asn Gln Val Phe Gly Leu Thr
            250                 255                 260

Ser Leu Phe Glu Thr Ser Pro Val Ser Val Tyr Arg Gln His Ala Asn
        265                 270                 275

Arg Leu Lys Asn Ala Ser Leu
    280                 285
```

(2) ANGABEN ZU SEQ ID NO: 9:

(i) SEQUENZKENNZEICHEN:

(A) LÄNGE: 1047 Basenpaare
(B) ART: Nucleotid
(C) STRANGFORM: nicht bekannt
(D) TOPOLOGIE: nicht bekannt

(ii) ART DES MOLEKÜLS: Genom-DNA

(ix) MERKMAL:

(A) NAME/SCHLÜSSEL: CDS
(B) LAGE:84..1016

(ix) MERKMAL:

(A) NAME/SCHLÜSSEL: mat_peptide
(B) LAGE:162..1016

(xi) SEQUENZBESCHREIBUNG: SEQ ID NO: 9:

```
GGATCCCCGG TTCTCCCGGA AGGATTCGGG CGATGGCTGG CAGGACGCGC CCCTCGGCCC     60

CATCAACCTG AGATGAGAAC AAC ATG AAG AAG AAG TCT CTG CTC CCC CTC       110
                          Met Lys Lys Lys Ser Leu Leu Pro Leu
                          -26 -25                 -20

GGC CTG GCC ATC GGT CTC GCC TCT CTC GCT GCC AGC CCT CTG ATC CAG      158
Gly Leu Ala Ile Gly Leu Ala Ser Leu Ala Ala Ser Pro Leu Ile Gln
    -15                     -10                     -5

GCC AGC ACC TAC ACC CAG ACC AAA TAC CCC ATC GTG CTG GCC CAC GGC      206
Ala Ser Thr Tyr Thr Gln Thr Lys Tyr Pro Ile Val Leu Ala His Gly
    1               5                   10                  15

ATG CTC GGC TTC GAC AAC ATC CTC GGG GTC GAC TAC TGG TTC GGC ATT      254
Met Leu Gly Phe Asp Asn Ile Leu Gly Val Asp Tyr Trp Phe Gly Ile
                20                  25                  30

CCC AGC GCC TTG CGC CGT GAC GGT GCC CAG GTC TAC GTC ACC GAA GTC      302
Pro Ser Ala Leu Arg Arg Asp Gly Ala Gln Val Tyr Val Thr Glu Val
            35                  40                  45

AGC CAG TTG GAC ACC TCG GAA GTC CGC GGC GAG CAG TTG CTG CAA CAG      350
Ser Gln Leu Asp Thr Ser Glu Val Arg Gly Glu Gln Leu Leu Gln Gln
        50                  55                  60

GTG GAG GAA ATC GTC GCC CTC AGC GGC CAG CCC AAG GTC AAC CTG ATC      398
Val Glu Glu Ile Val Ala Leu Ser Gly Gln Pro Lys Val Asn Leu Ile
    65                  70                  75

GGC CAC AGC CAC GGC GGG CCG ACC ATC CGC TAC GTC GCC GCC GTA CGT      446
Gly His Ser His Gly Gly Pro Thr Ile Arg Tyr Val Ala Ala Val Arg
80                  85                  90                  95

CCC GAC CTG ATC GCT TCC GCC ACC AGC GTC GGC GCC CCG CAC AAG GGT      494
Pro Asp Leu Ile Ala Ser Ala Thr Ser Val Gly Ala Pro His Lys Gly
                100                 105                 110

TCG GAC ACC GCC GAC TTC CTG CGC CAG ATC CCA CCG GGT TCG GCC GGC      542
Ser Asp Thr Ala Asp Phe Leu Arg Gln Ile Pro Pro Gly Ser Ala Gly
            115                 120                 125

GAG GCA GTC CTC TCC GGG CTG GTC AAC AGC CTC GGC GCG CTG ATC AGC      590
Glu Ala Val Leu Ser Gly Leu Val Asn Ser Leu Gly Ala Leu Ile Ser
        130                 135                 140

TTC CTT TCC AGC GGC AGC ACC GGT ACG CAG AAT TCA CTG GGC TCG CTG      638
Phe Leu Ser Ser Gly Ser Thr Gly Thr Gln Asn Ser Leu Gly Ser Leu
    145                 150                 155
```

```
GAG TCG CTG AAC AGC GAG GGT GCC GCG CGC TTC AAC GCC AAG TAC CCG      686
Glu Ser Leu Asn Ser Glu Gly Ala Ala Arg Phe Asn Ala Lys Tyr Pro
160                 165                 170                 175

CAG GGC ATC CCC ACC TCG GCC TGC GGC GAA GGC GCC TAC AAG GTC AAC      734
Gln Gly Ile Pro Thr Ser Ala Cys Gly Glu Gly Ala Tyr Lys Val Asn
                180                 185                 190

GGC GTG AGC TAT TAC TCC TGG AGC GGT TCC TCG CCG CTG ACC AAC TTC      782
Gly Val Ser Tyr Tyr Ser Trp Ser Gly Ser Ser Pro Leu Thr Asn Phe
            195                 200                 205

CTC GAT CCG AGC GAC GCC TTC CTC GGC GCC TCG TCG CTG ACC TTC AAG      830
Leu Asp Pro Ser Asp Ala Phe Leu Gly Ala Ser Ser Leu Thr Phe Lys
        210                 215                 220

AAC GGC ACC GCC AAC GAC GGC CTG GTC GGC ACC TGC AGT TCG CAC CTG      878
Asn Gly Thr Ala Asn Asp Gly Leu Val Gly Thr Cys Ser Ser His Leu
    225                 230                 235

GGC ATG GTG ATC CGC GAC AAC TAC CGG ATG AAC CAC CTG GAC GAG GTG      926
Gly Met Val Ile Arg Asp Asn Tyr Arg Met Asn His Leu Asp Glu Val
240                 245                 250                 255

AAC CAG GTC TTC GGC CTC ACC AGC CTG TTC GAG ACC AGC CCG GTC AGC      974
Asn Gln Val Phe Gly Leu Thr Ser Leu Phe Glu Thr Ser Pro Val Ser
                260                 265                 270

GTC TAC CGC CAG CAC GCC AAC CGC CTG AAG AAC GCC AGC CTG             1016
Val Tyr Arg Gln His Ala Asn Arg Leu Lys Asn Ala Ser Leu
            275                 280                 285

TAGGACCCCG GCCGGGGCCT CGGCCCGGGC C                                  1047
```

(2) ANGABEN ZU SEQ ID NO: 10:

(i) SEQUENZKENNZEICHEN:

(A) LÄNGE: 311 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 10:

```
Met Lys Lys Lys Ser Leu Leu Pro Leu Gly Leu Ala Ile Gly Leu Ala
-26 -25                 -20                 -15

Ser Leu Ala Ala Ser Pro Leu Ile Gln Ala Ser Thr Tyr Thr Gln Thr
-10                 -5                  1               5

Lys Tyr Pro Ile Val Leu Ala His Gly Met Leu Gly Phe Asp Asn Ile
            10                  15                  20

Leu Gly Val Asp Tyr Trp Phe Gly Ile Pro Ser Ala Leu Arg Arg Asp
        25                  30                  35
```

```
Gly Ala Gln Val Tyr Val Thr Glu Val Ser Gln Leu Asp Thr Ser Glu
     40                  45                  50

Val Arg Gly Glu Gln Leu Leu Gln Gln Val Glu Glu Ile Val Ala Leu
 55                  60                  65                  70

Ser Gly Gln Pro Lys Val Asn Leu Ile Gly His Ser His Gly Gly Pro
                 75                  80                  85

Thr Ile Arg Tyr Val Ala Ala Val Arg Pro Asp Leu Ile Ala Ser Ala
             90                  95                 100

Thr Ser Val Gly Ala Pro His Lys Gly Ser Asp Thr Ala Asp Phe Leu
        105                 110                 115

Arg Gln Ile Pro Pro Gly Ser Ala Gly Glu Ala Val Leu Ser Gly Leu
    120                 125                 130

Val Asn Ser Leu Gly Ala Leu Ile Ser Phe Leu Ser Ser Gly Ser Thr
135                 140                 145                 150

Gly Thr Gln Asn Ser Leu Gly Ser Leu Glu Ser Leu Asn Ser Glu Gly
                155                 160                 165

Ala Ala Arg Phe Asn Ala Lys Tyr Pro Gln Gly Ile Pro Thr Ser Ala
            170                 175                 180

Cys Gly Glu Gly Ala Tyr Lys Val Asn Gly Val Ser Tyr Tyr Ser Trp
        185                 190                 195

Ser Gly Ser Ser Pro Leu Thr Asn Phe Leu Asp Pro Ser Asp Ala Phe
    200                 205                 210

Leu Gly Ala Ser Ser Leu Thr Phe Lys Asn Gly Thr Ala Asn Asp Gly
215                 220                 225                 230

Leu Val Gly Thr Cys Ser Ser His Leu Gly Met Val Ile Arg Asp Asn
                235                 240                 245

Tyr Arg Met Asn His Leu Asp Glu Val Asn Gln Val Phe Gly Leu Thr
            250                 255                 260

Ser Leu Phe Glu Thr Ser Pro Val Ser Val Tyr Arg Gln His Ala Asn
        265                 270                 275

Arg Leu Lys Asn Ala Ser Leu
    280                 285
```

(2) ANGABEN ZU SEQ ID NO: 11:

(i) SEQUENZKENNZEICHEN:

(A) LÄNGE: 1050 Basenpaare
(B) ART: Nucleotid
(C) STRANGFORM: nicht bekannt
(D) TOPOLOGIE: nicht bekannt

(ii) ART DES MOLEKÜLS: Genom-DNA

(ix) MERKMAL:

(A) NAME/SCHLÜSSEL: CDS
(B) LAGE:85..1017

(ix) MERKMAL:

(A) NAME/SCHLÜSSEL: mat_peptide
(B) LAGE:163..1017

(xi) SEQUENZBESCHREIBUNG: SEQ ID NO: 11:

```
GGATCCCCCG GTTCTCCCGG AAGGATTCGG GCGATGGCTG GCAGGACGCG CCCCTCGGCC    60

CCATCAACCT GAGATGAGAA CAAC ATG AAG AAG AAG TCT CTG CTC CCC CTC      111
                           Met Lys Lys Lys Ser Leu Leu Pro Leu
                           -26 -25                 -20

GGC CTG GCC ATC GGT CTC GCC TCT CTC GCT GCC AGC CCT CTG ATC CAG      159
Gly Leu Ala Ile Gly Leu Ala Ser Leu Ala Ala Ser Pro Leu Ile Gln
        -15                 -10                 -5

GCC AGC ACC TAC ACC CAG ACC AAA TAC CCC ATC GTG CTG GCC CAC GGC      207
Ala Ser Thr Tyr Thr Gln Thr Lys Tyr Pro Ile Val Leu Ala His Gly
    1               5                   10                  15

ATG CTC GGC TTC GAC AAC ATC CTT GGG GTC GAC TAC TGG TTC GGC ATT      255
Met Leu Gly Phe Asp Asn Ile Leu Gly Val Asp Tyr Trp Phe Gly Ile
                20                  25                  30

CCC AAC GCC TTG CGC CGT GAC GGT GCC CAG GTC TAC GTC ACC GAA GGC      303
Pro Asn Ala Leu Arg Arg Asp Gly Ala Gln Val Tyr Val Thr Glu Gly
            35                  40                  45

AGC CAG TTG GAC ACC TCG GAA GTC CGC GGC GAG CAG TTG CTG CAA CAG      351
Ser Gln Leu Asp Thr Ser Glu Val Arg Gly Glu Gln Leu Leu Gln Gln
        50                  55                  60

GTG GAG GAA ATC GTC GCC CTC AGC GGC CAG CCC AAG GTC AAC CTG ATC      399
Val Glu Glu Ile Val Ala Leu Ser Gly Gln Pro Lys Val Asn Leu Ile
    65                  70                  75

GGC CAC AGC CAC GGC GGG CCG ACC ATC CGC TAC GTC GCC GCC GTA CGT      447
Gly His Ser His Gly Gly Pro Thr Ile Arg Tyr Val Ala Ala Val Arg
80                  85                  90                  95

CCC GAC CTG ATC GCT TCC GCC ACC AGC GTC GGC GCC CCG CAC AAG GGT      495
Pro Asp Leu Ile Ala Ser Ala Thr Ser Val Gly Ala Pro His Lys Gly
                100                 105                 110

TCG GAC ACC GCC GAC TTC CTG CGC CAG ATC CCA CCG GGT TCG GCC GGC      543
Ser Asp Thr Ala Asp Phe Leu Arg Gln Ile Pro Pro Gly Ser Ala Gly
            115                 120                 125

GAG GCA GTC CTC TCC GGG CTG GTC AAC AGC CTC GGC GCG CTG ATC AGC      591
Glu Ala Val Leu Ser Gly Leu Val Asn Ser Leu Gly Ala Leu Ile Ser
        130                 135                 140

TTC CTT TCC AGC GGC GGC ACC GGT ACG CAG AAT TTA CTG GGC TCG CTG      639
Phe Leu Ser Ser Gly Gly Thr Gly Thr Gln Asn Leu Leu Gly Ser Leu
    145                 150                 155
```

```
GAG TCG CTG AAC AGC GAG GGT GCC GCG CGC TTC AAC GCC AAG TAC CCG      687
Glu Ser Leu Asn Ser Glu Gly Ala Ala Arg Phe Asn Ala Lys Tyr Pro
160                 165                 170                 175

CAG GGC ATC CCC ACC TCG GCC TGC GGC GAA GGC GCT TAC AAG GTC AAC      735
Gln Gly Ile Pro Thr Ser Ala Cys Gly Glu Gly Ala Tyr Lys Val Asn
                180                 185                 190

GGC GTG AGC TAT TAC TCC TGG AGC GGT TCC TCG CCG CTG ACC AAC TTC      783
Gly Val Ser Tyr Tyr Ser Trp Ser Gly Ser Ser Pro Leu Thr Asn Phe
            195                 200                 205

CTC GAT CCG AGC GAC GCC TTC CTC GGC GCC TCG TCG CTG ACC TTC AAG      831
Leu Asp Pro Ser Asp Ala Phe Leu Gly Ala Ser Ser Leu Thr Phe Lys
        210                 215                 220

AAC GGC ACC GCC AAC GAC GGC CTG GTC GGC ACC TGC AGT TCG CAC CTG      879
Asn Gly Thr Ala Asn Asp Gly Leu Val Gly Thr Cys Ser Ser His Leu
    225                 230                 235

GGC ATG GTG ATC CGC GAC AAC TAC CGG ATG AAC CAC CTG GAC GAG GTG      927
Gly Met Val Ile Arg Asp Asn Tyr Arg Met Asn His Leu Asp Glu Val
240                 245                 250                 255

AAC CAG GTC CTC GGC CTC ACC AGC CTG TTC GAG ACC AGC CCG GTC AGC      975
Asn Gln Val Leu Gly Leu Thr Ser Leu Phe Glu Thr Ser Pro Val Ser
                260                 265                 270

GTC TAC CGC CAG CAC GCC AAC CGC CTG AAG AAC GCC AGC CTG             1017
Val Tyr Arg Gln His Ala Asn Arg Leu Lys Asn Ala Ser Leu
            275                 280                 285

TAGGACCCCG GCCGGGGCCT CGGCCCGGGC CCG                                1050
```

(2) ANGABEN ZU SEQ ID NO: 12:

(i) SEQUENZKENNZEICHEN:

(A) LÄNGE: 311 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 12:

```
Met Lys Lys Lys Ser Leu Leu Pro Leu Gly Leu Ala Ile Gly Leu Ala
-26 -25                 -20                 -15

Ser Leu Ala Ala Ser Pro Leu Ile Gln Ala Ser Thr Tyr Thr Gln Thr
-10                  -5                   1               5

Lys Tyr Pro Ile Val Leu Ala His Gly Met Leu Gly Phe Asp Asn Ile
                 10                  15                  20

Leu Gly Val Asp Tyr Trp Phe Gly Ile Pro Asn Ala Leu Arg Arg Asp
            25                  30                  35
```

```
Gly Ala Gln Val Tyr Val Thr Glu Gly Ser Gln Leu Asp Thr Ser Glu
     40                  45                  50

Val Arg Gly Glu Gln Leu Leu Gln Gln Val Glu Glu Ile Val Ala Leu
 55                  60                  65                  70

Ser Gly Gln Pro Lys Val Asn Leu Ile Gly His Ser His Gly Gly Pro
                 75                  80                  85

Thr Ile Arg Tyr Val Ala Ala Val Arg Pro Asp Leu Ile Ala Ser Ala
             90                  95                 100

Thr Ser Val Gly Ala Pro His Lys Gly Ser Asp Thr Ala Asp Phe Leu
        105                 110                 115

Arg Gln Ile Pro Pro Gly Ser Ala Gly Glu Ala Val Leu Ser Gly Leu
    120                 125                 130

Val Asn Ser Leu Gly Ala Leu Ile Ser Phe Leu Ser Ser Gly Gly Thr
135                 140                 145                 150

Gly Thr Gln Asn Leu Leu Gly Ser Leu Glu Ser Leu Asn Ser Glu Gly
                155                 160                 165

Ala Ala Arg Phe Asn Ala Lys Tyr Pro Gln Gly Ile Pro Thr Ser Ala
            170                 175                 180

Cys Gly Glu Gly Ala Tyr Lys Val Asn Gly Val Ser Tyr Tyr Ser Trp
        185                 190                 195

Ser Gly Ser Ser Pro Leu Thr Asn Phe Leu Asp Pro Ser Asp Ala Phe
    200                 205                 210

Leu Gly Ala Ser Ser Leu Thr Phe Lys Asn Gly Thr Ala Asn Asp Gly
215                 220                 225                 230

Leu Val Gly Thr Cys Ser Ser His Leu Gly Met Val Ile Arg Asp Asn
                235                 240                 245

Tyr Arg Met Asn His Leu Asp Glu Val Asn Gln Val Leu Gly Leu Thr
            250                 255                 260

Ser Leu Phe Glu Thr Ser Pro Val Ser Val Tyr Arg Gln His Ala Asn
        265                 270                 275

Arg Leu Lys Asn Ala Ser Leu
    280                 285
```

(2) ANGABEN ZU SEQ ID NO: 13:

(i) SEQUENZKENNZEICHEN:

(A) LÄNGE: 1049 Basenpaare
(B) ART: Nucleotid
(C) STRANGFORM: nicht bekannt
(D) TOPOLOGIE: nicht bekannt

(ii) ART DES MOLEKÜLS: Genom-DNA

(ix) MERKMAL:

(A) NAME/SCHLÜSSEL: CDS
(B) LAGE:85..1017

(ix) MERKMAL:

(A) NAME/SCHLÜSSEL: mat_peptide
(B) LAGE:163..1017

(xi) SEQUENZBESCHREIBUNG: SEQ ID NO: 13:

```
GGATCCCCCG GTTCTCCCGG AAGGATTCGG GCGATGGCTG GCAGGACGCG CCCCTCGGCC     60

CCATCAACCT GAGATGAGAA CAAC ATG AAG AAG AAG TCT CTG CTC CCC CTC       111
                           Met Lys Lys Lys Ser Leu Leu Pro Leu
                           -26 -25                 -20

GGC CTG GCC ATC GGT CTC GCC TCT CTC GCT GCC AGC CCT CTG ATC CAG      159
Gly Leu Ala Ile Gly Leu Ala Ser Leu Ala Ala Ser Pro Leu Ile Gln
        -15                 -10                 -5

GCC AGC ACC TAC ACC CAG ACC AAA TAC CCC ATC GTG CTG GCC CAC GGC      207
Ala Ser Thr Tyr Thr Gln Thr Lys Tyr Pro Ile Val Leu Ala His Gly
     1               5                  10                  15

ATG CTC GGC TTC GAC AAC ATC CTT GGG GTC GAC TAC TGG TTC GGC ATT      255
Met Leu Gly Phe Asp Asn Ile Leu Gly Val Asp Tyr Trp Phe Gly Ile
                20                  25                  30

CCC AGC GCC TTG CGC CGT GAC GGT GCC CAG GTC TAC GTC ACC GAA GGC      303
Pro Ser Ala Leu Arg Arg Asp Gly Ala Gln Val Tyr Val Thr Glu Gly
            35                  40                  45

AGC CAG TTG GAC ACC TCG GAA GTC CGC GGC GAG CAG TTG CTG CAA CAG      351
Ser Gln Leu Asp Thr Ser Glu Val Arg Gly Glu Gln Leu Leu Gln Gln
        50                  55                  60

GTG GAG GAA ATC GTC GCC CTC AGC GGC CAG CCC AAG GTC AAC CTG ATC      399
Val Glu Glu Ile Val Ala Leu Ser Gly Gln Pro Lys Val Asn Leu Ile
    65                  70                  75

GGC CAC AGC CAC GGC GGG CCG ACC ATC CGC TAC GTC GCC GCC GTA CGT      447
Gly His Ser His Gly Gly Pro Thr Ile Arg Tyr Val Ala Ala Val Arg
80                  85                  90                  95

CCC GAC CTG ATC GCT TCC GCC ACC AGC GTC GGC GCC CCG CAC AGG GGT      495
Pro Asp Leu Ile Ala Ser Ala Thr Ser Val Gly Ala Pro His Arg Gly
                100                 105                 110

TCG GAC ACC GCC GAC TTC CTG CGC CAG ATC CCA CCG GGT TCG GCC GGC      543
Ser Asp Thr Ala Asp Phe Leu Arg Gln Ile Pro Pro Gly Ser Ala Gly
            115                 120                 125

GAG GCA GTC CTC TCC GGG CTG GTC AAC AGC CTC GGC GCG CTG ATC AGC      591
Glu Ala Val Leu Ser Gly Leu Val Asn Ser Leu Gly Ala Leu Ile Ser
        130                 135                 140

TTC CTT TCC AGC GGC GGC ACC GGT ACG CAG AAT TTA CTG GGC TCG CTG      639
Phe Leu Ser Ser Gly Gly Thr Gly Thr Gln Asn Leu Leu Gly Ser Leu
    145                 150                 155
```

```
GAG TCG CTG AAC AGT GAG GGT GCC GCG CGC TTC AAC GCC AAG TAC CCG      687
Glu Ser Leu Asn Ser Glu Gly Ala Ala Arg Phe Asn Ala Lys Tyr Pro
160                 165                 170                 175

CAG GGC ATC CCC ACC TCG GCC TGC GGC GAA GGC GCT TAC AAG GTC AAC      735
Gln Gly Ile Pro Thr Ser Ala Cys Gly Glu Gly Ala Tyr Lys Val Asn
                180                 185                 190

GGC GTG AGC TAT TAC TCC TGG AGC GGT TCC TCG CCG CTG ACC AAC TTC      783
Gly Val Ser Tyr Tyr Ser Trp Ser Gly Ser Ser Pro Leu Thr Asn Phe
            195                 200                 205

CTC GAT CCG AGC GAC GCC TTC CTC GGC GCC TCG TCG CTG ACC TTC AAG      831
Leu Asp Pro Ser Asp Ala Phe Leu Gly Ala Ser Ser Leu Thr Phe Lys
        210                 215                 220

AAC GGC ACC GCC AAC GAC GGC CTG GTC GGC ACC TGC AGT TCG CAC CTG      879
Asn Gly Thr Ala Asn Asp Gly Leu Val Gly Thr Cys Ser Ser His Leu
    225                 230                 235

GGC ATG GTG ATC CGC GAC AAC TAC CGG ATG AAC CAC CTG GAC GAG GTG      927
Gly Met Val Ile Arg Asp Asn Tyr Arg Met Asn His Leu Asp Glu Val
240                 245                 250                 255

AAC CAG GTC CTC GGC CTC ACC AGC CTG TTC GAG ACC AGC CCG GTC AGC      975
Asn Gln Val Leu Gly Leu Thr Ser Leu Phe Glu Thr Ser Pro Val Ser
                260                 265                 270

GTC TAC CGC CAG CAC GCC AAC CGC CTG AAG AAC GCC AGC CTG             1017
Val Tyr Arg Gln His Ala Asn Arg Leu Lys Asn Ala Ser Leu
            275                 280                 285

TAGGACCCCG GCCGGGGCCT CGGCCCGGGC CC                                 1049
```

(2) ANGABEN ZU SEQ ID NO: 14:

(i) SEQUENZKENNZEICHEN:

(A) LÄNGE: 311 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 14:

```
Met Lys Lys Lys Ser Leu Leu Pro Leu Gly Leu Ala Ile Gly Leu Ala
-26 -25                 -20                 -15

Ser Leu Ala Ala Ser Pro Leu Ile Gln Ala Ser Thr Tyr Thr Gln Thr
-10                 -5                  1               5

Lys Tyr Pro Ile Val Leu Ala His Gly Met Leu Gly Phe Asp Asn Ile
            10                  15                  20

Leu Gly Val Asp Tyr Trp Phe Gly Ile Pro Ser Ala Leu Arg Arg Asp
        25                  30                  35

Gly Ala Gln Val Tyr Val Thr Glu Gly Ser Gln Leu Asp Thr Ser Glu
    40                  45                  50
```

```
Val Arg Gly Glu Gln Leu Leu Gln Gln Val Glu Glu Ile Val Ala Leu
 55                 60                  65                  70

Ser Gly Gln Pro Lys Val Asn Leu Ile Gly His Ser His Gly Gly Pro
                75                  80                  85

Thr Ile Arg Tyr Val Ala Ala Val Arg Pro Asp Leu Ile Ala Ser Ala
            90                  95                 100

Thr Ser Val Gly Ala Pro His Arg Gly Ser Asp Thr Ala Asp Phe Leu
        105                 110                 115

Arg Gln Ile Pro Pro Gly Ser Ala Gly Glu Ala Val Leu Ser Gly Leu
    120                 125                 130

Val Asn Ser Leu Gly Ala Leu Ile Ser Phe Leu Ser Ser Gly Gly Thr
135                 140                 145                 150

Gly Thr Gln Asn Leu Leu Gly Ser Leu Glu Ser Leu Asn Ser Glu Gly
                155                 160                 165

Ala Ala Arg Phe Asn Ala Lys Tyr Pro Gln Gly Ile Pro Thr Ser Ala
            170                 175                 180

Cys Gly Glu Gly Ala Tyr Lys Val Asn Gly Val Ser Tyr Tyr Ser Trp
        185                 190                 195

Ser Gly Ser Ser Pro Leu Thr Asn Phe Leu Asp Pro Ser Asp Ala Phe
    200                 205                 210

Leu Gly Ala Ser Ser Leu Thr Phe Lys Asn Gly Thr Ala Asn Asp Gly
215                 220                 225                 230

Leu Val Gly Thr Cys Ser Ser His Leu Gly Met Val Ile Arg Asp Asn
                235                 240                 245

Tyr Arg Met Asn His Leu Asp Glu Val Asn Gln Val Leu Gly Leu Thr
            250                 255                 260

Ser Leu Phe Glu Thr Ser Pro Val Ser Val Tyr Arg Gln His Ala Asn
        265                 270                 275

Arg Leu Lys Asn Ala Ser Leu
    280                 285
```

(2) ANGABEN ZU SEQ ID NO: 15:

(i) SEQUENZKENNZEICHEN:

(A) LÄNGE: 1049 Basenpaare
(B) ART: Nucleotid
(C) STRANGFORM: nicht bekannt
(D) TOPOLOGIE: nicht bekannt

(ii) ART DES MOLEKÜLS: Genom-DNA

(ix) MERKMAL:

(A) NAME/SCHLÜSSEL: CDS
(B) LAGE:85..1017

(ix) MERKMAL:

(A) NAME/SCHLÜSSEL: mat_peptide
(B) LAGE:163..1017

(xi) SEQUENZBESCHREIBUNG: SEQ ID NO: 15:

```
GGATCCCCCG GTTCTCCCGG AAGGATTCGG GCGATGGCTG GCAGGACGCG CCCCTCGGCC    60

CCATCAACCT GAGATGAGAA CAAC ATG AAG AAG AAG TCT CTG CTC CCC CTC      111
                           Met Lys Lys Lys Ser Leu Leu Pro Leu
                           -26 -25                 -20

GGC CTG GCC ATC GGT CTC GCC TCT CTC GCT GCC AGC CCT CTG ATC CAG     159
Gly Leu Ala Ile Gly Leu Ala Ser Leu Ala Ala Ser Pro Leu Ile Gln
        -15                 -10                 -5

GCC AGC ACC TAC ACC CAG ACC AAA TAC CCC ATC GTG CTG GCC CAC GGC     207
Ala Ser Thr Tyr Thr Gln Thr Lys Tyr Pro Ile Val Leu Ala His Gly
     1               5                  10                  15

ATG CTC GGC TTC GAC AAC ATC CTT GGG GTC GAC TAC TGG TTC GGC ATT     255
Met Leu Gly Phe Asp Asn Ile Leu Gly Val Asp Tyr Trp Phe Gly Ile
                20                  25                  30

CCC AGC GCC TTG CGC CGT GAC GGT GCC CAG GTC TAC GTC ACC GAA GGC     303
Pro Ser Ala Leu Arg Arg Asp Gly Ala Gln Val Tyr Val Thr Glu Gly
            35                  40                  45

AGC CAG TTG GAC ACC TCG GAA GTC CGC GGC GAG CAG TTG CTG CAA CAG     351
Ser Gln Leu Asp Thr Ser Glu Val Arg Gly Glu Gln Leu Leu Gln Gln
        50                  55                  60

GTG GAG GAA ATC GTC GCC CTC AGC GGC CAG CCC AAG GTC AAC CTG ATC     399
Val Glu Glu Ile Val Ala Leu Ser Gly Gln Pro Lys Val Asn Leu Ile
    65                  70                  75

GGC CAC AGC CAC GGC GGG CCG ACC ATC CGC TAC GTC GCC GCC GTA CGT     447
Gly His Ser His Gly Gly Pro Thr Ile Arg Tyr Val Ala Ala Val Arg
80                  85                  90                  95

CCC GAC CTG ATC GCT TCC GCC ACC AGC GTC GGC GCC CCG CAC AAG GGT     495
Pro Asp Leu Ile Ala Ser Ala Thr Ser Val Gly Ala Pro His Lys Gly
                100                 105                 110

TCG GAC ACC GCC GAC TTC CTG CGC CAG ATC CCA CCG GGT TCG GCC GGC     543
Ser Asp Thr Ala Asp Phe Leu Arg Gln Ile Pro Pro Gly Ser Ala Gly
            115                 120                 125

GAG GCA GTC CTC TCC GGG CTG GTC AAC AGC CTC GGC GCG CTG ATC AGC     591
Glu Ala Val Leu Ser Gly Leu Val Asn Ser Leu Gly Ala Leu Ile Ser
        130                 135                 140

TTC CTT TCC AGC GGC GGC ATC GGT ACG CAG AAT TTT CTG GGC TCG CTG     639
Phe Leu Ser Ser Gly Gly Ile Gly Thr Gln Asn Phe Leu Gly Ser Leu
    145                 150                 155
```

```
GAG TCG CTG AAC AGC GAG GGT GCC GCG CGC TTC AAC GCC AAG TAC CCG      687
Glu Ser Leu Asn Ser Glu Gly Ala Ala Arg Phe Asn Ala Lys Tyr Pro
160                 165                 170                 175

CAG GGC ATC CCC ACC TCG GCC TGC GGC GAA GGC GCC TAC AAG GTC AAC      735
Gln Gly Ile Pro Thr Ser Ala Cys Gly Glu Gly Ala Tyr Lys Val Asn
                180                 185                 190

GGC GTG AGC TAT TAC TCC TGG AGC GGT TCC TCG CCG CTG ACC AAC TTC      783
Gly Val Ser Tyr Tyr Ser Trp Ser Gly Ser Ser Pro Leu Thr Asn Phe
            195                 200                 205

CTC GAT CCG AGC GAC GCC TTC CTC GGC GCC TCG TCG CTG ACC TTC AAG      831
Leu Asp Pro Ser Asp Ala Phe Leu Gly Ala Ser Ser Leu Thr Phe Lys
        210                 215                 220

AAC GGC ACC GCC AAC GAC GGC CTG GTC GGC ACC TGC AGT TCG CAC CTG      879
Asn Gly Thr Ala Asn Asp Gly Leu Val Gly Thr Cys Ser Ser His Leu
    225                 230                 235

GGC ATG GTG ATC CGC GAC AAC TAC CGG ATG AAC CAC CTG GAC GAG GTG      927
Gly Met Val Ile Arg Asp Asn Tyr Arg Met Asn His Leu Asp Glu Val
240                 245                 250                 255

AAC CAG GTC TTC GGC CTC ACC AGC CTG TTC GAG ACC AGC CCG GTC AGC      975
Asn Gln Val Phe Gly Leu Thr Ser Leu Phe Glu Thr Ser Pro Val Ser
                260                 265                 270

GTC TAC CGC CAG CAC GCC AAC CGC CTG AAG AAC GCC AGC CTG             1017
Val Tyr Arg Gln His Ala Asn Arg Leu Lys Asn Ala Ser Leu
            275                 280                 285

TAGGACCCCG GCCGGGGCCT CGGCCCGGGC CC                                 1049
```

(2) ANGABEN ZU SEQ ID NO: 16:

(i) SEQUENZKENNZEICHEN:

(A) LÄNGE: 311 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 16:

```
Met Lys Lys Lys Ser Leu Leu Pro Leu Gly Leu Ala Ile Gly Leu Ala
-26 -25                 -20                 -15

Ser Leu Ala Ala Ser Pro Leu Ile Gln Ala Ser Thr Tyr Thr Gln Thr
-10                 -5                  1               5

Lys Tyr Pro Ile Val Leu Ala His Gly Met Leu Gly Phe Asp Asn Ile
            10                  15                  20

Leu Gly Val Asp Tyr Trp Phe Gly Ile Pro Ser Ala Leu Arg Arg Asp
        25                  30                  35
```

```
Gly Ala Gln Val Tyr Val Thr Glu Gly Ser Gln Leu Asp Thr Ser Glu
     40                  45                  50

Val Arg Gly Glu Gln Leu Leu Gln Gln Val Glu Glu Ile Val Ala Leu
 55                  60                  65                  70

Ser Gly Gln Pro Lys Val Asn Leu Ile Gly His Ser His Gly Gly Pro
                 75                  80                  85

Thr Ile Arg Tyr Val Ala Ala Val Arg Pro Asp Leu Ile Ala Ser Ala
             90                  95                 100

Thr Ser Val Gly Ala Pro His Lys Gly Ser Asp Thr Ala Asp Phe Leu
        105                 110                 115

Arg Gln Ile Pro Pro Gly Ser Ala Gly Glu Ala Val Leu Ser Gly Leu
    120                 125                 130

Val Asn Ser Leu Gly Ala Leu Ile Ser Phe Leu Ser Ser Gly Gly Ile
135                 140                 145                 150

Gly Thr Gln Asn Phe Leu Gly Ser Leu Glu Ser Leu Asn Ser Glu Gly
                155                 160                 165

Ala Ala Arg Phe Asn Ala Lys Tyr Pro Gln Gly Ile Pro Thr Ser Ala
            170                 175                 180

Cys Gly Glu Gly Ala Tyr Lys Val Asn Gly Val Ser Tyr Tyr Ser Trp
        185                 190                 195

Ser Gly Ser Ser Pro Leu Thr Asn Phe Leu Asp Pro Ser Asp Ala Phe
    200                 205                 210

Leu Gly Ala Ser Ser Leu Thr Phe Lys Asn Gly Thr Ala Asn Asp Gly
215                 220                 225                 230

Leu Val Gly Thr Cys Ser Ser His Leu Gly Met Val Ile Arg Asp Asn
                235                 240                 245

Tyr Arg Met Asn His Leu Asp Glu Val Asn Gln Val Phe Gly Leu Thr
            250                 255                 260

Ser Leu Phe Glu Thr Ser Pro Val Ser Val Tyr Arg Gln His Ala Asn
        265                 270                 275

Arg Leu Lys Asn Ala Ser Leu
    280                 285
```

(2) ANGABEN ZU SEQ ID NO: 17:

(i) SEQUENZKENNZEICHEN:

(A) LÄNGE: 1049 Basenpaare
(B) ART: Nucleotid
(C) STRANGFORM: nicht bekannt
(D) TOPOLOGIE: nicht bekannt

(ii) ART DES MOLEKÜLS: Genom-DNA

(ix) MERKMAL:

(A) NAME/SCHLÜSSEL: CDS
(B) LAGE:85..1017

(ix) MERKMAL:

(A) NAME/SCHLÜSSEL: mat_peptide
(B) LAGE:163..1017

(xi) SEQUENZBESCHREIBUNG: SEQ ID NO: 17:

```
GGATCCCCCG GTTCTCCCGG AAGGATTCGG GCGATGGCTG GCAGGACGCG CCCCTCGGCC    60

CCATCAACCT GAGATGAGAA CAAC ATG AAG AAG AAG TCT CTG CTC CCC CTC      111
                           Met Lys Lys Lys Ser Leu Leu Pro Leu
                           -26 -25                 -20

GGC CTG GCC ATC GGT CTC GCC TCT CTC GCT GCC AGC CCT CTG ATC CAG     159
Gly Leu Ala Ile Gly Leu Ala Ser Leu Ala Ala Ser Pro Leu Ile Gln
        -15                 -10                 -5

GCC AGC ACC TAC ACC CAG ACC AAA TAC CCC ATC GTG CTG GCC CAC GGC     207
Ala Ser Thr Tyr Thr Gln Thr Lys Tyr Pro Ile Val Leu Ala His Gly
     1               5                  10                  15

ATG CTC GGC TTC GAC AAC ATC CTT GGG GTC GAC TAC TGG TTC GGC ATT     255
Met Leu Gly Phe Asp Asn Ile Leu Gly Val Asp Tyr Trp Phe Gly Ile
                 20                  25                  30

CCC AGC GCC TTG CGC CGT GAC GGT GCC CAG GTC TAC GTC ACC GAA GGC     303
Pro Ser Ala Leu Arg Arg Asp Gly Ala Gln Val Tyr Val Thr Glu Gly
             35                  40                  45

AGC CAG TTG GAC ACC TCG GAA GTC CGC GGC GAG CAG TTG CTG CAA CAG     351
Ser Gln Leu Asp Thr Ser Glu Val Arg Gly Glu Gln Leu Leu Gln Gln
         50                  55                  60

GTG GAG GAA ATC GTC GCC CTC AGC GGC CAG CCC AAG GTC AAC CTG ATC     399
Val Glu Glu Ile Val Ala Leu Ser Gly Gln Pro Lys Val Asn Leu Ile
     65                  70                  75

GGC CAC AGC CAC GGC GGG CCG ACC ATC CGC TAC GTC GCC GCC GTA CGT     447
Gly His Ser His Gly Gly Pro Thr Ile Arg Tyr Val Ala Ala Val Arg
 80                  85                  90                  95

CCC GAC CTG ATC GCT TCC GCC ACC AGC GTC GGC GCC CCG CAC AAG GGT     495
Pro Asp Leu Ile Ala Ser Ala Thr Ser Val Gly Ala Pro His Lys Gly
                100                 105                 110

TCG GAC ACC GCC GAC TTC CTG CGC CAG ATC CCA CCG GGT TCG GCC GGC     543
Ser Asp Thr Ala Asp Phe Leu Arg Gln Ile Pro Pro Gly Ser Ala Gly
            115                 120                 125

GAG GCA GTC CTC TCC GGG CTG GTC AAC AGC CTC GGC GCG CTG ATC AGC     591
Glu Ala Val Leu Ser Gly Leu Val Asn Ser Leu Gly Ala Leu Ile Ser
        130                 135                 140
```

```
TTC CTT TCC AGC GGC GGC ACC GGT ACG CAG AAT TTA CTG GGC TCG CTG        639
Phe Leu Ser Ser Gly Gly Thr Gly Thr Gln Asn Leu Leu Gly Ser Leu
    145                 150                 155

GAG TCG CTG AAC AGC GAG GGT GCC GCG CGC TTC AAC GCC AAG TAC CCG        687
Glu Ser Leu Asn Ser Glu Gly Ala Ala Arg Phe Asn Ala Lys Tyr Pro
160                 165                 170                 175

CAG GGC ATC CCC ACC TCG GCC TGC GGC GAA GGC GCC TAC AAG GTC AAC        735
Gln Gly Ile Pro Thr Ser Ala Cys Gly Glu Gly Ala Tyr Lys Val Asn
                180                 185                 190

GGC GTG AGC TAT TAC TCC TGG AGC GGT TCC TCG CCG CTG ACC AAC TTC        783
Gly Val Ser Tyr Tyr Ser Trp Ser Gly Ser Ser Pro Leu Thr Asn Phe
            195                 200                 205

CTC GAT CCG AGC GAC GCC TTC CTC GGC GCC TCG TCG CTG ACC TTC AAG        831
Leu Asp Pro Ser Asp Ala Phe Leu Gly Ala Ser Ser Leu Thr Phe Lys
        210                 215                 220

AAC GGC ACC GCC AAC GAC GGC CTG GTC GGC ACC TGC AGT TCG CAC CTG        879
Asn Gly Thr Ala Asn Asp Gly Leu Val Gly Thr Cys Ser Ser His Leu
    225                 230                 235

GGC ATG GTG ATC CGC GAC AAC TAC CGG ATG AAC CAC CTG GAC GAG GTG        927
Gly Met Val Ile Arg Asp Asn Tyr Arg Met Asn His Leu Asp Glu Val
240                 245                 250                 255

AAC CAG GTC TTC GGC CTC ACC AGC CTG TTC GAG ACC AGC CCG GTC AGC        975
Asn Gln Val Phe Gly Leu Thr Ser Leu Phe Glu Thr Ser Pro Val Ser
                260                 265                 270

GTC TAC CGC CAG CAC GCC AAC CGC CTG AAG AAC GCC AGC CTG               1017
Val Tyr Arg Gln His Ala Asn Arg Leu Lys Asn Ala Ser Leu
            275                 280                 285

TAGGACCCCG GCCGGGGCCT CGGCCCGGGC CC                                    1049
```

(2) ANGABEN ZU SEQ ID NO: 18:

(i) SEQUENZKENNZEICHEN:

(A) LÄNGE: 311 Aminosäuren
(B) ART: Aminosäure
(D) TOPOLOGIE: linear

(ii) ART DES MOLEKÜLS: Protein

(xi) SEQUENZBESCHREIBUNG: SEQ ID NO: 18:

```
Met Lys Lys Lys Ser Leu Leu Pro Leu Gly Leu Ala Ile Gly Leu Ala
-26 -25                 -20                 -15

Ser Leu Ala Ala Ser Pro Leu Ile Gln Ala Ser Thr Tyr Thr Gln Thr
-10                 -5                  1                   5

Lys Tyr Pro Ile Val Leu Ala His Gly Met Leu Gly Phe Asp Asn Ile
                10                  15                  20
```

```
Leu Gly Val Asp Tyr Trp Phe Gly Ile Pro Ser Ala Leu Arg Arg Asp
        25                  30                  35

Gly Ala Gln Val Tyr Val Thr Glu Gly Ser Gln Leu Asp Thr Ser Glu
    40                  45                  50

Val Arg Gly Glu Gln Leu Leu Gln Gln Val Glu Glu Ile Val Ala Leu
 55                  60                  65                  70

Ser Gly Gln Pro Lys Val Asn Leu Ile Gly His Ser His Gly Gly Pro
                 75                  80                  85

Thr Ile Arg Tyr Val Ala Ala Val Arg Pro Asp Leu Ile Ala Ser Ala
             90                  95                 100

Thr Ser Val Gly Ala Pro His Lys Gly Ser Asp Thr Ala Asp Phe Leu
        105                 110                 115

Arg Gln Ile Pro Pro Gly Ser Ala Gly Glu Ala Val Leu Ser Gly Leu
    120                 125                 130

Val Asn Ser Leu Gly Ala Leu Ile Ser Phe Leu Ser Ser Gly Gly Thr
135                 140                 145                 150

Gly Thr Gln Asn Leu Leu Gly Ser Leu Glu Ser Leu Asn Ser Glu Gly
                155                 160                 165

Ala Ala Arg Phe Asn Ala Lys Tyr Pro Gln Gly Ile Pro Thr Ser Ala
            170                 175                 180

Cys Gly Glu Gly Ala Tyr Lys Val Asn Gly Val Ser Tyr Tyr Ser Trp
        185                 190                 195

Ser Gly Ser Ser Pro Leu Thr Asn Phe Leu Asp Pro Ser Asp Ala Phe
    200                 205                 210

Leu Gly Ala Ser Ser Leu Thr Phe Lys Asn Gly Thr Ala Asn Asp Gly
215                 220                 225                 230

Leu Val Gly Thr Cys Ser Ser His Leu Gly Met Val Ile Arg Asp Asn
                235                 240                 245

Tyr Arg Met Asn His Leu Asp Glu Val Asn Gln Val Phe Gly Leu Thr
            250                 255                 260

Ser Leu Phe Glu Thr Ser Pro Val Ser Val Tyr Arg Gln His Ala Asn
        265                 270                 275

Arg Leu Lys Asn Ala Ser Leu
    280                 285
```

(2) ANGABEN ZU SEQ ID NO: 19:

(i) SEQUENZKENNZEICHEN:

(A) LÄNGE: 30 Basenpaare
(B) ART: Nucleotid
(C) STRANGFORM: nicht bekannt
(D) TOPOLOGIE: linear

(ii) ART DES MOLEKÜLS: Sonstige Nucleinsäure

(A) BESCHREIBUNG: /desc = "synthetische DNA"

(xi) SEQUENZBESCHREIBUNG: SEQ ID NO: 19:

GCGCAATTAA CCCTCACTAA AGGGAACAAA 30

(2) ANGABEN ZU SEQ ID NO: 20:

(i) SEQUENZKENNZEICHEN:

(A) LÄNGE: 25 Basenpaare
(B) ART: Nucleotid
(C) STRANGFORM: nicht bekannt
(D) TOPOLOGIE: linear

(ii) ART DES MOLEKÜLS: Sonstige Nucleinsäure

(A) BESCHREIBUNG: /desc = "synthetische DNA"

(xi) SEQUENZBESCHREIBUNG: SEQ ID NO: 20:

GGTACGCAGA ATNNNCTGGG CTCGC 25

(2) ANGABEN ZU SEQ ID NO: 21:

(i) SEQUENZKENNZEICHEN:

(A) LÄNGE: 27 Basenpaare
(B) ART: Nucleotid
(C) STRANGFORM: nicht bekannt
(D) TOPOLOGIE: linear

(ii) ART DES MOLEKÜLS: Sonstige Nucleinsäure

(A) BESCHREIBUNG: /desc = "synthetische DNA"

(xi) SEQUENZBESCHREIBUNG: SEQ ID NO: 21:

GCGTAATACG ACTCACTATA GGGCGAA 27